# EUROPEAN PATENT SPECIFICATION

(11) **EP 2 471 943 B1**
(45) Date of publication and mention of the grant of the patent: **25.08.2021**
(21) Application number: 10811634.4
(22) Date of filing: 21.07.2010
(51) Int. Cl.: C12P 13/06, C12P 13/10, C12P 13/12, C12P 13/14, C12P 13/24

(54) **PROCESS FOR PRODUCTION OF L-AMINO ACID**
VERFAHREN ZUR HERSTELLUNG VON L-AMINOSÄURE
PROCÉDÉ DE FABRICATION D'UN L-AMINO ACIDE

(30) Priority: 25.08.2009 JP 2009194636
(43) Date of publication of application: 04.07.2012
(73) Proprietor: Ajinomoto Co., Inc., Tokyo 104-8315 (JP)
(72) Inventor: NAGAHIKO, Takeshi, Kawasaki-shi Kanagawa 210-8681 (JP); NAKAMURA, Jun, Kawasaki-shi Kanagawa 210-8681 (JP)
(74) Representative: Hoffmann Eitle
(86) International application number: PCT/JP2010/062253
(87) International publication number: WO 2011/024583

(56) References cited:
- EP-A1- 2 133 420
- WO-A1-2008/072640
- JP-A- 2007 222 163
- HASHIMOTO M ET AL: "Indispensability of the Escherichia coli carbonic anhydrases YadF and CynT in cell proliferation at a low CO2 partial pressure", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 67, no. 4, 1 April 2003 (2003-04-01), pages 919-922, XP002469753, ISSN: 0916-8451, DOI: 10.1271/BBB.67.919
- MITSUHASHI S. ET AL.: 'A gene homologous to b-type carbonic anhydrase is essential for the growth of Corynebacterium glutamicum under atmospheric conditions' APPL. MICROBIOL. BIOTECHNOL. vol. 63, 2004, pages 592 - 601, XP008154259

## Description

### Technical Field

The present description relates to a method for efficiently producing an L-amino acid selected from the group consisting of L-glutamic acid, L-glutamine, L-proline, L-arginine, L-alanine and L-aspartic acid.

### Background Art

L-Amino acids are industrially produced by fermentation mainly using L-amino acid-producing bacteria of the so-called coryneform bacteria belonging to the genus *Brevibacterium, Corynebacterium* or *Microbacterium,* or mutant strains thereof (refer to, for example, Non-patent document 1). As methods for producing an L-amino acid by fermentation using other bacterial strains, there are known methods of using a microorganism belonging to the genus *Bacillus, Streptomyces, Penicillium* or the like (refer to, for example, Patent document 1), methods of using a microorganism belonging to the genus *Pseudomonas, Arthrobacter, Serratia, Candida* or the like (refer to, for example, Patent document 2), methods of using a microorganism belonging to the genus *Bacillus, Pseudomonas,* or *Serratia, Aerobacter aerogenes* (currently referred to as *Enterobacter aerogenes*) or the like (refer to, for example, Patent document 3), methods of using a mutant strain of *Escherichia coli* (refer to, for example, Patent document 4), and so forth. In addition, methods for producing an L-amino acid using a microorganism belonging to the genus *Klebsiella, Erwinia, Pantoea* or *Enterobacter* have also been disclosed (refer to, for example, Patent documents 5 to 7).

Further, there have been disclosed various techniques of increasing L-amino acid-producing ability by enhancing an activity of an enzyme for biosynthesis of L-amino acid using recombinant DNA techniques. For example, it has been reported that introduction of a gene encoding a citrate synthase derived from *Escherichia coli* or *Corynebacterium glutamicum* into a *Corynebacterium* or *Brevibacterium* bacterium is effective for enhancement of L-glutamic acid-producing ability of the coryneform bacterium (for example, refer to Patent document 8). Furthermore, it has also been reported that introduction of a citrate synthase gene derived from a coryneform bacterium into an enterobacterium belonging to the genus *Enterobacter, Klebsiella, Serratia, Erwinia* or *Escherichia* is effective for enhancement of L-glutamic acid-producing ability of the bacterium (refer to, for example, Patent document 9).

Carbonic anhydrase is an enzyme involved in mutual conversion of carbon dioxide and bicarbonate radical. It has been reported that *Escherichia coli* has two kinds of carbonic anhydrases, i.e., Can (carbonic anhydrase 2) and CynT (carbonic anhydrase 1) (Non-patent documents 2 and 3). It has been elucidated that Can is a β type carbonic anhydrase, and is indispensable for growth of *Escherichia coli* under the usual atmospheric carbon dioxide partial pressure. Can and CynT are encoded by the *yadF* and *cynT* genes, respectively. It is also known that, in *Corynebacterium glutamicum,* β type and γ type carbonic anhydrases have been found, and the β type one mainly functions (refer to Non-patent document 4).

As production of a useful substance using a carbonic anhydrase, there is known use thereof in the production of ethanol from a vegetable raw material containing lignocellulose for the step of a pretreatment of the raw material (refer to Patent document 10). Further, it was reported that, in the study of enhancement of β-carbonic anhydrase of *Corynebacterium* bacteria, it did not increase production amount of lysine (refer to Non-patent document 4), and effectiveness of enhancement of β-carbonic anhydrase on production of a substance and relation between the β-carbonic anhydrase activity and L-amino acid productivity have still remained unknown.

### Prior Art References

### Patent documents

Patent document 1: U.S. Patent No. 3,220,929
Patent document 2: U.S. Patent No. 3,563,857
Patent document 3: Japanese Patent Publication (KOKOKU) No. 32-9393
Patent document 4: Japanese Patent Laid-open (KOKAI) No. 5-244970
Patent document 5: Japanese Patent Laid-open No. 2000-106869
Patent document 6: Japanese Patent Laid-open No. 2000-189169
Patent document 7: Japanese Patent Laid-open No. 2000-189175
Patent document 8: Japanese Patent Publication No. 7-121228
Patent document 9: Japanese Patent Laid-open No. 2000-189175
Patent document 10: U.S. Published Patent Application No. 2008/0171370

### Non-patent documents

Non-patent document 1: Akashi K. et al., Amino Acid Fermentation, Japan Scientific Societies Press, 195-215, 1986
Non-patent document 2: J. Biol. Chem, 267, 3731-3734, 1992
Non-patent document 3: Smith K.S., Ferry J.G., "Prokaryotic Carbonic Anhydrases", FEMS Microbiol. Rev., 24(4):335-66, 2000
Non-patent document 4: Appl. Microbiol. Biotechnol., 63, 592-601, 2004

Mitsuhashi et al., Appl. Microbiol. Biotechnol., vol. 63, 2004, pages 592-601, concerns a gene homologous to b-type carbonic anhydrase is essential for the growth of Corynebacterium glutamicum under atmospheric conditions.

JP 2007 222163 A concerns a method for producing L-amino acid.

EP2133420 discloses a method for producing an L-amino acid by fermentation of a recombinant microorganism of the Enterobacteriaceae family, which comprises the amplification or overexpression of a can-gene and/or cynT-gene, wherein both genes encode β-carbonic anhydrases.

WO2008/072640 concerns a method for producing an L-amino acid using a bacterium of the Enterobacteriaceae family which has been modified to attenuate expression of the cynTSX operon and the cynR gene. The cynT-gene encodes β-carbonic anhydrase.

### Summary of the Invention

The current invention refers to a method for producing an L-amino acid, which comprises culturing a coryneform bacterium having an L-amino acid-producing ability in a medium to produce and accumulate the L-amino acid in the medium or cells of the bacterium, and collecting the L-amino acid from the medium or cells, wherein said coryneform bacterium has been modified to enhance carbonic anhydrase activity, and wherein the L-amino acid is selected from the group consisting of L-glutamic acid, L-glutamine, L-proline, L-arginine, L-alanine and L-aspartic acid.

An object of the present invention is to provide a novel method for producing an L-amino acid, wherein the L-amino acid is selected from the group consisting of L-glutamic acid, L-glutamine, L-proline, L-arginine, L-alanine and L-aspartic acid, by fermentation.

The inventors of the present invention conducted various researches in order to achieve the aforementioned object. As a result, they found that by culturing a coryneform bacterium that has been imparted with an L-amino acid-producing ability and has been modified to enhance carbonic anhydrase (henceforth carbonic anhydrase refers to β-carbonic anhydrase unless specifically indicated) activity, an L-amino acid such as L-glutamic acid, L-glutamine, L-proline, L-arginine and L-aspartic acid can be efficiently produced.

According to the production method as described herein, L-amino acids such as L-glutamic acid, L-glutamine, L-proline, L-arginine and L-aspartic acid can be efficiently produced.

### Brief Description of the Drawing

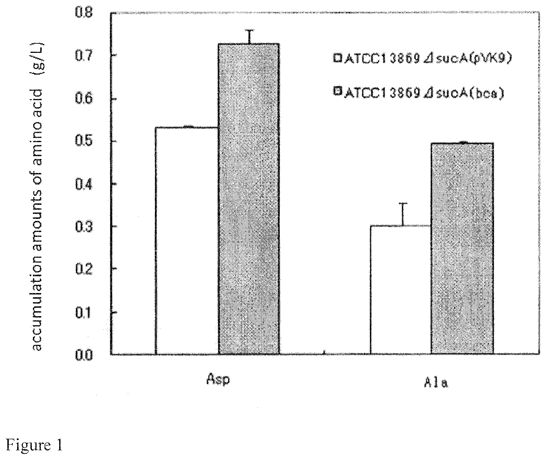

[Fig. 1] Fig. 1 shows L-amino acid accumulation amount observed with a carbonic anhydrase (BCA)-enhanced strain.

### Modes for Carrying out the Invention

Hereafter, the present invention will be explained in detail.

### <1> Coryneform bacteria used for the method of the present invention

The coryneform bacterium used for the method of the present invention is a coryneform bacterium having an L-amino acid-producing ability and modified to enhance carbonic anhydrase activity. The coryneform bacterium used for the method of the present invention can be obtained by using a coryneform bacterium having an L-amino acid-producing ability as a parent strain, and modifying it so that carbonic anhydrase activity thereof is enhanced. The coryneform bacterium used for the method of the present invention may be a coryneform bacterium inherently having an L-amino acid-producing ability, or may be a coryneform bacterium imparted with an L-amino acid-producing ability by breeding utilizing a mutation method, recombinant DNA technique, or the like. In the present invention, the "coryneform bacteria" also include bacteria which have previously been classified into the genus *Brevibacterium* but are presently classified into the genus *Corynebacterium* (Int. J. Syst. Bacteriol., 41:255-260, 1991), and bacteria belonging to the genus *Brevibacterium,* which are closely related to the genus *Corynebacterium.* Examples of such coryneform bacteria include the followings:
*Corynebacterium acetoacidophilum*
*Corynebacterium acetoglutamicum*
*Corynebacterium alkanolyticum*
*Corynebacterium callunae*
*Corynebacterium glutamicum*
*Corynebacterium lilium*
*Corynebacterium melassecola*
*Corynebacterium thermoaminogenes (Corynebacterium efficiens)*
*Corynebacterium herculis*
*Brevibacterium divaricatum*
*Brevibacterium flavum*
*Brevibacterium immariophilum*
*Brevibacterium lactofermentum*
*Brevibacterium roseum*
*Brevibacterium saccharolyticum*
*Brevibacterium thiogenitalis*
*Corynebacterium ammoniagenes*
*Brevibacterium album*
*Brevibacterium cerinum*
*Microbacterium ammoniaphilum*

Specific examples of these bacteria include the following strains:
*Corynebacterium acetoacidophilum* ATCC 13870
*Corynebacterium acetoglutamicum* ATCC 15806
*Corynebacterium alkanolyticum* ATCC 21511
*Corynebacterium callunae* ATCC 15991
*Corynebacterium glutamicum* ATCC 13020, ATCC 13032, ATCC 13060
*Corynebacterium lilium* ATCC 15990
*Corynebacterium melassecola* ATCC 17965
*Corynebacterium thermoaminogenes* AJ12340 (FERM BP-1539)
*Corynebacterium herculis* ATCC 13868
*Brevibacterium divaricatum* ATCC 14020
*Brevibacterium flavum* ATCC 13826, ATCC 14067
*Brevibacterium immariophilum* ATCC 14068
*Brevibacterium lactofermentum* ATCC 13869
*Brevibacterium roseum* ATCC 13825
*Brevibacterium saccharolyticum* ATCC 14066
*Brevibacterium thiogenitalis* ATCC 19240
*Corynebacterium ammoniagenes* ATCC 6871, ATCC 6872
*Brevibacterium album* ATCC 15111
*Brevibacterium cerinum* ATCC 15112
*Microbacterium ammoniaphilum* ATCC 15354

These strains are available from, for example, the American Type Culture Collection (Address: 12301 Parklawn Drive, Rockville, Maryland 20852, P.O. Box 1549, Manassas, VA 20108, United States of America). That is, registration numbers are given to each of the strains, and the strains can be ordered by using these registration numbers (refer to http://www.atcc.org/). The registration numbers of the strains are listed in the catalogue of the American Type Culture Collection. The AJ12340 strain was deposited on October 27, 1987 at National Institute of Bioscience and Human Technology, Agency of Industrial Science and Technology, Ministry of Economy, Trade and Industry (currently the independent administrative agency, National Institute of Advanced Industrial Science and Technology, International Patent Organism Depositary, Tsukuba Central 6, 1-1, Higashi 1-Chome, Tsukuba-shi, Ibaraki-ken, 305-5466, Japan) with an accession number of FERM BP-1539 under the provisions of Budapest Treaty.

In the present invention, the "L-amino acid-producing ability" means an ability of the coryneform bacterium used for the method of the present invention to accumulate an L-amino acid in a medium, when the bacterium is cultured in the medium. This L-amino acid-producing ability may be a property that a wild-type strain of the coryneform bacterium has, or a property imparted or enhanced by breeding. Examples of the L-amino acid include L-lysine, L-glutamic acid, L-threonine, L-valine, L-leucine, L-isoleucine, L-serine, L-aspartic acid, L-asparagine, L-glutamine, L-arginine, L-cysteine (cystine), L-methionine, L-phenylalanine, L-tryptophan, L-tyrosine, L-glycine, L-alanine, L-proline, L-ornithine, L-citrulline, and L-homoserine.

### <1-1> Impartation of L-amino acid-producing ability

Hereafter, methods for imparting an L-amino acid-producing ability to a coryneform bacterium and coryneform bacteria imparted with an L-amino acid-producing ability will be explained with reference to examples.

Examples of the method for imparting or enhancing L-glutamic acid-producing ability by breeding include, for example, a method of modifying a bacterium so that expression of a gene encoding an enzyme involved in the L-glutamic acid biosynthesis is enhanced. Examples of such an enzyme involved in the L-glutamic acid biosynthesis include, for example, glutamate dehydrogenase, glutamine synthetase, glutamate synthase, isocitrate dehydrogenase, aconitate hydratase, citrate synthase, phosphoenolpyruvate carboxylase, pyruvate carboxylase, pyruvate dehydrogenase, pyruvate kinase, phosphoenolpyruvate synthase, enolase, phosphoglyceromutase, phosphoglycerate kinase, glyceraldehydes-3-phosphate dehydrogenase, triosephosphate isomerase, fructose bisphosphate aldolase, phosphofructokinase, glucose phosphate isomerase, and so forth.

Examples of the method for enhancing expression of such genes as mentioned above include introduction of an amplification plasmid obtained by introducing a DNA fragment containing any of these genes into an appropriate plasmid, for example, a plasmid containing at least a gene responsible for replication and proliferation functions of the plasmid in a coryneform bacterium, increasing copy number of any of these genes on a chromosome by conjugation, gene transfer, or the like, and introduction of a mutation into a promoter region of any of these genes (refer to International Patent Publication WO95/34672)

When the aforementioned amplification plasmid is used, or copy number is increased on a chromosome, the promoter for expressing these genes may be any kind of promoter so long as a promoter that functions in coryneform bacteria is chosen, and may be a promoter of the gene to be used. Expression amount of a gene can also be controlled by appropriately choosing a promoter. Examples of coryneform bacteria modified by such methods as mentioned above so that expression of a citrate synthase gene, phosphoenolpyruvate carboxylase gene and/or glutamate dehydrogenase gene is enhanced include the coryneform bacteria disclosed in WO00/18935 and so forth.

The modification for imparting L-glutamic acid-producing ability may be attained by decreasing or eliminating activity of an enzyme that catalyzes a reaction branching off from the L-glutamic acid biosynthesis pathway and producing a compound other than L-glutamic acid. Examples of such an enzyme that catalyzes a reaction branching off from the L-glutamic acid biosynthesis pathway and producing a compound other than L-glutamic acid include isocitrate lyase, α-ketoglutarate dehydrogenase, phosphate acetyltransferase, acetate kinase, acetohydroxy acid synthase, acetolactate synthase, formate acetyltransferase, lactate dehydrogenase, glutamate decarboxylase, 1-pyrroline dehydrogenase, and so forth.

In order to reduce or eliminate activities of these enzymes, a mutation may be introduced into genes of the enzymes on a chromosome by a usual mutagenesis method so that intracellular activities of the enzymes are reduced or eliminated. Such introduction of a mutation can be achieved by, for example, using genetic recombination to eliminate the genes encoding the enzymes on the chromosome or modify an expression control sequence such as a promoter or the Shine-Dalgarno (SD) sequence. It can also be achieved by introducing a mutation for amino acid substitution (missense mutation), a stop codon (nonsense mutation), or a frame shift mutation for adding or deleting one or two nucleotides into regions encoding the enzymes on the chromosome, or partially deleting the genes (J. Biol. Chem., 272:8611-8617, 1997). The enzymatic activities can also be decreased or eliminated by constructing a gene encoding a mutant enzyme, of which coding region is deleted, and substituting it for a normal gene on a chromosome by homologous recombination or the like.

For example, in order to decrease the α-ketoglutarate dehydrogenase activity, modification may be performed by using the *sucA* (*odhA*) gene encoding the E1o subunit of the enzyme.

The nucleotide sequence of the *sucA* gene and the amino acid sequence encoded thereby are shown as SEQ ID NOS: 9 and 10. For example, disruption of the *sucA* gene can be performed by the method described in the example described later using the primers of SEQ ID NOS: 1 to 6.

In addition, examples of strains with decreased α-ketoglutarate dehydrogenase activity include, for example, the following strains:
*Brevibacterium lactofermentum* ΔS strain (WO95/34672)
*Brevibacterium lactofermentum* AJ12821 (FERM BP-4172, French Patent No. 9401748)
*Brevibacterium flavum* AJ12822 (FERM BP-4173, French Patent No. 9401748)
*Corynebacterium glutamicum* AJ12823 (FERM BP-4174, French Patent No. 9401748)

Examples of other methods for imparting or enhancing L-glutamic acid-producing ability also include a method of imparting resistance to an organic acid analogue, a respiratory chain inhibitor, or the like, and a method of imparting sensitivity to a cell wall synthesis inhibitor. Examples of such methods include the method of imparting resistance to benzopyrones or naphthoquinones (Japanese Patent Laid-open No. 56-1889), the method of imparting resistance to HOQNO (Japanese Patent Laid-open No. 56-140895), the method of imparting resistance to α-ketomalonic acid (Japanese Patent Laid-open No. 57-2689), the method of imparting resistance to guanidine (Japanese Patent Laid-open No. 56-35981), the method of imparting sensitivity to penicillin (Japanese Patent Laid-open No. 4-88994), and so forth.

Specific examples of such resistant strains include the following strains:
*Brevibacterium flavum* AJ11355 (FERM P-5007, refer to Japanese Patent Laid-open No. 56-1889)
*Corynebacterium glutamicum* AJ11368 (FERM P-5020, refer to Japanese Patent Laid-open No. 56-1889)
*Brevibacterium flavum* AJ11217 (FERM P-4318, refer to Japanese Patent Laid-open No. 57-2689)
*Corynebacterium glutamicum* AJ11218 (FERM P-4319, refer to Japanese Patent Laid-open No. 57-2689)
*Brevibacterium flavum* AJ11564 (FERM BP-5472, refer to Japanese Patent Laid-open No. 56-140895)
*Brevibacterium flavum* AJ11439 (FERM BP-5136, refer to Japanese Patent Laid-open No. 56-35981)
*Corynebacterium glutamicum* H7684 (FERM BP-3004, refer to Japanese Patent Laid-open No. 04-88994)

Examples of method for imparting L-glutamine-producing ability include, for example, a method of modifying a bacterium so that expression of a gene encoding an enzyme involved in the L-glutamine biosynthesis is enhanced. Examples of the enzyme involved in the L-glutamine biosynthesis include, for example, glutamine synthetase and glutamate dehydrogenase (Japanese Patent Laid-open No. 2002-300887).

Modification for imparting L-glutamine-producing ability may also be attained by reducing or deleting activity of an enzyme that catalyzes a reaction branching off from the biosynthesis pathway of L-glutamine and producing another compound. For example, it is conceivable to reduce intracellular glutaminase activity (Japanese Patent Laid-open No. 2004-187684).

Examples of methods for imparting or enhancing L-glutamine-producing ability also include imparting resistance to amino acid analogues and so forth. Specific examples include imparting 6-diazo-5-oxo-norleucine resistance (Japanese Patent Laid-open No. 3-232497), imparting purine analogue resistance and/or methionine sulfoxide resistance (Japanese Patent Laid-open No. 61-202694), imparting α -ketomalonic acid resistance (Japanese Patent Laid-open No. 56-151495), imparting resistance to a peptide containing glutamic acid (Japanese Patent Laid-open No. 2-186994), and so forth.

Specific examples of coryneform bacteria having L-glutamine-producing ability include the following strains:
*Brevibacterium flavum* AJ11573 (FERM P-5492, Japanese Patent Laid-open No. 56-151495)
*Brevibacterium flavum* AJ12210 (FERM P-8123, Japanese Patent Laid-open No. 61-202694)
*Brevibacterium flavum* AJ12212 (FERM P-8125, Japanese Patent Laid-open No. 61-202694)
*Brevibacterium flavum* AJ12418 (FERM-BP2205, Japanese Patent Laid-open No. 2-186994)
*Brevibacterium flavum* DH18 (FERM P-11116, Japanese Patent Laid-open No. 3-232497)
*Corynebacterium melassecola* DH344 (FERM P-11117, Japanese Patent Laid-open No. 3-232497)
*Corynebacterium glutamicum* AJ11574 (FERM P-5493, Japanese Patent Laid-open No. 56-151495)

Examples of method for imparting L-proline-producing ability include, for example, a method of modifying a bacterium so that expression of a gene encoding an enzyme involved in the L-proline biosynthesis is enhanced. Examples of the enzyme involved in the L-proline biosynthesis include, for example, glutamate-5-kinase, γ-glutamylphosphate reductase, and pyroline-5-carboxylate reductase.

Modification for imparting L-proline-producing ability may be performed by reducing or deleting an activity of an enzyme that catalyzes a reaction branching off from the biosynthesis pathway of L-proline to produce another compound. Examples include, for example, reducing intracellular ornithine aminotransferase activity.

Examples of method for imparting L-arginine-producing ability include a method of modifying a bacterium so that expression of a gene encoding an enzyme involved in the L-arginine biosynthesis is enhanced. Examples of L-arginine biosynthetic enzymes include one or more chosen from N-acetylglutamyl phosphate reductase (*argC*), ornithine acetyltransferase (*argJ*), N-acetylglutamate kinase (*argB*), acetylornithine transaminase (*argD*), ornithine carbamoyl transferase (*argF*), argininosuccinate synthase (*argG*), argininosuccinate lyase (*argH*), and carbamoylphosphate synthase.

Another method for imparting L-arginine-producing ability is a method of imparting resistance to an amino acid analogue or the like. Examples of bacteria obtained by such a method include coryneform bacteria exhibiting L-histidine, L-proline, L-threonine, L-isoleucine, L-methionine, or L-tryptophan auxotrophy in addition to the resistance to 2-thiazolealanine (Japanese Patent Laid-open No. 54-44096); coryneform bacteria resistant to ketomalonic acid, fluoromalonic acid, or monofluoroacetic acid (Japanese Patent Laid-open No. 57-18989); coryneform bacteria resistant to argininol (Japanese Patent Publication No. 62-24075); coryneform bacteria resistant to X-guanidine (X represents a derivative of fatty acid or aliphatic chain, Japanese Patent Laid-open No. 2-186995); coryneform bacteria resistant to arginine hydroxamate and 6-azauracil (Japanese Patent Laid-open No. 57-150381), and so forth.

In addition, since L-arginine, L-glutamine and L-proline comprise L-glutamic acid as a basic structure, a bacterium having ability to produce any of these amino acids may be bred by amplifying a gene encoding an enzyme that catalyses a reaction that generates any of the L-amino acids from L-glutamic acid in such L-glutamic acid-producing bacteria as mentioned above.

Further, the biosynthetic pathways of L-citrulline and L-ornithine are common to that of L-arginine, and therefore abilities to produce them can be imparted by increasing enzymatic activities of N-acetylglutamate synthase (*argA*), N-acetylglutamyl phosphate reductase (*argC*), ornithine acetyltransferase (*argJ*), N-acetylglutamate kinase (*argB*), acetylornithine transaminase (*argD*) and acetylornithine deacetylase (*argE*).

As coryneform bacteria having L-cysteine-producing ability, there is known, for example, a coryneform bacterium of which intracellular serine acetyltransferase activity is increased by having a serine acetyltransferase desensitized to the feedback inhibition by L-cysteine (Japanese Patent Laid-open No. 2002-233384).

Examples of method for imparting L-valine-producing ability include, for example, a method of modifying a bacterium so that expression of a gene encoding an enzyme involved in the L-valine biosynthesis is enhanced. Examples of the enzyme involved in the L-valine biosynthesis include enzymes encoded by the genes present on the *ilvBNC* operon, that is, acetohydroxy acid synthetase encoded by *ilvBN* and isomeroreductase encoded by *ilvC* (WO00/50624). Since the *ilvBNC* operon is subject to expression regulation of the operon by L-valine and/or L-isoleucine and/or L-leucine, it is desirable to eliminate attenuation to avoid expression suppression by L-valine that is produced.

A coryneform bacterium having L-valine-producing ability can also be obtained by decreasing or eliminating activity of at least one kind of enzyme involved in a metabolic pathway that decreases L-valine production. For example, decreasing the activity of threonine dehydratase involved in the L-leucine synthesis, or activity of an enzyme involved in the D-pantothenate synthesis is conceivable (WO00/50624).

Examples of methods for imparting L-valine-producing ability also include imparting resistance to an amino acid analogue or the like. Examples of bacteria obtained by such a method include, for example, mutant strains which are auxotrophic for L-isoleucine and L-methionine, and resistant to D-ribose, purine ribonucleoside or pyrimidine ribonucleoside, and have an ability to produce L-valine (FERM P-1841, FERM P-29, Japanese Patent Publication No. 53-025034), mutant strains resistant to polyketides (FERM P-1763, FERM P-1764, Japanese Patent Publication No. 06-065314), and mutant strains resistant to L-valine in a medium containing acetic acid as the sole carbon source and sensitive to pyruvic acid analogues (β-fluoropyruvic acid etc.) in a medium containing glucose as the sole carbon source (FERM BP-3006, BP-3007, Japanese Patent No. 3006929).

Examples of coryneform bacteria imparted with L-alanine-producing ability include, for example, coryneform bacteria lacking the H⁺-ATPase activity (Appl. Microbiol. Biotechnol., 2001 Nov., 57(4):534-40), coryneform bacteria in which aspartate β-decarboxylase gene is amplified (Japanese Patent Laid-open No. 07-163383), and so forth.

Examples of coryneform bacteria imparted with L-lysine-producing ability include lysine analogue resistant strains or metabolic regulation mutant strains having L-lysine-producing ability. Specific examples include S-(2-aminoethyl)-cysteine (henceforth abbreviated as "AEC") resistant mutant strains (*Brevibacterium lactofermentum* AJ11082 (NRRL B-11470) strain etc., refer to Japanese Patent Publication Nos. 56-1914, 56-1915, 57-14157, 57-14158, 57-30474, 58-10075, 59-4993, 61-35840, 62-24074, 62-36673, 5-11958, 7-112437 and 7-112438); mutant strains requiring an amino acid such as L-homoserine for their growth (refer to Japanese Patent Publication Nos. 48-28078 and 56-6499); mutant strains showing resistance to AEC and further requiring an amino acid such as L-leucine, L-homoserine, L-proline, L-serine, L-arginine, L-alanine and L-valine (refer to U.S. Patent Nos. 3,708,395 and 3,825,472); L-lysine-producing mutant strains showing resistance to DL-α-amino-ε-caprolactam, α-amino-laurolactam, aspartic acid analogue, sulfa drug, quinoid and N-lauroylleucine; L-lysine-producing mutant strains showing resistance to oxaloacetate decarboxylase or a respiratory tract enzyme inhibitor (Japanese Patent Laid-open Nos. 50-53588, 50-31093, 52-102498, 53-9394, 53-86089, 55-9783, 55-9759, 56-32995, 56-39778, Japanese Patent Publication Nos. 53-43591 and 53-1833); L-lysine-producing mutant strains requiring inositol or acetic acid (Japanese Patent Laid-open Nos. 55-9784 and 56-8692); L-lysine-producing mutant strains that are susceptible to fluoropyruvic acid or a temperature of 34°C or higher (Japanese Patent Laid-open Nos. 55-9783 and 53-86090); L-lysine-producing mutant strains of *Brevibacterium* or *Corynebacterium* bacteria showing resistance to ethylene glycol (U.S. Patent No. 4,411,997), and so forth.

Furthermore, a coryneform bacterium imparted with L-lysine-producing ability can also be obtained by increasing activity of an L-lysine biosynthetic enzyme. Increase of activity of such an enzyme can be attained by increasing copy number of a gene encoding the enzyme in cells, or by modifying an expression control sequence thereof.

Examples of genes encoding L-lysine biosynthetic enzymes include genes encoding enzymes of the diaminopimelate pathway such as dihydrodipicolinate synthase gene (*dapA*), aspartokinase gene (*lysC*), dihydrodipicolinate reductase gene (*dapB*), diaminopimelate decarboxylase gene (*lysA*), diaminopimelate dehydrogenase gene (*ddh*) (WO96/40934 for all the foregoing genes), phosphoenolpyrvate carboxylase gene (*ppc*) (Japanese Patent Laid-open No. 60-87788), aspartate aminotransferase gene (*aspC*) (Japanese Patent Publication No. 6-102028), diaminopimelate epimerase gene (*dapF*) (Japanese Patent Laid-open No. 2003-135066), and aspartate semialdehyde dehydrogenase gene (*asd*) (WO00/61723), genes encoding enzymes of the aminoadipic acid pathway such as homoaconitate hydratase gene (Japanese Patent Laid-open No. 2000-157276), and so forth. Coryneform bacteria modified by using these genes are disclosed in Japanese Patent Laid-open Nos. 10-215883, 10-165180, WO96/40934, etc.

As the gene encoding aspartokinase III (*lysC*), a gene modified so that the enzyme is desensitized to feedback inhibition by L-lysine is preferably used. Such a modified *lysC* gene for desensitization to the feedback inhibition can be obtained by the method described in U.S. Patent No. 5,932,453.

Furthermore, coryneform bacteria imparted with L-lysine-producing ability may have reduced activity of an enzyme that catalyzes a reaction producing a compound other than L-lysine or may be deficient in such an activity, or they may have reduced activity of an enzyme that negatively acts on L-lysine production, or may be deficient in such an activity. Examples of such enzymes include homoserine dehydrogenase, lysine decarboxylase (*cadA, ldcC*), and malic enzyme, and strains in which activities of these enzymes are decreased or deleted are disclosed in WO95/23864, and so forth.

Preferred examples of coryneform bacteria imparted with L-tryptophan-producing ability are bacteria in which one or two or more activities among the anthranilate synthetase activity, phosphoglycerate dehydrogenase activity, and tryptophan synthase activity are enhanced. Since anthranilate synthetase and phosphoglycerate dehydrogenase suffer from feedback inhibition by L-tryptophan and L-serine, respectively, the enzymatic activities thereof can be enhanced by making a bacterium have a desensitized type mutant enzyme.

Further, L-tryptophan-producing ability can also be imparted by introducing a recombinant DNA containing the tryptophan operon. Moreover, L-tryptophan-producing ability may be improved or imparted by enhancing expression of a gene encoding tryptophan synthase in the tryptophan operon (*trpBA*). Tryptophan synthase consists of α and β subunits, which are encoded by the *trpA* and *trpB* genes, respectively. The nucleotide sequence of the tryptophan operon and the nucleotide sequences of *trpA* and *trpB* are registered as GenBank Accession No. J01714 (WO2005/103275).

Examples of coryneform bacteria imparted with L-tryptophan-producing ability include *Brevibacterium flavum* AJ11667 (refer to Japanese Patent Laid-open No. 57-174096).

Examples of coryneform bacteria imparted with L-tyrosine-producing ability include *Corynebacterium glutamicum* AJ11655 (FERM P-5836, refer to Japanese Patent Publication No. 2-6517), and *Brevibacterium lactofermentum* AJ12081 (FERM P-7249, refer to Japanese Patent Laid-open No. 60-70093).

Examples of coryneform bacteria having L-phenylalanine-producing ability include the strain showing tyrosine auxotrophy and L-phenylalanyl-L-tyrosine resistance (Japanese Patent Laid-open No. 5-49489) and *Brevibacterium lactofermentum* AJ12637 (FERM BP-4160, refer to the French Patent Laid-open No. 2,686,898).

L-Tryptophan, L-phenylalanine, and L-tyrosine are all aromatic amino acids and share a common biosynthesis pathway. Examples of the genes encoding the biosynthetic enzymes for these aromatic amino acids include deoxyarabino-heptulosonate phosphate synthase (*aroG*), 3-dehydroquinate synthase (*aroB*), shikimic acid dehydratase, shikimate kinase (*aroL*), 5-enolpyruvylshikimate-3-phosphate synthase (*aroA*), and chorismate synthase (*aroC*) (European Patent Laid-open No. 763127). Therefore, an ability to produce an aromatic amino acid can be improved by increasing copy number of a gene encoding any of these enzymes on a plasmid or genome. It is known that these genes are controlled by the tyrosine repressor (*tyrR*), and so activity of an aromatic amino acid biosynthetic enzyme may also be increased by deleting the *tyrR* gene (see European Patent Laid-open No. 763127).

Further, examples of coryneform bacteria having L-threonine-producing ability include *Corynebacterium acetoacidophilum* AJ12318 (FERM BP-1172, refer to U.S. Patent No. 5,188,949), and so forth.

Examples of coryneform bacteria imparted with L-leucine-producing ability include *Brevibacterium lactofermentum* AJ3718 (FERM P-2516, 2-thiazolealanine and β-hydroxyleucine resistant, and isoleucine and methionine auxotrophic).

Examples of coryneform bacteria having L-isoleucine-producing ability include *Brevibacterium flavum* AJ12149 (FERM BP-759, refer to U.S. Patent No. 4,656,135), and so forth.

### <1-2> Enhancement of carbonic anhydrase activity

By modifying such coryneform bacteria imparted with an L-amino acid-producing ability as mentioned above so that carbonic anhydrase activity thereof is enhanced, coryneform bacteria used for the production method of the present invention can be obtained. However, either the modification for enhancing the carbonic anhydrase activity or the impartation of an L-amino acid-producing ability may be performed first.

The expression "has been modified to enhance carbonic anhydrase activity" include a state where the number of carbonic anhydrase molecules per cell has been increased, as well as a state where the activity per molecule of carbonic anhydrase has been increased, compared with a parent or wild-type strain, or the like. Further, the wild-type strain used as the object of the comparison may be, for example, the *Corynebacterium glutamicum* (*Brevibacterium lactofermentum*) ATCC 13869 strain or ATCC 13032 strain.

The enhancement of the carbonic anhydrase activity can be confirmed by comparing the carbonic anhydrase activity or amount of mRNA of a gene encoding the carbonic anhydrase with that of a wild-type or unmodified strain. Examples of method for confirming expression amount include Northern hybridization and RT-PCR (Molecular Cloning, Cold Spring Harbor Laboratory Press, Cold Spring Harbor, USA, 2001). The enzyme activity or expression amount can be increased to any level so long as it is increased compared with that of a wild-type or unmodified strain, and for example, it is desirably increased not less than 1.5 times, more preferably not less than 2 times, still more preferably not less than 3 times, as compared with that of, for example, a wild-type or an unmodified strain.

Carbonic anhydrase is an enzyme involved in the mutual conversion of carbon dioxide and bicarbonate radical (EC 4.2.1.1). The carbonic anhydrase activity can be measured by the method of Wilbur et al, (Wilbur K.M., Anderson N.G., Electrometric and Colorimetric Determination of Carbonic Anhydrase, J. Biol. Chem., 176:147-154, 1948).

As genes encoding carbonic anhydrase (*ca* gene) of coryneform bacteria, two kinds of genes, the gene encoding β type carbonic anhydrase (*bca*) and gene encoding γ type carbonic anhydrase (*gca*), have been reported. More preferably, NCg12579 of the *C*. *glutamicum* ATCC 13032 strain registered at GenBank (corresponding to *bca,* complementary strand of 2837954 to 2838577 of Accession BA_000036.3) can be used. The nucleotide sequence of the gene is shown as SEQ ID NO: 13 (coding region corresponds to the nucleotide numbers 1 to 621), and the amino acid sequence of the encoded protein is shown as SEQ ID NO: 14. Further, the nucleotide sequence of the *bca* gene of the *C*. *glutamicum* ATCC 13869 strain is shown as the nucleotide numbers 562 to 1182 in SEQ ID NO: 11, and the amino acid sequence of the encoded protein is shown as SEQ ID NO: 12.

Further, as the gene that can be used for the present invention, a homologue of the *ca* gene derived from another microorganism may be used, so long as it can express a protein that shows the carbonic anhydrase activity in coryneform bacteria. Such a homologue of the *ca* gene can be searched for by using BLAST or the like with reference to the nucleotide sequence of the nucleotide numbers 562 to 1182 of SEQ ID NO: 11 or the nucleotide sequence of SEQ ID NO: 13 (http:/blast.genome.jp/).

Since the sequence of the *bca* gene that can be used for the present invention has already been elucidated, a region including *bca* and a control region of *bca* can be obtained by PCR using primers produced on the basis of the above nucleotide sequence, for example, the primers shown as SEQ ID NOS: 7 and 8, and a chromosomal DNA of a coryneform bacterium as a template. Homologues of *bca* of other microorganisms can also be obtained in a similar manner.

Further, since the nucleotide sequence of the *bca* gene can differ depending on the species or strain of coryneform bacteria, the *bca* gene used for the present invention is not limited to the nucleotide sequence of nucleotide numbers 562 to 1182 of SEQ ID NO: 11 or the nucleotide sequence of SEQ ID NO: 13, but it can be a mutant or artificially modified gene that codes for a protein having the sequence of SEQ ID NO: 12 or 14, but which includes substitutions, deletions, insertions, additions, etc. of one or several amino acid residues at one or more positions so long as the protein has the carbonic anhydrase activity. Although the number meant by the term "one or several" can differ depending on positions in the three-dimensional structure of the protein or types of amino acid residues, specifically, it can be 1 to 20, preferably 1 to 10, more preferably 1 to 5. The substitutions, deletions, insertions, additions, inversions or the like of amino acid residues described above can also include those caused by a naturally occurring mutation based on individual differences, or differences in species of microorganisms that contain the *bca* gene (mutant or variant).

The aforementioned substitution is preferably a conservative substitution that is a neutral substitution, that is, one that does not result in a functional change. The conservative mutation is a mutation wherein substitution takes place mutually among Phe, Trp and Tyr, if the substitution site is an aromatic amino acid; among Leu, Ile and Val, if the substitution site is a hydrophobic amino acid; between Gln and Asn, if it is a polar amino acid; among Lys, Arg and His, if it is a basic amino acid; between Asp and Glu, if it is an acidic amino acid; and between Ser and Thr, if it is an amino acid having hydroxyl group. Specific examples of conservative substitutions include: substitution of Ser or Thr for Ala; substitution of Gln, His or Lys for Arg; substitution of Glu, Gln, Lys, His or Asp for Asn; substitution of Asn, Glu or Gln for Asp; substitution of Ser or Ala for Cys; substitution of Asn, Glu, Lys, His, Asp or Arg for Gln; substitution of Gly, Asn, Gln, Lys or Asp for Glu; substitution of Pro for Gly; substitution of Asn, Lys, Gln, Arg or Tyr for His; substitution of Leu, Met, Val or Phe for Ile; substitution of Ile, Met, Val or Phe for Leu; substitution of Asn, Glu, Gln, His or Arg for Lys; substitution of Ile, Leu, Val or Phe for Met; substitution of Trp, Tyr, Met, Ile or Leu for Phe; substitution of Thr or Ala for Ser; substitution of Ser or Ala for Thr; substitution of Phe or Tyr for Trp; substitution of His, Phe or Trp for Tyr; and substitution of Met, Ile or Leu for Val.

Furthermore, the *bca* gene can include a nucleotide sequence encoding a protein having an identity not less than 80%, preferably not less than 90%, more preferably not less than 95%, particularly preferably not less than 97%, to the entire amino acid sequence of SEQ ID NO: 12 or 14, and having the carbonic anhydrase activity. Furthermore, the degree of degeneracy of the gene can vary depending on the host into which the *bca* gene is introduced, and therefore codons can be replaced with those which are favorable for the chosen host.

Moreover, the *bca* gene can code for a protein with an elongated or deleted Nor C-terminal sequence, so long as the protein has the carbonic anhydrase activity. The length of the amino acid sequence to be elongated or deleted can be 50 amino acid residues or less, preferably 20 or less, more preferably 10 or less, particularly preferably 5 or less. More specifically, the *bca* gene can encode a protein having the amino acid sequence of SEQ ID NO: 12 or 24, but wherein the sequence is elongated by 5 to 50 amino acid residues on the N-terminal or C-terminal side, or 5 to 50 residues are deleted on either side.

Such genes homologous to the *bca* gene described above can be obtained by modifying the nucleotide sequence of nucleotide numbers 562 to 1182 of SEQ ID NO: 11 or the nucleotide sequence of SEQ ID NO: 13 so that the protein encoded by the gene includes substitutions, deletions, insertions, or additions of amino acid residues at a specific site(s), for example, by site-specific mutagenesis. Furthermore, homologous genes can also be obtained by conventionally known mutation treatments, such as those described below. For example, the nucleotide sequence mentioned above can be treated with hydroxylamine or the like *in vitro,* or the microorganism, for example, coryneform bacteria, containing the gene can be treated with ultraviolet ray irradiation or a mutagen typically used for mutation, such as N-methyl-N'-nitro-N-nitrosoguanidine (NTG) or ethyl methanesulfonate (EMS), or a mutation can be artificially introduced into *bca* by genetic recombination based on error-prone PCR, DNA shuffling, or StEP-PCR, so that a highly active *bca* gene can be obtained (Firth A.E., Patrick W.M., Bioinformatics, 2005 Jun 2, Statistics of Protein Library Construction).

Examples of the *bca* gene also include a DNA that hybridizes with a complement of the nucleotide sequence of nucleotide numbers 562 to 1182 of SEQ ID NO: 11 or the nucleotide sequence of SEQ ID NO: 13, or a probe that can be prepared from these sequences under stringent conditions and codes for a protein which has the carbonic anhydrase activity. The "stringent conditions" can be conditions under which a so-called specific hybrid is formed, and non-specific hybrid is not formed. Examples include, for example, conditions under which DNAs having high homology to each other, for example, DNAs having a homology of, for example, not less than 80%, not less than 90%, not less than 95%, or not less than 97%, hybridize with each other, and DNAs having homology lower than the above levels do not hybridize with each other. "Stringent conditions" can also include washing conditions which are typical in Southern hybridization, for example, washing once, or twice or three times, at salt concentrations and a temperature of 1 x SSC, 0.1% SDS at 60°C, preferably 0.1 x SSC, 0.1% SDS at 60°C, more preferably 0.1 x SSC, 0.1% SDS at 68°C.

A partial sequence of the nucleotide sequence of the nucleotide numbers 562 to 1182 of SEQ ID NO: 11 or the nucleotide sequence of SEQ ID NO: 13 can also be used as the probe. Such a probe can be prepared by PCR using oligonucleotides prepared on the basis of either of these nucleotide sequences as primers and a DNA fragment containing any one of the nucleotide sequence of the nucleotide numbers 562 to 1182 of SEQ ID NO: 11 and the nucleotide sequence of SEQ ID NO: 13 as a template. When a DNA fragment having a length of about 300 bp is used as the probe, for example, the washing conditions after hybridization under the aforementioned conditions can be exemplified by 2 x SSC, 0.1% SDS at 50°C.

Expression amount of the *bca* gene can be enhanced by increasing copy number of the *bca* gene. For example, the copy number of the gene can be increased by ligating a fragment containing the *bca* gene to a vector, preferably a multi copy vector, that functions in coryneform bacteria, to prepare a recombinant DNA, and transforming such a microorganism having an L-amino acid-producing ability as mentioned above with the DNA. Alternatively, after the transformation of a wild-type strain of a coryneform bacterium by introducing such a recombinant DNA as mentioned above, the ability to produce an L-amino acid can be imparted to the transformed bacterium. The copy number of the gene can also be increased by transferring a single copy or multiple copies of the *bca* gene to the bacterial chromosome. Transfer of the *bca* gene to the chromosome can be confirmed by Southern hybridization using a portion of the *bca* gene as a probe.

Expression of the *bca* gene can also be enhanced by modifying an expression control sequence of the *bca* gene. For example, the promoter sequence of the *bca* gene can be replaced with a stronger promoter, or by making a promoter sequence closer to a consensus sequence (WO00/18935).

Methods for modifying a coryneform bacterium to enhance carbonic anhydrase activity are explained below. These methods can be performed as described in a manual such as Molecular Cloning (Cold Spring Harbor Laboratory Press, Cold Spring Harbor (USA), 2001).

Expression amount of the *bca* gene can be increased by increasing copy number of the *bca* gene, and the copy number can be increased by amplifying the *bca* gene using a plasmid as described below. First, the *bca* gene is cloned from chromosome of a coryneform bacterium. Chromosomal DNA can be prepared from a bacterium as a DNA donor, for example, by the method of Saito and Miura (see H. Saito and K. Miura, Biochem. Biophys. Acta, 72, 619, 1963; Text for Bioengineering Experiments, Edited by the Society for Bioscience and Bioengineering, Japan, 97-98, Baifukan Co., Ltd., 1992), or the like. Oligonucleotides for use in PCR can be synthesized on the basis of the aforementioned known information, and for example, the synthetic oligonucleotides shown in SEQ ID NOS: 7 and 8 can be used to amplify the *bca* gene.

A gene fragment including the *bca* gene amplified by PCR can be ligated to a vector DNA autonomously replicable in cells of *Escherichia coli* and/or coryneform bacteria to prepare a recombinant DNA, and this recombinant DNA can be introduced into *Escherichia coli,* which makes the operation thereafter easier. Examples of vectors autonomously replicable in a cell of *Escherichia coli* include pUC19, pUC18, pHSG299, pHSG399, pHSG398, RSF1010, pBR322, pACYC184, pMW219, and so forth.

The aforementioned DNA is introduced into a vector that functions in coryneform bacteria. The vector that functions in coryneform bacteria is, for example, a plasmid autonomously replicable in coryneform bacteria. Specific examples of the plasmid that is autonomously replicable in coryneform bacteria include plasmid pCRY30 described in Japanese Patent Laid-open No. 3-210184; plasmids pCRY21, pCRY2KE, pCRY2KX, pCRY31, pCRY3KE, and pCRY3KX described in Japanese Patent Laid-open No. 2-72876 and U.S. Patent No. 5,185,262; plasmids pCRY2 and pCRY3 described in Japanese Patent Laid-open No. 1-191686; pAM330 described in Japanese Patent Laid-open No. 58-67679; pHM1519 described in Japanese Patent Laid-open No. 58-77895; pAJ655, pAJ611, and pAJ1844 described in Japanese Patent Laid-open No. 58-192900; pCG1 described in Japanese Patent Laid-open No. 57-134500; pCG2 described in Japanese Patent Laid-open No. 58-35197; pCG4, pCG11 etc. described in Japanese Patent Laid-open No. 57-183799; and pVK7 described in Japanese Patent Laid-open No. 10-215883.

Further, if a DNA fragment which enables a plasmid to autonomously replicate in coryneform bacteria is excised from any of those vectors and the fragment is inserted into any of the aforementioned vectors for *Escherichia coli,* the resultant can be used as a so-called shuttle vector which is autonomously replicable both in *Escherichia coli* and coryneform bacteria.

To prepare a recombinant DNA by ligation of the *bca* gene to a vector that functions in coryneform bacteria, the vector is digested with a restriction enzyme suitable for the ends of the gene. Such a restriction enzyme site may be introduced in advance into a synthetic oligonucleotide to be used for amplifying the *bca* gene. Ligation is usually performed by using a ligase such as T4 DNA ligase.

In order to introduce a recombinant plasmid prepared as described above into a coryneform bacterium, any known transformation method reported so far can be employed. For example, recipient cells can be treated with calcium chloride so as to increase permeability for the DNA, which has been reported for *Escherichia coli* K-12 (Mandel, M. and Higa, A., J. Mol. Biol., 53, 159, 1970). Also, competent cells can be prepared from growing cells and DNA can be introduced into these cells, which has been reported for *Bacillus subtilis* (Duncan, C.H., Wilson, G.A. and Young, F.E., Gene, 1, 153, 1977). Another method is to make DNA recipient cells into protoplasts or spheroplasts which easily take up a recombinant DNA, and a recombinant DNA can be introduced into these cells, which are known for *Bacillus subtilis,* actinomycetes, and yeasts (Chang, S. and Choen, S.N., Mol. Gen. Genet., 168, 111, 1979; Bibb, M.J., Ward, J.M. and Hopwood, O.A., Nature, 274, 398, 1978; Hinnen, A., Hicks, J.B. and Fink, G.R., Proc. Natl. Acad. Sci. USA, 75, 1929, 1978). In addition, coryneform bacteria can also be transformed by the electric pulse method (Japanese Patent Laid-open No. 2-207791) or by the conjugal transfer method (Biotechnology (NY). 1991 January; 9(1):84-7).

The copy number of the *bca* gene can also be increased by integrating multiple copies of the *bca* gene into the chromosomal DNA of the coryneform bacterium, which can be accomplished by homologous recombination. This technique is performed by targeting a sequence which is present in multiple copies on the chromosomal DNA. Such sequences can include a repetitive DNA or inverted repeats present at the end of a transposable element. Alternatively, as disclosed in Japanese Patent Laid-open No. 2-109985, multiple copies of the *bca* gene can be introduced into a chromosomal DNA by incorporating them into a transposon, and transferring the transposon (Japanese Patent Laid-open Nos. 2-109985, 7-107976; Mol. Gen. Genet., 245, 397-405, 1994; Plasmid, 2000 November; 44(3): 285-91).

Also conceivable is a method of inserting the *bca* gene into a plasmid having a replication origin that cannot be replicated in a host, or a plasmid having a replication origin that cannot replicate in a host and an ability to cause conjugal transfer to a host, and introducing the obtained plasmid into the host to amplify the gene on a chromosome. Examples of such a plasmid include pSUP301 (Simo et al., Bio/Technology 1, 784-791, 1983), pK18mob or pK19mob (Schaefer et al., Gene, 145, 69-73, 1994), pGEM-T (Promega Corporation, Madison, WI, USA), pCR2.1-TOPO (Shuman, Journal of Biological Chemistry, 269:32678-84, 1994; U.S. Patent No. 5,487,993), pCR Blunt (Invitrogen, Groningen, Netherlands; Bernard et al., Journal of Molecular Biology, 234: 534-541, 1993), pEM1 (Schrumpf et al., Journal of Bacteriology, 173:4510-4516, 1991), pBGS8 (Spratt et al., Gene, 41:337-342, 1986), and so forth. A plasmid vector comprising the *bca* gene is transferred into a coryneform bacterium by conjugation or transformation to transfer the gene into a chromosome. The conjugation method is described by, for example, Schaefer et al. (Applied and Environmental Microbiology, 60, 756-759, 1994). The transformation method is described by, for example, Theirbach et al. (Applied Microbiology and Biotechnology, 29, 356-362, 1988), Dunican and Shivinan (Bio/Technology 7, 1067-1070, 1989), and Tauch et al. (FEMS Microbiological Letters, 123, 343-347, 1994).

The activity of Bca can also be enhanced by replacing a native expression control sequence, such as a promoter, of the *bca* gene, on the chromosomal DNA or a plasmid with a stronger one. Other methods include modifying a factor involved in expression control of the *bca* gene, such as operator or repressor, or ligating a strong terminator (Hamilton et al., Journal of Bacteriology 171:4617-4622). For example, the lac promoter, trp promoter, trc promoter, PS2 promoter, and so forth are known as strong promoters. Methods for evaluating the strength of promoters and examples of strong promoters are described in the paper of Goldstein et al. (Prokaryotic Promoters in Biotechnology, Biotechnol. Annu. Rev., 1, 105-128, 1995), and so forth. Furthermore, as disclosed in WO00/18935, strength of a promoter can be increased by making several nucleotide substitutions in the promoter region of a target gene so as to make the sequence closer to a consensus sequence. For example, the -35 region can be replaced with TTGACA or TTGCCA, and the -10 region can be replaced with TATAAT or TATAAC. In addition, it is known that the translation efficiency of mRNA is significantly affected by substituting several nucleotides in the spacer region between the ribosome-binding site (RBS) and the translation initiation codon, in particular, the sequence immediately upstream of the initiation codon, and they can be modified.

Examples of the upstream region of the *bca* gene include, for example, the region of the nucleotide numbers 1 to 561 of SEQ ID NO: 11. An expression control sequence such as promoter upstream of the *bca* gene may also be identified by using a promoter search vector or gene analysis software such as GENETYX. By such substitution or modification of promoter as described above, expression of the *bca* gene is enhanced. Substitution of an expression control sequence can be attained by, for example, using a temperature sensitive plasmid. Modification of an expression control sequence may be combined with increase of copy number of the *bca* gene.

Increase of expression amount can also be attained by extending the survival time of the mRNA or by preventing degradation of the encoded protein in the cells.

As genes encoding carbonic anhydrase of *Escherichia coli, yadF* and *cynT* genes encoding two kinds of carbonic anhydrases, Can (carbonic anhydrase 2) and CynT (carbonic anhydrase 1), respectively, have been reported, and these can also be used instead of the aforementioned *bca* gene. As *yadF, yadF* of *Escherichia coli* registered at GenBank (Accession EG_12319) is exemplified. The nucleotide sequence of this gene is shown as SEQ ID NO: 27 (coding region: 201 to 860), and the encoded amino acid sequence is shown as SEQ ID NO: 28. As the *cynT* gene, *cynT* of *Escherichia coli* (Accession EG_10176) is exemplified. The nucleotide sequence of this *cynT* gene is shown as SEQ ID NO: 29 (coding region: 201 to 857), and the encoded amino acid sequence is shown as SEQ ID NO: 30. A gene encoding a protein showing 80% or more, preferably 90% or more, more preferably 95% or more, particularly preferably 97% or more, of identity for the full length of any of these amino acid sequences, and having the carbonic anhydrase activity can also be used.

The microorganism used for the production method of the present invention may be a microorganism modified to impart D-xylose-5-phosphate phosphoketolase activity and/or fructose-6-phosphate phosphoketolase activity in addition to enhancing the carbonic anhydrase activity.

Either one or both of the D-xylose-5-phosphate phosphoketolase activity and fructose-6-phosphate phosphoketolase activity may be imparted. In this specification, D-xylose-5-phosphate phosphoketolase and fructose-6-phosphate phosphoketolase may be collectively referred to as phosphoketolase.

The D-xylose-5-phosphate phosphoketolase activity means an activity for converting xylose-5-phosphate into glycelaldehyde-3-phosphate and acetyl phosphate with consuming phosphoric acid to release one molecule of H₂O. This activity can be measured by the method described by Goldberg, M. et al. (Methods Enzymol., 9, 515-520, 1996) or by the method described by L. Meile (J. Bacteriol., 183:2929-2936, 2001).

The fructose-6-phosphate phosphoketolase activity means an activity for converting fructose-6-phosphate into erythrose-4-phosphate and acetyl phosphate with consuming phosphoric acid to release one molecule of H₂O. This activity can be determined by the method described by Racker, E. (Methods Enzymol., 5, 276-280, 1962) or by the method described by L. Meile (J. Bacteriol., 183:2929-2936, 2001).

The phosphoketolase activity can be imparted by introducing a gene encoding a phosphoketolase into cells of a coryneform bacterium by using a plasmid, by incorporating such a gene into chromosome of a coryneform bacterium, or the like.

Coryneform bacteria do not inherently have the phosphoketolase activity, but the phosphoketolase activity can be imparted by introducing a plasmid containing a gene encoding a phosphoketolase derived from another organism into cells of a coryneform bacterium, by incorporating a gene encoding a phosphoketolase derived from another organism into chromosome of a coryneform bacterium, or the like.

A gene encoding D-xylose-5-phosphate phosphoketolase can be obtained by PCR using chromosomal DNA of a microorganism having the D-xylose-5-phosphate phosphoketolase activity as a template, or the like. Examples of such a microorganism include bacteria such as lactic acid bacteria, methanol-assimilating bacteria, methane-assimilating bacteria, bacteria belonging to the genus *Streptococcus, Acetobacter, Bifidobacterium, Lactobacillus, Thiobacillus, Methylococcus, Butyrivibrio, Fibrobacter* or the like; yeasts belong to the genus *Candida, Rhodotorula, Rhodosporidium, Pichia, Yarrowia, Hansenula, Kluyveromyces, Saccharomyces, Trichosporon, Wingea,* or the like, etc.

A gene encoding fructose-6-phosphate phosphoketolase can be obtained by PCR using chromosomal DNA of a microorganism having the fructose-6-phosphate phosphoketolase activity as a template, or the like. Examples of such a microorganism include bacteria belonging to the genus *Acetobacter, Bifidobacterium, Chlorobium, Brucella, Methylococcus, Gardnerella,* or the like; yeasts belong to the genus *Candida, Rhodotorula, Saccharomyces,* or the like, etc.

Specific example of the gene encoding D-xylose-5-phosphate phosphoketolase include the *xpkA* gene encoding D-xylose-5-phosphate phosphoketolase of *Lactobacillus pentosus* MD363. The nucleotide sequence thereof is registered at the EMBL/GenBank database with an accession number of AJ309011 (Posthuma, CC. et al, Appl. Environ. Microbiol., 68(2), 831-7, 2002, SEQ ID NO: 15).

The *xpkl* gene derived from *Lactobacillus plantarum* can also be used. The nucleotide sequence thereof is registered at the EMBL/GenBank database with an accession number of NC_004567 Region: complement (2362936 to 2365302) (Kleerebezem, M., et al, Proc. Natl. Acad. Sci. U.S.A. 100 (4), 1990-1995, 2003, SEQ ID NO: 17).

In addition, examples of homologues of these genes include a gene of *Lactobacillus plantarum* as GenBank Accession No. NC_004567 complement (3169067 to 3171478), a gene of *Streptococcus agalactiae* encoding the amino acid sequence of GenBank Accession No. NP_736274, a gene of *Lactococcus lactis* subsp. *Lactis* encoding the amino acid sequence of GenBank Accession No. NP_267658, a gene of *Lactobacillus johnsonii* which is registered as GenBank Accession No. NC_005362 (696462 to 698867), a gene of *Lactobacillus acidophilus* encoding the amino acid sequence of GenBank Accession No. YP_193510, and so forth.

A gene encoding a protein having the activities of both D-xylose-5-phosphate phosphoketolase and fructose-6-phosphate phosphoketolase can also be used. Examples of such a gene include the *xfp* gene of *Bifidobacterium lactis.* The nucleotide sequence thereof is registered at the EMBL/GenBank database as accession number of AJ293946 (Meile, L. et al, J. Bacteriol., 183(9), 2929-36, 2001, SEQ ID NO: 19).

As homologues of the *xfp* gene, there can also be used a gene of *Bifidobacterium longum* encoding the amino acid sequence of GenBank Accession No. NP_696135, a gene of *Chlorobium tepidum* encoding the amino acid sequence of GenBank Accession No. NP_662409, a gene of *Brucella suis* encoding the amino acid sequence of GenBank Accession No. NP_699578, a gene of *Brucella abortus* encoding the amino acid sequence of GenBank Accession No. YP_223570, and so forth.

In addition, the phosphoketolase gene may be a DNA hybridizable with a complement of any of the aforementioned nucleotide sequences or a probe that can be prepared from the complement under stringent conditions, and encoding a protein having the phosphoketolase activity. Further, there may be also used a DNA encoding a protein showing 80% or more, preferably 90% or more, more preferably 95% or more, particularly preferably 97% or more, of identity to the full length of the amino acid sequence of SEQ ID NO: 16, 18, or 20, and having the phosphoketolase activity.

The microorganism used for the production method of the present invention may be a microorganism modified to enhance phosphotransacetylase activity as compared with a wild-type strain, in addition to the enhancement of the carbonic anhydrase activity. The phosphotransacetylase is an enzyme involved in the acetic acid metabolism. In *Escherichia coli,* it is responsible for the reaction that generates acetyl phosphate from phosphoric acid and acetyl-CoA, which is a part of the main pathway of the acetic acid generation. It is known that, on the other hand, in *Corynebacterium glutamicum,* the activity of phosphotransacetylase increases when acetic acid is assimilated, and acetyl-CoA is generated. Moreover, it is also known that RamB, which is a transcription factor, is involved in the negative control of the phosphotransacetylase activity (Microbiology, 145, 503-513, 1999; Journal of Bacteriology, 186, 9, 2798-2809, 2004). The phosphotransacetylase activity can be enhanced by increasing copy number of a gene encoding phosphotransacetylase, modifying a promoter of a gene encoding phosphotransacetylase, or the like, as in the case of the enhancement of the phosphotransacetylase activity mentioned above. Further, the enhancement may also be attained by deleting the *ramB* gene mentioned above, or modifying the RamB protein-binding site located upstream of the gene encoding phosphotransacetylase.

As the gene encoding phosphotransacetylase (*pta* gene) of a coryneform bacterium, the nucleotide sequence NCgl2657 of ATCC 13032 registered at Genbank (complementary strand of 2936506 to 2937891 of Accession NC 003450.3) can be used. The nucleotide sequence of this gene is shown as SEQ ID NO: 21, and the encoded amino acid sequence is shown as SEQ ID NO: 22, respectively. Furthermore, the nucleotide sequence of the *pta* gene of the *C*. *glutamicum* ATCC 13869 strain is shown as the nucleotide numbers 1214 to 2641 in SEQ ID NO: 23, and the amino acid sequence encoded by the gene is shown as SEQ ID NO: 24.

Furthermore, as the gene of the present invention, a homologue gene of the *pta* gene derived from another microorganism may be used so long as it encodes a protein showing the phosphotransacetylase activity in coryneform bacteria. Such a homologue of the *pta* gene can be searched for by using BLAST (http://blast.genome.jp/) or the like with reference to the nucleotide sequence of the nucleotides numbers 1214 to 2641 of SEQ ID NO: 23.

The nucleotide sequence of the *pta* gene that can be used for the present invention has already been elucidated. Therefore, a region containing the *pta* gene and a expression control sequence thereof can be obtained by PCR (polymerase chain reaction, refer to White, T.J. et al., Trends Genet. 5, 185, 1989) using primers prepared on the basis of the known nucleotide sequence, for example, the primers of SEQ ID NOS: 25 and 26, and chromosomal DNA of a coryneform bacterium as a template. A homologue of the *pta* gene derived from another microorganism can also be obtained in the same manner.

The nucleotide sequence of the *pta* gene can differ depending on the species or strain of coryneform bacteria, the *pta* gene used for the present invention is not limited to the nucleotide sequence of the nucleotide numbers 1214 to 2641 of SEQ ID NO: 23 or the nucleotide sequence of SEQ ID NO: 21, and it can be a mutant or artificially modified gene that codes for a protein having the sequence of SEQ ID NO: 24 or 22, but which includes substitutions, deletions, insertions, additions, etc. of one or several amino acid residues at one or more positions so long as the encoded protein has the function of the Pta protein, the phosphotransacetylase activity. Although the number meant by the term "one or several" can differ depending on positions in the three-dimensional structure of the protein or types of amino acid residues, specifically, it can be 1 to 20, preferably 1 to 10, more preferably 1 to 5. The substitutions, deletions, insertions, additions, inversions or the like of amino acid residues described above can also include those caused by a naturally occurring mutation based on individual differences, differences in species of microorganisms that contain the *pta* gene (mutant or variant), or the like.

Further, a DNA encoding a protein showing an identity not less than 80%, preferably not less than 90%, more preferably not less than 95%, particularly preferably not less than 97%, to the entire amino acid sequence of SEQ ID NO: 24 or 22, and having the phosphotransacetylase activity can also be used.

The coryneform bacterium used for the production method of the present invention may be a microorganism modified to enhance pyruvate carboxylase activity as compared with a wild-type strain, in addition to the aforementioned modifications. As the pyruvate carboxylase gene, for example, genes derived from coryneform bacteria and *Bacillus* bacteria can be used, and the *pyc* gene of the *C*. *glutamicum* ATCC 13032 strain (GenBank Accession No. NCgl0659) and the *pyc* gene of *B. subtilis* (European Patent No. 1092776) can be used.

The coryneform bacterium used for the production method of the present invention may be a bacterium modified to enhance phosphoenolpyruvate carboxylase activity as compared with a wild-type strain, in addition to the aforementioned modifications. As the phosphoenolpyruvate carboxylase gene, for example, genes derived from coryneform bacteria and *Escherichia* bacteria can be used, and the *ppc* gene of the *C*. *glutamicum* ATCC 13032 strain (GenBank Accession No. NCgl1523) and the *ppc* gene derived from the *E. coli* MG1655 strain (GenBank Accession No. NP_418391) can be used.

Since the phosphoenolpyruvate carboxylase may suffer from feedback inhibition by aspartic acid, it is preferably modified so that it is desensitized to the feedback inhibition by aspartic acid (European Patent No. 0723011).

### <2> Method for producing L-amino acid

An L-amino acid selected from L-glutamic acid, L-glutamine, L-proline, L-arginine, L-alanine and L-aspartic acid can be produced by culturing a coryneform bacterium obtained as described above in a medium to produce and accumulate the L-amino acid in the medium, and collecting the L-amino acid from the medium.

As the medium used for the culture, a typical medium containing a carbon source, nitrogen source, and mineral salts as well as organic trace nutrients such as amino acids and vitamins as required may be used. Either a synthetic medium or a natural medium may be used. Any kind of carbon source and nitrogen source may be used for the medium so long as they can be utilized by the chosen strain to be cultured.

As the carbon source, sugars such as glucose, glycerol, fructose, sucrose, maltose, mannose, galactose, starch hydrolysates and molasses can be used. In addition, organic acids such as acetic acid and citric acid, and alcohols such as ethanol can also be used each alone or in combination with other carbon sources. As the nitrogen source, ammonia, ammonium salts such as ammonium sulfate, ammonium carbonate, ammonium chloride, ammonium phosphate and ammonium acetate, nitric acid salts and so forth can be used. As the organic trace nutrients, amino acids, vitamins, fatty acids, nucleic acids, those containing those substances such as peptone, casamino acid, yeast extract, and soybean protein decomposition product, and so forth can be used. When an auxotrophic mutant strain that requires an amino acid or the like for its growth is used, the required nutrient is preferably supplemented. As the inorganic salts, phosphoric acid salts, magnesium salts, calcium salts, iron salts, manganese salts and so forth can be used.

The culture may be performed as an aerobic culture, while the fermentation temperature is preferably controlled to be 20 to 45°C, and pH to be 3 to 9. When the pH decreases during the culture, calcium carbonate may be added, or culture is neutralized with an alkaline substance such as ammonia gas. The target L-amino acid such as L-glutamic acid is accumulated in a marked amount in the culture medium after, for example, 10 to 120 hours of the culture under such conditions as described above.

Moreover, when L-glutamic acid is produced, the culture can be performed by precipitating L-glutamic acid in a medium by using, as the medium, a liquid medium adjusted to satisfy a condition under which L-glutamic acid is precipitated. Examples of the conditions under which L-glutamic acid is precipitated include, for example, pH of 5.0 to 4.0, preferably pH 4.5 to 4.0, more preferably pH 4.3 to 4.0, particularly preferably pH 4.0 (European Patent Laid-open No. 1078989).

Collection of the L-amino acid from the culture medium after the culture may be performed by a known collection method. For example, after the cells were removed from the culture medium, the L-amino acid can be collected by concentrating the medium to crystallize the L-amino acid, ion exchange chromatography, or the like. When the culture is performed under such conditions that L-glutamic acid is precipitated, L-glutamic acid which precipitates in the medium can be collected by centrifugation or filtration. In this case, L-glutamic acid which dissolves in the medium may be crystallized and then separated together with already precipitated L-glutamic acid.

### Example

Hereafter, the present invention will be specifically explained with reference to an example. However, the present invention is not limited to the following example.

### 1. Construction of bca-amplified C. glutamicum ATCC 13869 strain

A *sucA*-deficient strain was used as a parent strain for *bca* gene amplification. A *sucA*-deficient strain can be constructed by the method described below.

### (1-1) Construction of sucA-deficient strain

A *sucA*-deficient strain of ATCC 13869 (ATCC13869ΔsucA) was constructed as follows.

The *sucA* gene encoding the E10 subunit of α-ketoglutarate dehydrogenase was disrupted by using a plasmid pBS3 carrying the *sacB* gene encoding levansucrase. For construction of a *sacB*-carrying vector for gene disruption, pBS3 described in International Patent Publications WO2005/113745 and WO2005/113744 was used.

A gene fragment that includes *sucA* derived from the *C*. *glutamicum* ATCC 13869 strain, but lacking the ORF was obtained by overlap PCR using, as primers, synthetic DNAs designed with reference to the nucleotide sequence of that gene of C. *glutamicum* ATCC 13032 which has already been reported (SEQ ID NO: 9, GenBank Database Accession No.NC_003450). Specifically, PCR was performed in a conventional manner with the chromosomal DNA of the *C*. *glutamicum* ATCC 13869 strain as a template and the synthetic DNAs of SEQ ID NOS: 1 and 2 as primers to obtain an amplification product of the N-terminus side of the *sucA* gene. Separately, in order to obtain an amplification product of the C-terminus side of the *sucA* gene, PCR was performed in a conventional manner with the genomic DNA of the *C*. *glutamicum* ATCC 13869 strain as a template and the synthetic DNAs of SEQ ID NOS: 3 and 4 as primers. The nucleotide sequences of SEQ ID NOS: 2 and 3 are complementary to each other, and have a structure that includes *sucA* lacking the entire sequence of the ORF.

Then, in order to obtain a *sucA* gene fragment lacking the internal sequence, equimolar amounts of the aforementioned gene products of the N- and C-terminus sides of *sucA* were mixed, and used as a template to perform PCR in a conventional manner with the synthetic DNAs of SEQ ID NOS: 5 and 6 as primers and thereby obtain a mutation-introduced *sucA* gene amplification product. The produced PCR product was purified in a conventional manner and then digested with *Bam*HI, and the resultant was inserted into pBS3 mentioned above at the *Bam*HI site. Competent cells of *Escherichia coli* JM109 (Takara Bio) were transformed with the obtained DNA, plated on an LB plate medium containing 100 µM of IPTG, 40 µg/ml of X-Gal and 25 µg/ml of Km, and cultured overnight, and the white colonies that appeared were picked up, and separated into single colonies to obtain transformants. Plasmids were extracted from the obtained transformants, and a plasmid into which the target PCR product was inserted was designated pBS3ΔsucA.

### (1-2) Construction of sucA-deficient strain

The pBS3ΔsucA obtained in (1-1) mentioned above did not contain any region enabling autonomous replication of the plasmid in cells of coryneform bacteria. Therefore, when coryneform bacteria were transformed with this plasmid, a strain in which this plasmid was incorporated into chromosome by homologous recombination appeared as a transformant even though it occurred at low frequency. The *C*. *glutamicum* ATCC 13869 strain was transformed by the electric pulse method using the plasmid pBS3ΔsucA at a high concentration, applied on the CM-Dex plate medium (5 g/L of glucose, 10 g/L of polypeptone, 10 g/L of yeast extract, 1 g/L of KH₂PO₄, 0.4 g/L of MgSO₄·7H₂O, 0.01 g/L of FeSO₄·7H₂O, 0.01 g/L of MnSO₄·7H₂O, 3 g/L of urea, 1.2 g/L of soybean hydrolysate, 10 µg/L of biotin, 15 g/L of agar, adjusted to pH 7.5 with NaOH) containing 25 µg/ml of kanamycin, and cultured at 31.5°C for about 30 hours. The strain grown on this medium was a strain in which the kanamycin resistance gene and the *sacB* gene originating in the plasmid were inserted into the genome as a result of homologous recombination between the *sucA* gene fragment of the plasmid and that gene on the genome of the ATCC 13869 strain.

Then, these first recombinants were cultured overnight at 31.5°C in the CM-Dex liquid medium (prepared with the components of the CM-Dex plate medium except for agar) not containing kanamycin, the medium was appropriately diluted and applied to the 10% sucrose-containing Dex-S10 medium (100 g/L of sucrose, 10 g/L of polypeptone, 10 g/L of yeast extract, 1 g/L of KH₂PO₄, 0.4 g/L of MgSO₄·7H₂O, 0.01 g/L of FeSO₄·7H₂O, 0.01 g/L of MnSO₄·4H₂O, 3 g/L of urea, 1.2 g/L of soybean hydrolysate, 10 µg/L of biotin, 15 g/L of agar, adjusted to pH 7.5 with KOH) not containing kanamycin, and culture was performed at 31.5°C for about 30 hours. As a result, strains were obtained that had become insensitive to sucrose due to elimination of the *sacB* gene resulting from the second homologous recombination.

The strains obtained as described above included those in which the *sucA* gene was replaced with that of mutant type derived from pBS3ΔsucA and those in which the *sucA* gene was reverted to wild type. Whether the *sucA* gene is that of mutant type or wild type can be easily determined by directly using the cells obtained by the culture on the Dex-S10 plate medium for PCR to detect the *sucA* gene. Strains that provided a PCR product smaller than that obtained with the chromosomal DNA of the ATCC 13869 strain used as a template in analysis using the primers for PCR amplification of the *sucA* gene (SEQ ID NOS: 5 and 6) were used as *sucA*-deficient strains in the following experiments.

L-Glutamic acid-producing ability of the *sucA*-deficient strains was evaluated by the following method. The strains were cultured on the CM-Dex plate medium, and the grown strains were each cultured at 31.5°C with shaking in 20 ml of a medium containing 30 g of sucrose, 1 g of KH₂PO₄, 0.4 g of MgSO₄, 15 g of (NH₄)₂SO₄, 0.01 g of FeSO₄·7H₂O, 0.01 g of MnSO₄·7H₂O, 13.7 ml of soybean hydrolysate, 200 µg of thiamin hydrochloride, 300 µg of biotin, and 50 g of CaCO₃ in 1 L of pure water (pH was adjusted to 8.0 with KOH) contained in a Sakaguchi flask. When all glucose in the medium was consumed, the culture was terminated. L-Glutamic acid concentration was measured for the culture supernatant appropriately diluted with water by using Biotech Analyzer (AS-210, Sakura SI). A strain that showed a high L-glutamic acid fermentation yield was selected and designated ATCC13869ΔsucA.

### (1-3) Construction of plasmid for bca amplification

In order to construct a strain in which expression of the carbonic anhydrase gene (*bca*) was enhanced, the pVK9 shuttle vector was treated with *Bam*HI, the resultant was ligated with a DNA fragment encoding the enzyme (obtained by amplification by PCR using the sequences of SEQ ID NOS: 7 and 8 as primers and the chromosomal DNA of the *C*. *glutamicum* ATCC 13869 strain as a template, and treatment with *Bam*HI), the ligation product was used to transform competent cells of *Escherichia coli* JM109 (Takara Bio), and the cells were applied to the LB plate medium containing 100 µM of IPTG, 40 µg/ml of X-Gal and 25 µg/ml of Cm, and cultured overnight. Then, white colonies that appeared were picked up, and separated into single colonies to obtain transformants. Plasmids were extracted from the obtained transformants, and a plasmid in which the *bca* gene was ligated in the forward direction with respect to the *lacZ* gene was designated pVK9-bca.

pVK9 is a shuttle vector obtained by blunt-ending the *Ava*II site of pHSG299 (Takara Bio) and inserting a fragment, which is obtained by excising a region of pHK4 (Japanese Patent Laid-open No. 05-007491) autonomously replicable in coryneform bacteria with *Bam*HI and *Kpn*I*,* and blunt-ending the region, into the blunt-ended site of pHSG299.

The synthetic DNAs of SEQ ID NOS: 7 and 8 can be designed with reference to the nucleotide sequence of the carbonic anhydrase gene of *Corynebacterium* *glutamicum* ATCC 13032 which have been previously reported (GenBank Database Accession No.NC_003450, SEQ ID NO: 13).

### (1-4) Introduction of plasmid for BCA amplification into ATCC13869ΔsucA strain

Strains were obtained by transforming the ATCC13869ΔsucA strain with pVK9 (plasmid for control) and pVK9-bca (plasmid for BCA amplification). The transformation was performed by the electric pulse method, and the cells were applied to the CM-Dex plate medium containing 25 µg/ml of kanamycin, and cultured at 31.5°C for about 30 hours to obtain transformants. Strains introduced with each of the aforementioned plasmids were designated ATCC13869ΔsucA(pVK9) and ATCC13869ΔsucA(pVK9-bca), respectively.

### 2. Confirmation of L-glutamic acid accumulation improvement effect in BCA-enhanced ATCC13869ΔsucA strain

Effect of the *bca* amplification was evaluated by using the flask culture evaluation system mentioned in (1-2).

### (2-1) Glutamic acid accumulation observed with BCA-enhanced strain

Effect of the enhancement of BCA on improvement of L-glutamic acid accumulation was evaluated with the *C*. *glutamicum* ATCC13869ΔsucA(pVK9) strain and the ATCC13869ΔsucA(pVK9-*bca*) strain by culture in the same manner as that used for the evaluation of the *sucA*-deficient strain described in (1-2) mentioned above. The results obtained after the culture of 12 hours are shown in the following table. It became clear that the L-glutamic acid accumulation amount was higher for the BCA-enhanced strain compared with the control strain (n = 4).

**[Table 1]**

| | L-Glutamic acid accumulation after culture for 12 hours (g/L, mean ± standard deviation % (n = sample number)) |
|---|---|
| ATCC13869ΔsucA(pVK9) | 7.1 ± 0.63 (n = 4) |
| ATCC13869ΔsucA(pVK9-bca) | 8.0 ± 0.78 (n = 4) |

### (2-2) L-amino acid accumulation observed with BCA-enhanced strain

Effect of the enhancement of BCA on improvement of L-amino acid accumulation was evaluated with the *C*. *glutamicum* ATCC13869ΔsucA(pVK9) strain and the ATCC13869ΔsucA(pVK9-bca) strain by culture in the same manner as that used for the evaluation of the *sucA*-deficient strain described in (1-2) mentioned above. One sample for each of culture supernatants obtained after the culture of the two kinds of strains for 24 hours was diluted 51 times with 0.02 N hydrochloric acid, and various L-amino acids in the dilution were quantified with an amino acid analyzer (L-8500, Hitachi Co., Ltd.). The results are shown in Fig. 1. It became clear that accumulation amounts of L- aspartic acid and L-alanine were improved by the enhancement of BCA compared with the control strain (n = 2).

### Explanation of Sequence Listing

SEQ ID NO: 1: Nucleotide sequence of primer for disruption of *C*. *glutamicum* ATCC 13869 *sucA* gene
SEQ ID NO: 2: Nucleotide sequence of primer for disruption of *C*. *glutamicum* ATCC 13869 *sucA* gene
SEQ ID NO: 3: Nucleotide sequence of primer for disruption of *C*. *glutamicum* ATCC 13869 *sucA* gene
SEQ ID NO: 4: Nucleotide sequence of primer for disruption of *C*. *glutamicum* ATCC 13869 *sucA* gene
SEQ ID NO: 5: Nucleotide sequence of primer for amplification of *C*. *glutamicum* ATCC 13869 *sucA* gene
SEQ ID NO: 6: Nucleotide sequence of primer for amplification of *C*. *glutamicum* ATCC 13869 *sucA* gene
SEQ ID NO: 7: Nucleotide sequence of primer for amplification of *C*. *glutamicum bca* gene
SEQ ID NO: 8: Nucleotide sequence of primer for amplification of *C*. *glutamicum bca* gene
SEQ ID NO: 9: Nucleotide sequence of *C*. *glutamicum* ATCC 13869 *sucA* gene
SEQ ID NO: 10: Amino acid sequence of *C*. *glutamicum* ATCC 13869 α-KGDH
SEQ ID NO: 11: Nucleotide sequence of *C*. *glutamicum* ATCC 13869 *bca* gene
SEQ ID NO: 12: Amino acid sequence of *C*. *glutamicum* ATCC 13869 Bca
SEQ ID NO: 13: Nucleotide sequence of *C*. *glutamicum* ATCC 13032 *bca* gene
SEQ ID NO: 14: Amino acid sequence of *C*. *glutamicum* ATCC 13032 Bca
SEQ ID NO: 15: Nucleotide sequence of *Lactobacillus pentosus* MD363 *xpkA* gene
SEQ ID NO: 16: Amino acid sequence of *Lactobacillus pentosus* MD363 XpkA
SEQ ID NO: 17: Nucleotide sequence of *Lactobacillus plantarum xpkl* gene
SEQ ID NO: 18: Amino acid sequence of *Lactobacillus plantarum* Xpk1
SEQ ID NO: 19: Nucleotide sequence of *Bifidobacterium lactis xfp* gene
SEQ ID NO: 20: Amino acid sequence of *Bifidobacterium lactis* Xfp
SEQ ID NO: 21: Nucleotide sequence of *C*. *glutamicum* ATCC 13869 *pta* gene
SEQ ID NO: 22: Amino acid sequence of *C*. *glutamicum* ATCC 13869 Pta
SEQ ID NO: 23: Nucleotide sequence of *C*. *glutamicum* ATCC 13032 *pta* gene
SEQ ID NO: 24: Amino acid sequence of *C*. *glutamicum* ATCC 13032 Pta
SEQ ID NO: 25: Nucleotide sequence of primer for amplification of *C*. *glutamicum pta* gene
SEQ ID NO: 26: Nucleotide sequence of primer for amplification of *C*. *glutamicum pta* gene
SEQ ID NO: 27: Nucleotide sequence of *E. coli* MG1655 *yadF* gene
SEQ ID NO: 28: Amino acid sequence of *E. coli* MG1655 YadF
SEQ ID NO: 29: Nucleotide sequence of *E. coli* MG1655 *cynT* gene
SEQ ID NO: 30: Amino acid sequence of *E. coli* MG1655 CynT

## Claims

1. A method for producing an L-amino acid, which comprises culturing a coryneform bacterium having an L-amino acid-producing ability in a medium to produce and accumulate the L-amino acid in the medium or cells of the bacterium, and collecting the L-amino acid from the medium or cells, wherein said coryneform bacterium has been modified to enhance carbonic anhydrase activity, and wherein the L-amino acid is selected from the group consisting of L-glutamic acid, L-glutamine, L-proline, L-arginine, L-alanine and L-aspartic acid.

2. The method according to claim 1, wherein said carbonic anhydrase activity is enhanced by increasing a copy number of a gene encoding carbonic anhydrase, or by modifying an expression control sequence of the gene.

3. The method according to claim 2, wherein the gene encoding the carbonic anhydrase is a DNA defined in the following (a) or (b):
(a) a DNA comprising the nucleotide sequence of the nucleotide numbers 562 to 1182 of SEQ ID NO: 11, or the nucleotide sequence of SEQ ID NO: 13, or
(b) a DNA that is able to hybridize with a complement of the nucleotide sequence of the nucleotide numbers 562 to 1182 of SEQ ID NO: 11, or the nucleotide sequence of SEQ ID NO: 13 under stringent conditions, and encoding a protein having carbonic anhydrase activity.

4. The method according to any one of claims 1 to 3, wherein the bacterium has been further modified to impart D-xylose-5-phosphate phosphoketolase activity and/or fructose-6-phosphate phosphoketolase activity.

5. The method according to any one of claims 1 to 4, wherein the bacterium has been further modified to enhance phosphotransacetylase activity.

6. The method according to any one of claims 1 to 5, wherein the bacterium has been further modified to enhance pyruvate carboxylase activity.

7. The method according to any one of claims 1 to 6, wherein the bacterium has been further modified to enhance phosphoenolpyruvate carboxylase activity.

## Patentansprüche

1. Verfahren zur Herstellung einer L-Aminosäure, umfassend das Kultivieren eines coryneformen Bakteriums mit einer L-Aminosäure-produzierenden Fähigkeit in einem Medium, um die L-Aminosäure in dem Medium oder den Zellen des Bakteriums zu produzieren und zu akkumulieren, und das Gewinnen der L-Aminosäure aus dem Medium oder den Zellen, wobei das coryneforme Bakterium modifiziert wurde, um die Carboanhydraseaktivität (carbonic anhydrase activity) zu erhöhen, und wobei die L-Aminosäure aus der Gruppe ausgewählt ist, die aus L-Glutaminsäure, L-Glutamin, L-Prolin, L-Arginin, L-Alanin und L-Asparaginsäure besteht.

2. Verfahren gemäß Anspruch 1, wobei die Carboanhydraseaktivität durch Erhöhen einer Kopienzahl eines Gens, das für Carboanhydrase kodiert, oder durch Modifizieren einer Expressionskontrollsequenz des Gens erhöht wird.

3. Verfahren gemäß Anspruch 2, wobei das Gen, das für die Carboanhydrase kodiert, eine DNA ist, die in den folgenden (a) oder (b) definiert ist:
(a) eine DNA, die die Nukleotidsequenz der Nukleotide Nummer 562 bis 1182 der SEQ ID NO: 11 oder die Nukleotidsequenz der SEQ ID NO: 13 umfasst, oder
(b) eine DNA, die in der Lage ist, mit einem Komplement der Nukleotidsequenz der Nukleotide Nummer 562 bis 1182 von SEQ ID NO: 11 oder der Nukleotidsequenz von SEQ ID NO: 13 unter stringenten Bedingungen zu hybridisieren, und die ein Protein mit Carboanhydrase-Aktivität kodiert.

4. Verfahren gemäß irgendeinem der Ansprüche 1 bis 3, wobei das Bakterium weitergehend modifiziert worden ist, um D-Xylose-5-phosphat-Phosphoketolase-Aktivität und/oder Fructose-6-phosphat-Phosphoketolase-Aktivität zu verleihen.

5. Verfahren gemäß irgendeinem der Ansprüche 1 bis 4, wobei das Bakterium weitergehend modifiziert worden ist, um die Phosphotransacetylase-Aktivität zu verstärken.

6. Verfahren gemäß irgendeinem der Ansprüche 1 bis 5, wobei das Bakterium weitergehend modifiziert wurde, um die Pyruvat-Carboxylase-Aktivität zu erhöhen.

7. Verfahren nach einem der Ansprüche 1 bis 6, wobei das Bakterium weitergehend modifiziert wurde, um die Phosphoenolpyruvat-Carboxylase-Aktivität zu erhöhen.

## Revendications

1. Procédé de production d'un acide L-aminé, qui comprend la mise en culture d'une bactérie corynéforme présentant une capacité de production d'acide L-aminé dans un milieu pour produire et accumuler l'acide L-aminé dans le milieu ou des cellules de la bactérie, et récolter l'acide L-aminé du milieu ou des cellules, dans lequel ladite bactérie corynéforme a été modifiée pour améliorer l'activité anhydrase carbonique, et dans lequel l'acide L-aminé est choisi dans le groupe consistant en l'acide L-glutamique, la L-glutamine, la L-proline, la L-arginine, la L-alanine et l'acide L-aspartique.

2. Procédé selon la revendication 1, dans lequel ladite activité anhydrase carbonique est améliorée en augmentant un nombre de copies d'un gène codant pour l'anhydrase carbonique ou en modifiant une séquence de régulation de l'expression du gène.

3. Procédé selon la revendication 2, dans lequel le gène codant pour l'anhydrase carbonique est un ADN défini dans le (a) ou (b) suivant :
(a) un ADN comprenant la séquence nucléotidique des numéros de nucléotides 562 à 1182 de SEQ ID NO: 11 ou la séquence nucléotidique de SEQ ID NO: 13, ou
(b) un ADN qui est capable de s'hybrider avec un complément de la séquence nucléotidique des numéros de nucléotides 562 à 1182 de SEQ ID NO: 11 ou la séquence nucléotidique de SEQ ID NO: 13 dans des conditions rigoureuses et qui code pour une protéine présentant une activité anhydrase carbonique.

4. Procédé selon l'une quelconque des revendications 1 à 3, dans lequel la bactérie a été davantage modifiée pour conférer une activité D-xylose-5-phosphate phosphocétolase et/ou une activité fructose-6-phosphate phosphocétolase.

5. Procédé selon l'une quelconque des revendications 1 à 4, dans lequel la bactérie a été davantage modifiée pour améliorer l'activité phosphotransacétylase.

6. Procédé selon l'une quelconque des revendications 1 à 5, dans lequel la bactérie a été davantage modifiée pour améliorer l'activité pyruvate carboxylase.

7. Procédé selon l'une quelconque des revendications 1 à 6, dans lequel la bactérie a été davantage modifiée pour améliorer l'activité phosphoénolpyruvate carboxylase.


### SEQUENCE LISTING

<110> Ajinomoto Co., Inc.
<120> Method for producing L-amino acid
<130> D158-10114
<140> EP10811634.4
   <141> 2010-07-21
<150> JP2009-194636
   <151> 2009-08-25
<160> 30
<170> PatentIn version 3.3
<210> 1
   <211> 20
   <212> DNA
   <213> Artificial sequence
<220>
   <223> primer
<400> 1
   ccaggcactc gtcctcggtt 20
<210> 2
   <211> 48
   <212> DNA
   <213> Artificial sequence
<220>
   <223> primer
<400> 2
   aggctagtgc aggactataa agaccagttc tcctaaaaat aacgtgtc 48
<210> 3
   <211> 48
   <212> DNA
   <213> Artificial sequence
<220>
   <223> primer
<400> 3
   gacacgttat ttttaggaga actggtcttt atagtcctgc actagcct 48
<210> 4
   <211> 20
   <212> DNA
   <213> Artificial sequence
<220>
   <223> primer
<400> 4
   tccatcgtgg ccaccgatcc 20
<210> 5
   <211> 25
   <212> DNA
   <213> Artificial sequence
<220>
   <223> primer
<400> 5
   cgggatcccc accggcgtac tcgtg 25
<210> 6
   <211> 28
   <212> DNA
   <213> Artificial sequence
<220>
   <223> primer
<400> 6
   ccacggatcc ttccaatgct attggttg 28
<210> 7
   <211> 27
   <212> DNA
   <213> Artificial sequence
<220>
   <223> primer
<400> 7
   tgcgaggtca ctttttcgct gttcagg 27
<210> 8
   <211> 27
   <212> DNA
   <213> Artificial sequence
<220>
   <223> primer
<400> 8
   gtcgataagc tcttgctttt tcgcagg 27
<210> 9
   <211> 4394
   <212> DNA
   <213> Corynebacterium glutamicum
<220>
   <221> CDS
   <222> (443)..(4213)
<400> 9
   gtcgacaagc aaaatcgaag cggcagcacg ccgcgtcgga gccttaaacg ccatcgccgc     60
   catccctgat ggtttcaatc atcaagtcgg tgaacgcggg cgcaacctgt catccggaca    120
   gcgccaactg atcgcgctgg cgcgcgccga actcatcgag ccttccatca tgcttctcga    180
   cgaagccacc tccaccctcg accccgccac cgaagccgtt atcctcaacg cctccgatcg    240
   agtcactaag ggacgcacca gcatcatcgt cgcgcaccgc ttggcaaccg ctaaaagggc    300
   cgaccgtatt cttgttgttg aacaaggacg tatcattgag gacggatctc acgacgcgtt    360
   gttgtctgct aacggcacct acgcccgcat gtggcattta atggcctgac acgttatttt    420
   taggagaact gtcaacaaat ta atg cta caa ctg ggg ctt agg cat aat cag     472
                            Met Leu Gln Leu Gly Leu Arg His Asn Gln
                            1               5                   10
   cca acg acc aac gtt aca gtg gat aaa ata aag ctc aat aaa ccc tca      520
   Pro Thr Thr Asn Val Thr Val Asp Lys Ile Lys Leu Asn Lys Pro Ser
                   15                  20                  25
   aga agc aag gaa aag agg cga gta cct gcc gtg agc agc gct agt act      568
   Arg Ser Lys Glu Lys Arg Arg Val Pro Ala Val Ser Ser Ala Ser Thr
               30                  35                  40
   ttc ggc cag aat gcg tgg ctg gta gac gag atg ttc cag cag ttc cag      616
   Phe Gly Gln Asn Ala Trp Leu Val Asp Glu Met Phe Gln Gln Phe Gln
           45                  50                  55
   aag gac ccc aag tcc gtg gac aag gaa tgg aga gaa ctc ttt gag gcg      664
   Lys Asp Pro Lys Ser Val Asp Lys Glu Trp Arg Glu Leu Phe Glu Ala
       60                  65                  70
   cag ggg gga cca aat gct acc ccc gct aca aca gaa gca cag cct tca      712
   Gln Gly Gly Pro Asn Ala Thr Pro Ala Thr Thr Glu Ala Gln Pro Ser
   75                  80                  85                  90
   gcg ccc aag gag tct gcg aaa cca gca cca aag gct gcc cct gca gcc      760
   Ala Pro Lys Glu Ser Ala Lys Pro Ala Pro Lys Ala Ala Pro Ala Ala
                   95                  100                 105
   aag gca gca ccg cgc gta gaa acc aag ccg gcc gcc aag acc gcc cct      808
   Lys Ala Ala Pro Arg Val Glu Thr Lys Pro Ala Ala Lys Thr Ala Pro
               110                 115                 120
   aag gcc aag gag tcc tca gtg cca cag caa cct aag ctt ccg gag cca      856
   Lys Ala Lys Glu Ser Ser Val Pro Gln Gln Pro Lys Leu Pro Glu Pro
           125                 130                 135
   gga caa acc cca atc agg ggt att ttc aag tcc atc gcg aag aac atg      904
   Gly Gln Thr Pro Ile Arg Gly Ile Phe Lys Ser Ile Ala Lys Asn Met
       140                 145                 150
   gat atc tcc ctg gaa atc cca acc gca acc tcg gtt cgc gat atg cca      952
   Asp Ile Ser Leu Glu Ile Pro Thr Ala Thr Ser Val Arg Asp Met Pro
   155                 160                 165                 170
   gct cgc ctc atg ttc gaa aac cgc gcg atg gtc aac gat cag ctc aag      1000
   Ala Arg Leu Met Phe Glu Asn Arg Ala Met Val Asn Asp Gln Leu Lys
                   175                 180                 185
   cgc acc cgc ggt ggc aag atc tcc ttc acc cac atc att ggc tac gcc      1048
   Arg Thr Arg Gly Gly Lys Ile Ser Phe Thr His Ile Ile Gly Tyr Ala
               190                 195                 200
   atg gtg aag gca gtc atg gct cac ccg gac atg aac aac tcc tac gac      1096
   Met Val Lys Ala Val Met Ala His Pro Asp Met Asn Asn Ser Tyr Asp
           205                 210                 215
   gtc atc gac ggc aag cca acc ctg atc gtg cct gag cac atc aac ctg      1144
   Val Ile Asp Gly Lys Pro Thr Leu Ile Val Pro Glu His Ile Asn Leu
       220                 225                 230
   ggc ctt gcc atc gac ctt cct cag aag gac ggc tcc cgc gca ctt gtc      1192
   Gly Leu Ala Ile Asp Leu Pro Gln Lys Asp Gly Ser Arg Ala Leu Val
   235                 240                 245                 250
   gta gca gcc atc aag gaa acc gag aag atg aac ttc tcc gag ttc ctc      1240
   Val Ala Ala Ile Lys Glu Thr Glu Lys Met Asn Phe Ser Glu Phe Leu
                   255                 260                 265
   gca gca tac gaa gac atc gtg aca cgc tcc cgc aag ggc aag ctc acc      1288
   Ala Ala Tyr Glu Asp Ile Val Thr Arg Ser Arg Lys Gly Lys Leu Thr
               270                 275                 280
   atg gat gac tac cag ggc gtt acc gtt tcc ttg acc aac cca ggt ggc      1336
   Met Asp Asp Tyr Gln Gly Val Thr Val Ser Leu Thr Asn Pro Gly Gly
           285                 290                 295
   atc ggt acc cgc cac tct gtc cca cgt ctg acc aag ggc cag ggc acc      1384
   Ile Gly Thr Arg His Ser Val Pro Arg Leu Thr Lys Gly Gln Gly Thr
       300                 305                 310
   atc atc ggt gtc ggt tcc atg gat tac cca gca gag ttc cag ggc gct      1432
   Ile Ile Gly Val Gly Ser Met Asp Tyr Pro Ala Glu Phe Gln Gly Ala
   315                 320                 325                 330
   tcc gaa gac cgc ctt gca gag ctc ggc gtt gga aag ctt gtc acc atc      1480
   Ser Glu Asp Arg Leu Ala Glu Leu Gly Val Gly Lys Leu Val Thr Ile
                   335                 340                 345
   acc tcc acc tac gat cac cgc gtg atc cag ggt gct gtg tcc ggt gaa      1528
   Thr Ser Thr Tyr Asp His Arg Val Ile Gln Gly Ala Val Ser Gly Glu
               350                 355                 360
   ttc ctg cgt acc atg tct cgc ctg ctc acc gat gat tcc ttc tgg gat      1576
   Phe Leu Arg Thr Met Ser Arg Leu Leu Thr Asp Asp Ser Phe Trp Asp
           365                 370                 375
   gag atc ttc gac gca atg aac gtt cct tac acc cca atg cgt tgg gca      1624
   Glu Ile Phe Asp Ala Met Asn Val Pro Tyr Thr Pro Met Arg Trp Ala
       380                 385                 390
   cag gac gtt cca aac acc ggt gtt gat aag aac acc cgc gtc atg cag      1672
   Gln Asp Val Pro Asn Thr Gly Val Asp Lys Asn Thr Arg Val Met Gln
   395                 400                 405                 410
   ctc att gag gca tac cgc tcc cgt gga cac ctc atc gct gac acc aac      1720
   Leu Ile Glu Ala Tyr Arg Ser Arg Gly His Leu Ile Ala Asp Thr Asn
                   415                 420                 425
   cca ctt tca tgg gtt cag cct ggc atg cca gtt cca gac cac cgc gac      1768
   Pro Leu Ser Trp Val Gln Pro Gly Met Pro Val Pro Asp His Arg Asp
               430                 435                 440
   ctc gac atc gag acc cac agc ctg acc atc tgg gat ctg gac cgt acc      1816
   Leu Asp Ile Glu Thr His Ser Leu Thr Ile Trp Asp Leu Asp Arg Thr
           445                 450                 455
   ttc agc gtc ggt ggc ttc ggc ggc aag gag acc atg acc ctg cgc gag      1864
   Phe Ser Val Gly Gly Phe Gly Gly Lys Glu Thr Met Thr Leu Arg Glu
       460                 465                 470
   gta ctg tcc cgc ctg cgc gct gcc tac acc ttg aag gtc ggc tcc gaa      1912
   Val Leu Ser Arg Leu Arg Ala Ala Tyr Thr Leu Lys Val Gly Ser Glu
   475                 480                 485                 490
   tac acc cac atc ctg gac cgc gac gag cgc acc tgg ctg cag gac cgc      1960
   Tyr Thr His Ile Leu Asp Arg Asp Glu Arg Thr Trp Leu Gln Asp Arg
                   495                 500                 505
   ctc gaa gcc gga atg cca aag cca acc cag gca gag cag aag tac atc      2008
   Leu Glu Ala Gly Met Pro Lys Pro Thr Gln Ala Glu Gln Lys Tyr Ile
               510                 515                 520
   ctg cag aag ctg aac gcc gca gag gct ttc gag aac ttc ctg cag acc      2056
   Leu Gln Lys Leu Asn Ala Ala Glu Ala Phe Glu Asn Phe Leu Gln Thr
           525                 530                 535
   aag tac gtc ggc cag aag cgc ttc tcc ctc gaa ggt gca gaa gct ctc      2104
   Lys Tyr Val Gly Gln Lys Arg Phe Ser Leu Glu Gly Ala Glu Ala Leu
       540                 545                 550
   atc cca ctg atg gac tcc gcc atc gac acc gcc gca ggc cag ggc ctc      2152
   Ile Pro Leu Met Asp Ser Ala Ile Asp Thr Ala Ala Gly Gln Gly Leu
   555                 560                 565                 570
   gac gaa gtt gtc atc ggt atg cca cac cgt ggt cgc ctc aac gtg ctg      2200
   Asp Glu Val Val Ile Gly Met Pro His Arg Gly Arg Leu Asn Val Leu
                   575                 580                 585
   ttc aac atc gtg ggc aag cca ctg gca tcc atc ttc aac gag ttt gaa      2248
   Phe Asn Ile Val Gly Lys Pro Leu Ala Ser Ile Phe Asn Glu Phe Glu
               590                 595                 600
   ggc caa atg gag cag ggc cag atc ggt ggc tcc ggt gac gtg aag tac      2296
   Gly Gln Met Glu Gln Gly Gln Ile Gly Gly Ser Gly Asp Val Lys Tyr
           605                 610                 615
   cac ctc ggt tcc gaa ggc cag cac ctg cag atg ttc ggc gac ggc gag      2344
   His Leu Gly Ser Glu Gly Gln His Leu Gln Met Phe Gly Asp Gly Glu
       620                 625                 630
   atc aag gtc tcc ctg act gct aac ccg tcc cac ctg gaa gct gtt aac      2392
   Ile Lys Val Ser Leu Thr Ala Asn Pro Ser His Leu Glu Ala Val Asn
   635                 640                 645                 650
   cca gtg atg gaa ggt atc gtc cgc gca aag cag gac tac ctg gac aag      2440
   Pro Val Met Glu Gly Ile Val Arg Ala Lys Gln Asp Tyr Leu Asp Lys
                   655                 660                 665
   ggc gta gac ggc aag act gtt gtg cca ctg ctg ctc cac ggt gac gct      2488
   Gly Val Asp Gly Lys Thr Val Val Pro Leu Leu Leu His Gly Asp Ala
               670                 675                 680
   gca ttc gca ggc ctg ggc atc gtg cca gaa acc atc aac ctg gct aag     2536
   Ala Phe Ala Gly Leu Gly Ile Val Pro Glu Thr Ile Asn Leu Ala Lys
           685                 690                 695
   ctg cgt ggc tac gac gtc gga ggc acc atc cac atc gtg gtg aac aac     2584
   Leu Arg Gly Tyr Asp Val Gly Gly Thr Ile His Ile Val Val Asn Asn
       700                 705                 710
   cag atc ggc ttc acc acc acc cca gac tcc agc cgc tcc atg cac tac     2632
   Gln Ile Gly Phe Thr Thr Thr Pro Asp Ser Ser Arg Ser Met His Tyr
   715                 720                 725                 730
   gca acc gac tac gcc aag gca ttc ggc tgc cca gtc ttc cac gtc aat     2680
   Ala Thr Asp Tyr Ala Lys Ala Phe Gly Cys Pro Val Phe His Val Asn
                   735                 740                 745
   ggt gat gac cca gag gca gtt gtc tgg gtt ggc cag ctg gca acc gag     2728
   Gly Asp Asp Pro Glu Ala Val Val Trp Val Gly Gln Leu Ala Thr Glu
               750                 755                 760
   tac cgt cgt cgc ttc ggc aag gac gtc ttc atc gac ctc gtt tgc tac     2776
   Tyr Arg Arg Arg Phe Gly Lys Asp Val Phe Ile Asp Leu Val Cys Tyr
           765                 770                 775
   cgc ctc cgc ggc cac aac gaa gct gat gat cct tcc atg acc cag cca     2824
   Arg Leu Arg Gly His Asn Glu Ala Asp Asp Pro Ser Met Thr Gln Pro
       780                 785                 790
   aag atg tat gag ctc atc acc ggc cgc gag acc gtt cgt gct cag tac     2872
   Lys Met Tyr Glu Leu Ile Thr Gly Arg Glu Thr Val Arg Ala Gln Tyr
   795                 800                 805                 810
   acc gaa gac ctg ctc gga cgt gga gac ctc tcc aac gaa gat gca gaa     2920
   Thr Glu Asp Leu Leu Gly Arg Gly Asp Leu Ser Asn Glu Asp Ala Glu
                   815                 820                 825
   gca gtc gtc cgc gac ttc cac gac cag atg gaa tct gtg ttc aac gaa     2968
   Ala Val Val Arg Asp Phe His Asp Gln Met Glu Ser Val Phe Asn Glu
               830                 835                 840
   gtc aag gaa ggc ggc aag aag cag gct gag gca cag acc ggc atc acc     3016
   Val Lys Glu Gly Gly Lys Lys Gln Ala Glu Ala Gln Thr Gly Ile Thr
           845                 850                 855
   ggc tcc cag aag ctt cca cac ggc ctt gag acc aac atc tcc cgt gaa     3064
   Gly Ser Gln Lys Leu Pro His Gly Leu Glu Thr Asn Ile Ser Arg Glu
       860                 865                 870
   gag ctc ctg gaa ctg gga cag gct ttc gcc aac acc cca gaa ggc ttc     3112
   Glu Leu Leu Glu Leu Gly Gln Ala Phe Ala Asn Thr Pro Glu Gly Phe
   875                 880                 885                 890
   aac tac cac cca cgt gtg gct cca gtt gct aag aag cgc gtc tcc tct     3160
   Asn Tyr His Pro Arg Val Ala Pro Val Ala Lys Lys Arg Val Ser Ser
                   895                 900                 905
   gtc acc gaa ggt ggc atc gac tgg gca tgg ggc gag ctc ctc gcc ttc     3208
   Val Thr Glu Gly Gly Ile Asp Trp Ala Trp Gly Glu Leu Leu Ala Phe
               910                 915                 920
   ggt tcc ctg gct aac tcc ggc cgc ttg gtt cgc ctt gca ggt gaa gat     3256
   Gly Ser Leu Ala Asn Ser Gly Arg Leu Val Arg Leu Ala Gly Glu Asp
           925                 930                 935
   tcc cgc cgc ggt acc ttc acc cag cgc cac gca gtt gcc atc gac cca     3304
   Ser Arg Arg Gly Thr Phe Thr Gln Arg His Ala Val Ala Ile Asp Pro
       940                 945                 950
   gcg acc gct gaa gag ttc aac cca ctc cac gag ctt gca cag tcc aag     3352
   Ala Thr Ala Glu Glu Phe Asn Pro Leu His Glu Leu Ala Gln Ser Lys
   955                 960                 965                 970
   ggc aac aac ggt aag ttc ctg gtc tac aac tcc gca ctg acc gag tac     3400
   Gly Asn Asn Gly Lys Phe Leu Val Tyr Asn Ser Ala Leu Thr Glu Tyr
                   975                 980                 985
   gca ggc atg ggc ttc gag tac ggc tac tcc gta gga aac gaa  gac tcc    3448
   Ala Gly Met Gly Phe Glu Tyr Gly Tyr Ser Val Gly Asn Glu  Asp Ser
               990                 995                 1000
   gtc gtt gca  tgg gaa gca cag ttc  ggc gac ttc gcc aac  ggc gct      3493
   Val Val Ala  Trp Glu Ala Gln Phe  Gly Asp Phe Ala Asn  Gly Ala
           1005                1010                1015
   cag acc atc  atc gat gag tac gtc  tcc tca ggc gaa gct  aag tgg      3538
   Gln Thr Ile  Ile Asp Glu Tyr Val  Ser Ser Gly Glu Ala  Lys Trp
           1020                 1025                 1030
   ggc cag acc  tcc aag ctg atc ctt  ctg ctg cct cac ggc  tac gaa      3583
   Gly Gln Thr  Ser Lys Leu Ile Leu  Leu Leu Pro His Gly  Tyr Glu
           1035                 1040                 1045
   ggc cag ggc  cca gac cac tct tcc  gca cgt atc gag cgc  ttc ctg      3628
   Gly Gln Gly  Pro Asp His Ser Ser  Ala Arg Ile Glu Arg  Phe Leu
           1050                 1055                 1060
   cag ctg tgc  gct gag ggt tcc atg  act gtt gct cag cca  tcc acc      3673
   Gln Leu Cys  Ala Glu Gly Ser Met  Thr Val Ala Gln Pro  Ser Thr
           1065                 1070                 1075
   cca gca aac  cac ttc cac ctg ctg  cgt cgt cac gct ctg  tcc gac      3718
   Pro Ala Asn  His Phe His Leu Leu  Arg Arg His Ala Leu  Ser Asp
           1080                 1085                 1090
   ctg aag cgt  cca ctg gtt atc ttc  acc ccg aag tcc atg  ctg cgt      3763
   Leu Lys Arg  Pro Leu Val Ile Phe  Thr Pro Lys Ser Met  Leu Arg
           1095                 1100                 1105
   aac aag gct  gct gcc tcc gca cca  gaa gac ttc act gag  gtc acc      3808
   Asn Lys Ala  Ala Ala Ser Ala Pro  Glu Asp Phe Thr Glu  Val Thr
           1110                 1115                 1120
   aag ttc caa  tcc gtg atc gac gat  cca aac gtt gca gat  gca gcc      3853
   Lys Phe Gln  Ser Val Ile Asp Asp  Pro Asn Val Ala Asp  Ala Ala
           1125                 1130                 1135
   aag gtg aag  aag gtc atg ctg gtc  tcc ggc aag ctg tac  tac gaa      3898
   Lys Val Lys  Lys Val Met Leu Val  Ser Gly Lys Leu Tyr  Tyr Glu
           1140                 1145                 1150
   ttg gca aag  cgc aag gag aag gac  gga cgc gac gac atc  gcg atc      3943
   Leu Ala Lys  Arg Lys Glu Lys Asp  Gly Arg Asp Asp Ile  Ala Ile
           1155                 1160                 1165
   gtt cgt atc  gaa atg ctc cac cca  att ccg ttc aac cgc  atc tcc      3988
   Val Arg Ile  Glu Met Leu His Pro  Ile Pro Phe Asn Arg  Ile Ser
           1170                 1175                 1180
   gag gct ctt  gcc ggc tac cct aac  gct gag gaa gtc ctc  ttc gtt      4033
   Glu Ala Leu  Ala Gly Tyr Pro Asn  Ala Glu Glu Val Leu  Phe Val
           1185                 1190                 1195
   cag gat gag  cca gca aac cag ggc  cca tgg ccg ttc tac  cag gag      4078
   Gln Asp Glu  Pro Ala Asn Gln Gly  Pro Trp Pro Phe Tyr  Gln Glu
           1200                 1205                 1210
   cac ctc cca  gag ctg atc ccg aac  atg cca aag atg cgc  cgc gtt      4123
   His Leu Pro  Glu Leu Ile Pro Asn  Met Pro Lys Met Arg  Arg Val
           1215                 1220                 1225
   tcc cgc cgc  gct cag tcc tcc acc  gca act ggt gtt gct  aag gtg      4168
   Ser Arg Arg  Ala Gln Ser Ser Thr  Ala Thr Gly Val Ala  Lys Val
           1230                 1235                 1240
   cac cag ctg  gag gag aag cag ctt  atc gac gag gct ttc  gag gct      4213
   His Gln Leu  Glu Glu Lys Gln Leu  Ile Asp Glu Ala Phe  Glu Ala
           1245                 1250                 1255
   taagtcttta tagtcctgca ctagcctaga gggccttatg cagtgtgaat cacacagcat   4273
   aaggcccttt ttgctgccgt ggttgcctaa ggtggaaggc atgaaacgaa tctgtgcggt   4333
   cacgatctct tcagtacttt tgctaagtgg ctgctcctcc acttccacca cgcagctcga   4393
   g                                                                   4394
<210> 10
   <211> 1257
   <212> PRT
   <213> Corynebacterium glutamicum
<400> 10
   Met Leu Gln Leu Gly Leu Arg His Asn Gln Pro Thr Thr Asn Val Thr
   1               5                   10                  15
   Val Asp Lys Ile Lys Leu Asn Lys Pro Ser Arg Ser Lys Glu Lys Arg
               20                  25                  30
   Arg Val Pro Ala Val Ser Ser Ala Ser Thr Phe Gly Gln Asn Ala Trp
           35                  40                  45
   Leu Val Asp Glu Met Phe Gln Gln Phe Gln Lys Asp Pro Lys Ser Val
       50                  55                  60
   Asp Lys Glu Trp Arg Glu Leu Phe Glu Ala Gln Gly Gly Pro Asn Ala
   65                  70                  75                  80
   Thr Pro Ala Thr Thr Glu Ala Gln Pro Ser Ala Pro Lys Glu Ser Ala
                   85                  90                  95
   Lys Pro Ala Pro Lys Ala Ala Pro Ala Ala Lys Ala Ala Pro Arg Val
               100                 105                 110
   Glu Thr Lys Pro Ala Ala Lys Thr Ala Pro Lys Ala Lys Glu Ser Ser
           115                 120                 125
   Val Pro Gln Gln Pro Lys Leu Pro Glu Pro Gly Gln Thr Pro Ile Arg
       130                 135                 140
   Gly Ile Phe Lys Ser Ile Ala Lys Asn Met Asp Ile Ser Leu Glu Ile
   145                 150                 155                 160
   Pro Thr Ala Thr Ser Val Arg Asp Met Pro Ala Arg Leu Met Phe Glu
                   165                 170                 175
   Asn Arg Ala Met Val Asn Asp Gln Leu Lys Arg Thr Arg Gly Gly Lys
               180                 185                 190
   Ile Ser Phe Thr His Ile Ile Gly Tyr Ala Met Val Lys Ala Val Met
           195                 200                 205
   Ala His Pro Asp Met Asn Asn Ser Tyr Asp Val Ile Asp Gly Lys Pro
       210                 215                 220
   Thr Leu Ile Val Pro Glu His Ile Asn Leu Gly Leu Ala Ile Asp Leu
   225                 230                 235                 240
   Pro Gln Lys Asp Gly Ser Arg Ala Leu Val Val Ala Ala Ile Lys Glu
                   245                 250                 255
   Thr Glu Lys Met Asn Phe Ser Glu Phe Leu Ala Ala Tyr Glu Asp Ile
               260                 265                 270
   Val Thr Arg Ser Arg Lys Gly Lys Leu Thr Met Asp Asp Tyr Gln Gly
           275                 280                 285
   Val Thr Val Ser Leu Thr Asn Pro Gly Gly Ile Gly Thr Arg His Ser
       290                 295                 300
   Val Pro Arg Leu Thr Lys Gly Gln Gly Thr Ile Ile Gly Val Gly Ser
   305                 310                 315                 320
   Met Asp Tyr Pro Ala Glu Phe Gln Gly Ala Ser Glu Asp Arg Leu Ala
                   325                 330                 335
   Glu Leu Gly Val Gly Lys Leu Val Thr Ile Thr Ser Thr Tyr Asp His
               340                 345                 350
   Arg Val Ile Gln Gly Ala Val Ser Gly Glu Phe Leu Arg Thr Met Ser
           355                 360                 365
   Arg Leu Leu Thr Asp Asp Ser Phe Trp Asp Glu Ile Phe Asp Ala Met
       370                 375                 380
   Asn Val Pro Tyr Thr Pro Met Arg Trp Ala Gln Asp Val Pro Asn Thr
   385                 390                 395                 400
   Gly Val Asp Lys Asn Thr Arg Val Met Gln Leu Ile Glu Ala Tyr Arg
                   405                 410                 415
   Ser Arg Gly His Leu Ile Ala Asp Thr Asn Pro Leu Ser Trp Val Gln
               420                 425                 430
   Pro Gly Met Pro Val Pro Asp His Arg Asp Leu Asp Ile Glu Thr His
           435                 440                 445
   Ser Leu Thr Ile Trp Asp Leu Asp Arg Thr Phe Ser Val Gly Gly Phe
       450                 455                 460
   Gly Gly Lys Glu Thr Met Thr Leu Arg Glu Val Leu Ser Arg Leu Arg
   465                 470                 475                 480
   Ala Ala Tyr Thr Leu Lys Val Gly Ser Glu Tyr Thr His Ile Leu Asp
                   485                 490                 495
   Arg Asp Glu Arg Thr Trp Leu Gln Asp Arg Leu Glu Ala Gly Met Pro
               500                 505                 510
   Lys Pro Thr Gln Ala Glu Gln Lys Tyr Ile Leu Gln Lys Leu Asn Ala
           515                 520                 525
   Ala Glu Ala Phe Glu Asn Phe Leu Gln Thr Lys Tyr Val Gly Gln Lys
       530                 535                 540
   Arg Phe Ser Leu Glu Gly Ala Glu Ala Leu Ile Pro Leu Met Asp Ser
   545                 550                 555                 560
   Ala Ile Asp Thr Ala Ala Gly Gln Gly Leu Asp Glu Val Val Ile Gly
                   565                 570                 575
   Met Pro His Arg Gly Arg Leu Asn Val Leu Phe Asn Ile Val Gly Lys
               580                 585                 590
   Pro Leu Ala Ser Ile Phe Asn Glu Phe Glu Gly Gln Met Glu Gln Gly
           595                 600                 605
   Gln Ile Gly Gly Ser Gly Asp Val Lys Tyr His Leu Gly Ser Glu Gly
       610                 615                 620
   Gln His Leu Gln Met Phe Gly Asp Gly Glu Ile Lys Val Ser Leu Thr
   625                 630                 635                 640
   Ala Asn Pro Ser His Leu Glu Ala Val Asn Pro Val Met Glu Gly Ile
                   645                 650                 655
   Val Arg Ala Lys Gln Asp Tyr Leu Asp Lys Gly Val Asp Gly Lys Thr
               660                 665                 670
   Val Val Pro Leu Leu Leu His Gly Asp Ala Ala Phe Ala Gly Leu Gly
           675                 680                 685
   Ile Val Pro Glu Thr Ile Asn Leu Ala Lys Leu Arg Gly Tyr Asp Val
       690                 695                 700
   Gly Gly Thr Ile His Ile Val Val Asn Asn Gln Ile Gly Phe Thr Thr
   705                 710                 715                 720
   Thr Pro Asp Ser Ser Arg Ser Met His Tyr Ala Thr Asp Tyr Ala Lys
                   725                 730                 735
   Ala Phe Gly Cys Pro Val Phe His Val Asn Gly Asp Asp Pro Glu Ala
               740                 745                 750
   Val Val Trp Val Gly Gln Leu Ala Thr Glu Tyr Arg Arg Arg Phe Gly
           755                 760                 765
   Lys Asp Val Phe Ile Asp Leu Val Cys Tyr Arg Leu Arg Gly His Asn
       770                 775                 780
   Glu Ala Asp Asp Pro Ser Met Thr Gln Pro Lys Met Tyr Glu Leu Ile
   785                 790                 795                 800
   Thr Gly Arg Glu Thr Val Arg Ala Gln Tyr Thr Glu Asp Leu Leu Gly
                   805                 810                 815
   Arg Gly Asp Leu Ser Asn Glu Asp Ala Glu Ala Val Val Arg Asp Phe
               820                 825                 830
   His Asp Gln Met Glu Ser Val Phe Asn Glu Val Lys Glu Gly Gly Lys
           835                 840                 845
   Lys Gln Ala Glu Ala Gln Thr Gly Ile Thr Gly Ser Gln Lys Leu Pro
       850                 855                 860
   His Gly Leu Glu Thr Asn Ile Ser Arg Glu Glu Leu Leu Glu Leu Gly
   865                 870                 875                 880
   Gln Ala Phe Ala Asn Thr Pro Glu Gly Phe Asn Tyr His Pro Arg Val
                   885                 890                 895
   Ala Pro Val Ala Lys Lys Arg Val Ser Ser Val Thr Glu Gly Gly Ile
               900                 905                 910
   Asp Trp Ala Trp Gly Glu Leu Leu Ala Phe Gly Ser Leu Ala Asn Ser
           915                 920                 925
   Gly Arg Leu Val Arg Leu Ala Gly Glu Asp Ser Arg Arg Gly Thr Phe
       930                 935                 940
   Thr Gln Arg His Ala Val Ala Ile Asp Pro Ala Thr Ala Glu Glu Phe
   945                 950                 955                 960
   Asn Pro Leu His Glu Leu Ala Gln Ser Lys Gly Asn Asn Gly Lys Phe
                   965                 970                 975
   Leu Val Tyr Asn Ser Ala Leu Thr Glu Tyr Ala Gly Met Gly Phe Glu
               980                 985                 990
   Tyr Gly Tyr Ser Val Gly Asn Glu  Asp Ser Val Val Ala  Trp Glu Ala
           995                 1000                1005
   Gln Phe  Gly Asp Phe Ala Asn  Gly Ala Gln Thr Ile  Ile Asp Glu
       1010                1015                1020
   Tyr Val  Ser Ser Gly Glu Ala  Lys Trp Gly Gln Thr  Ser Lys Leu
       1025                1030                1035
   Ile Leu  Leu Leu Pro His Gly  Tyr Glu Gly Gln Gly  Pro Asp His
       1040                1045                1050
   Ser Ser  Ala Arg Ile Glu Arg  Phe Leu Gln Leu Cys  Ala Glu Gly
       1055                1060                1065
   Ser Met  Thr Val Ala Gln Pro  Ser Thr Pro Ala Asn  His Phe His
       1070                1075                1080
   Leu Leu  Arg Arg His Ala Leu  Ser Asp Leu Lys Arg  Pro Leu Val
       1085                 1090                 1095
   Ile Phe  Thr Pro Lys Ser Met  Leu Arg Asn Lys Ala  Ala Ala Ser
       1100                 1105                 1110
   Ala Pro  Glu Asp Phe Thr Glu  Val Thr Lys Phe Gln  Ser Val Ile
       1115                 1120                 1125
   Asp Asp  Pro Asn Val Ala Asp  Ala Ala Lys Val Lys  Lys Val Met
       1130                 1135                 1140
   Leu Val  Ser Gly Lys Leu Tyr  Tyr Glu Leu Ala Lys  Arg Lys Glu
       1145                 1150                 1155
   Lys Asp  Gly Arg Asp Asp Ile  Ala Ile Val Arg Ile  Glu Met Leu
       1160                 1165                 1170
   His Pro  Ile Pro Phe Asn Arg  Ile Ser Glu Ala Leu  Ala Gly Tyr
       1175                 1180                 1185
   Pro Asn  Ala Glu Glu Val Leu  Phe Val Gln Asp Glu  Pro Ala Asn
       1190                 1195                 1200
   Gln Gly  Pro Trp Pro Phe Tyr  Gln Glu His Leu Pro  Glu Leu Ile
       1205                 1210                 1215
   Pro Asn  Met Pro Lys Met Arg  Arg Val Ser Arg Arg  Ala Gln Ser
       1220                 1225                 1230
   Ser Thr  Ala Thr Gly Val Ala  Lys Val His Gln Leu  Glu Glu Lys
       1235                 1240                 1245
   Gln Leu  Ile Asp Glu Ala Phe  Glu Ala
       1250                 1255
<210> 11
   <211> 1471
   <212> DNA
   <213> Corynebacterium glutamicum
<220>
   <221> CDS
   <222> (562)..(1182)
<400> 11
   gtcgataagc tcttgctttt tcgcaggccc cgcaaggtaa cgtccggcta ccgcgcgctg     60
   gtacacgcga cgcacgttcg tatcgaccac cggcacgcgc tgcccaaaat gaaacgccgc    120
   gaccgcgcgc gccgtgtaat caccgatccc cggcaacgcg agcagcgcct ccaccgtatc    180
   cggcacctcg ccggcatgct tttcgacgat cacctccgca cattccttca acctcagcgc    240
   cctacgtgga tagcccaact tgccccacga ccgcaaaatc tcatcggtgc tcgcattcgc    300
   gaaatcttcc ggagtgggcc atttttccat ccactcacgc caaatcggct cgactcgcgc    360
   gacgggagtt tgttggctca tcacctctga aaggagaatt ccccatgctg aagtattggg    420
   atcacgccac gcaagatcgc gggcatttgc tctaaaccac acgagcaggg ctgtttgaaa    480
   agctgtaaat gacatgacct aaatgattgt actgactggc acattaggtc atacgtcaca    540
   ccgagtggaa gaatagagct t atg cct ttg cgt aat gtt gat aga act ccg       591
                           Met Pro Leu Arg Asn Val Asp Arg Thr Pro
                           1               5                   10
   ccc gca gta tgg gaa gca ctg ctt gcc gga aac gaa aga ttc atc agt       639
   Pro Ala Val Trp Glu Ala Leu Leu Ala Gly Asn Glu Arg Phe Ile Ser
                   15                  20                  25
   ttc aac gaa gat cga cca aac cag gac gcc ccg cgc aga aaa gaa ctt       687
   Phe Asn Glu Asp Arg Pro Asn Gln Asp Ala Pro Arg Arg Lys Glu Leu
               30                  35                  40
   cgc aat ggg caa acg cct gca gct gtt gtc att tcc tgt tca gat tct       735
   Arg Asn Gly Gln Thr Pro Ala Ala Val Val Ile Ser Cys Ser Asp Ser
           45                  50                  55
   cga gtg cca gtt gaa ttt att ttt gac gtc ggt ctc ggt gac ctc ttt       783
   Arg Val Pro Val Glu Phe Ile Phe Asp Val Gly Leu Gly Asp Leu Phe
       60                  65                  70
   gtt gtc cgt act gcc gga gaa atc ctc gac caa gca gtg ctt gca tcc       831
   Val Val Arg Thr Ala Gly Glu Ile Leu Asp Gln Ala Val Leu Ala Ser
   75                  80                  85                  90
   atc gaa tac gcc act gaa tcc atc ggc gtt cca ttg gtt atc gtc atg       879
   Ile Glu Tyr Ala Thr Glu Ser Ile Gly Val Pro Leu Val Ile Val Met
                   95                  100                 105
   ggc cac gaa tcc tgt ggt gca gtt gca gca act gca gca gca ctt gaa       927
   Gly His Glu Ser Cys Gly Ala Val Ala Ala Thr Ala Ala Ala Leu Glu
               110                 115                 120
   ggc ggt gca ctt ccc gga ggc tac caa cga gtt ttg gta gaa aag gtt       975
   Gly Gly Ala Leu Pro Gly Gly Tyr Gln Arg Val Leu Val Glu Lys Val
           125                 130                 135
   gca cca tcc att cta gaa gcc aag gca gag ggc ctg agc tcc atc aag      1023
   Ala Pro Ser Ile Leu Glu Ala Lys Ala Glu Gly Leu Ser Ser Ile Lys
       140                 145                 150
   gaa ttc gag gaa cac cac gtt gtg gca acg gta aac caa ctg ttg tcc      1071
   Glu Phe Glu Glu His His Val Val Ala Thr Val Asn Gln Leu Leu Ser
   155                 160                 165                 170
   cgt tct cca gag att cat cag aag gtc gaa acc ggc gag ttg gga atc      1119
   Arg Ser Pro Glu Ile His Gln Lys Val Glu Thr Gly Glu Leu Gly Ile
                   175                 180                 185
   att ggt ttg cgc tac cga ctc tct gac ggt cgt act gaa cct gta att      1167
   Ile Gly Leu Arg Tyr Arg Leu Ser Asp Gly Arg Thr Glu Pro Val Ile
               190                 195                 200
   agc aag aac gtg ggt tagttttcgg tctgagattg cctatgatcg gtgatttggg      1222
   Ser Lys Asn Val Gly
           205
   ggccgtgggc agtgtggttt ggtagttcgg gggactcaag ttagagaaac cagacaggcg   1282
   tgccaagact ctgggttttt ccaggttttg gaacgcctgt ccggtttaga ggtttagaga   1342
   ttgagaaccg gttgggaacc ggacacaggt gccagaagtt cggcttttta ggtaatcttg   1402
   gcacgccagt ttgatcgctg tggaaactat cacaattccc gtcctgaaca gcgaaaaagt   1462
   gacctcgca                                                           1471
<210> 12
   <211> 207
   <212> PRT
   <213> Corynebacterium glutamicum
<400> 12
   Met Pro Leu Arg Asn Val Asp Arg Thr Pro Pro Ala Val Trp Glu Ala
   1               5                   10                  15
   Leu Leu Ala Gly Asn Glu Arg Phe Ile Ser Phe Asn Glu Asp Arg Pro
               20                  25                  30
   Asn Gln Asp Ala Pro Arg Arg Lys Glu Leu Arg Asn Gly Gln Thr Pro
           35                  40                  45
   Ala Ala Val Val Ile Ser Cys Ser Asp Ser Arg Val Pro Val Glu Phe
       50                  55                  60
   Ile Phe Asp Val Gly Leu Gly Asp Leu Phe Val Val Arg Thr Ala Gly
   65                  70                  75                  80
   Glu Ile Leu Asp Gln Ala Val Leu Ala Ser Ile Glu Tyr Ala Thr Glu
                   85                  90                  95
   Ser Ile Gly Val Pro Leu Val Ile Val Met Gly His Glu Ser Cys Gly
               100                 105                 110
   Ala Val Ala Ala Thr Ala Ala Ala Leu Glu Gly Gly Ala Leu Pro Gly
           115                 120                 125
   Gly Tyr Gln Arg Val Leu Val Glu Lys Val Ala Pro Ser Ile Leu Glu
       130                 135                 140
   Ala Lys Ala Glu Gly Leu Ser Ser Ile Lys Glu Phe Glu Glu His His
   145                 150                 155                 160
   Val Val Ala Thr Val Asn Gln Leu Leu Ser Arg Ser Pro Glu Ile His
                   165                 170                 175
   Gln Lys Val Glu Thr Gly Glu Leu Gly Ile Ile Gly Leu Arg Tyr Arg
               180                 185                 190
   Leu Ser Asp Gly Arg Thr Glu Pro Val Ile Ser Lys Asn Val Gly
           195                 200                 205
<210> 13
   <211> 624
   <212> DNA
   <213> Corynebacterium glutamicum
<220>
   <221> CDS
   <222> (1)..(621)
<400> 13
   atg cct ttg cgt aat gtt gat aga act ccg ccc gca gta tgg gaa gca      48
   Met Pro Leu Arg Asn Val Asp Arg Thr Pro Pro Ala Val Trp Glu Ala
   1               5                   10                  15
   ttg ctt gcc gga aac gaa aga ttc atc agt ttc aac gaa gat cga cca      96
   Leu Leu Ala Gly Asn Glu Arg Phe Ile Ser Phe Asn Glu Asp Arg Pro
               20                  25                  30
   aac cag gac gcc ccg cgc aga aga gaa ctt cgc aat gga caa acg cct      144
   Asn Gln Asp Ala Pro Arg Arg Arg Glu Leu Arg Asn Gly Gln Thr Pro
           35                  40                  45
   gca gct gtt gtt att tcc tgt tca gat tct cga gtg cca gtt gag att      192
   Ala Ala Val Val Ile Ser Cys Ser Asp Ser Arg Val Pro Val Glu Ile
       50                  55                  60
   att ttt gac gtc ggt ctc ggt gac ctc ttt gtt gtc cgt act gcc gga      240
   Ile Phe Asp Val Gly Leu Gly Asp Leu Phe Val Val Arg Thr Ala Gly
   65                  70                  75                  80
   gaa atc ctc gac caa gca gtg ctt gcg tcc atc gaa tac gcc act gaa      288
   Glu Ile Leu Asp Gln Ala Val Leu Ala Ser Ile Glu Tyr Ala Thr Glu
                   85                  90                  95
   tcc atc ggc gtt cca ttg gtt atc gtc atg ggc cac gaa tcc tgt ggt      336
   Ser Ile Gly Val Pro Leu Val Ile Val Met Gly His Glu Ser Cys Gly
               100                 105                 110
   gca gtt gca gca act gca gca gca ctt gaa ggc ggt gca ctt ccc gga      384
   Ala Val Ala Ala Thr Ala Ala Ala Leu Glu Gly Gly Ala Leu Pro Gly
           115                 120                 125
   ggc tac caa cga gtt ttg gtt gaa aag gtt gca cca tcc att cta gaa      432
   Gly Tyr Gln Arg Val Leu Val Glu Lys Val Ala Pro Ser Ile Leu Glu
       130                 135                 140
   gcc aag gca gag ggc ctg agc tcc atc aag gaa ttc gag gaa cac cac      480
   Ala Lys Ala Glu Gly Leu Ser Ser Ile Lys Glu Phe Glu Glu His His
   145                 150                 155                 160
   gtt gtg gca acg gta aac caa ctg ttg tcc cgt tct cca gag att cat      528
   Val Val Ala Thr Val Asn Gln Leu Leu Ser Arg Ser Pro Glu Ile His
                   165                 170                 175
   cag aag gtc gaa acc ggt gag ttg gga atc att ggt ttg cgc tac cga      576
   Gln Lys Val Glu Thr Gly Glu Leu Gly Ile Ile Gly Leu Arg Tyr Arg
               180                 185                 190
   ctc tct gac ggt cgt act gaa cct gta att agc aag aac gtg ggt tag      624
   Leu Ser Asp Gly Arg Thr Glu Pro Val Ile Ser Lys Asn Val Gly
           195                 200                 205
<210> 14
   <211> 207
   <212> PRT
   <213> Corynebacterium glutamicum
<400> 14
   Met Pro Leu Arg Asn Val Asp Arg Thr Pro Pro Ala Val Trp Glu Ala
   1               5                   10                  15
   Leu Leu Ala Gly Asn Glu Arg Phe Ile Ser Phe Asn Glu Asp Arg Pro
               20                  25                  30
   Asn Gln Asp Ala Pro Arg Arg Arg Glu Leu Arg Asn Gly Gln Thr Pro
           35                  40                  45
   Ala Ala Val Val Ile Ser Cys Ser Asp Ser Arg Val Pro Val Glu Ile
       50                  55                  60
   Ile Phe Asp Val Gly Leu Gly Asp Leu Phe Val Val Arg Thr Ala Gly
   65                  70                  75                  80
   Glu Ile Leu Asp Gln Ala Val Leu Ala Ser Ile Glu Tyr Ala Thr Glu
                   85                  90                  95
   Ser Ile Gly Val Pro Leu Val Ile Val Met Gly His Glu Ser Cys Gly
               100                 105                 110
   Ala Val Ala Ala Thr Ala Ala Ala Leu Glu Gly Gly Ala Leu Pro Gly
           115                 120                 125
   Gly Tyr Gln Arg Val Leu Val Glu Lys Val Ala Pro Ser Ile Leu Glu
       130                 135                 140
   Ala Lys Ala Glu Gly Leu Ser Ser Ile Lys Glu Phe Glu Glu His His
   145                 150                 155                 160
   Val Val Ala Thr Val Asn Gln Leu Leu Ser Arg Ser Pro Glu Ile His
                   165                 170                 175
   Gln Lys Val Glu Thr Gly Glu Leu Gly Ile Ile Gly Leu Arg Tyr Arg
               180                 185                 190
   Leu Ser Asp Gly Arg Thr Glu Pro Val Ile Ser Lys Asn Val Gly
           195                 200                 205
<210> 15
   <211> 2367
   <212> DNA
   <213> Corynebacterium glutamicum
<220>
   <221> CDS
   <222> (1)..(2364)
<400> 15
   atg tct aca gat tac tca tca cca gca tat ttg caa aaa gtt gat aag      48
   Met Ser Thr Asp Tyr Ser Ser Pro Ala Tyr Leu Gln Lys Val Asp Lys
   1               5                   10                  15
   tac tgg cgt gct gcc aac tat tta tca gtt ggt caa ctt tat tta aaa      96
   Tyr Trp Arg Ala Ala Asn Tyr Leu Ser Val Gly Gln Leu Tyr Leu Lys
               20                  25                  30
   gat aat cct tta tta caa cgg cca tta aag gct agt gac gtt aag gtt     144
   Asp Asn Pro Leu Leu Gln Arg Pro Leu Lys Ala Ser Asp Val Lys Val
           35                  40                  45
   cac cca atc ggt cac tgg ggc acg att gcc ggc caa aac ttc atc tat     192
   His Pro Ile Gly His Trp Gly Thr Ile Ala Gly Gln Asn Phe Ile Tyr
       50                  55                  60
   gcg cat ctt aac cgg gtc atc aac aag tac ggt ttg aag atg ttc tac     240
   Ala His Leu Asn Arg Val Ile Asn Lys Tyr Gly Leu Lys Met Phe Tyr
   65                  70                  75                  80
   gtt gaa ggt cca ggt cat ggt ggc caa gtg atg gtc tcc aac tca tac     288
   Val Glu Gly Pro Gly His Gly Gly Gln Val Met Val Ser Asn Ser Tyr
                   85                  90                  95
   ctt gat ggg act tac acg gat att tat cct gaa att acg cag gat gtt     336
   Leu Asp Gly Thr Tyr Thr Asp Ile Tyr Pro Glu Ile Thr Gln Asp Val
               100                 105                 110
   gaa ggg atg caa aaa ctc ttc aag caa ttc tca ttc cca ggt ggc gtg     384
   Glu Gly Met Gln Lys Leu Phe Lys Gln Phe Ser Phe Pro Gly Gly Val
           115                 120                 125
   gct tcc cat gct gct cct gaa aca cca ggc tca atc cac gaa ggt ggc     432
   Ala Ser His Ala Ala Pro Glu Thr Pro Gly Ser Ile His Glu Gly Gly
       130                 135                 140
   gaa ctt ggt tac tca att tca cac ggt gtt ggg gca atc ctt gac aac     480
   Glu Leu Gly Tyr Ser Ile Ser His Gly Val Gly Ala Ile Leu Asp Asn
   145                 150                 155                 160
   cct gat gaa atc gcc gca gtc gtt gtt ggt gat ggg gaa tcc gaa acc     528
   Pro Asp Glu Ile Ala Ala Val Val Val Gly Asp Gly Glu Ser Glu Thr
                   165                 170                 175
   ggc cca tta gca act tca tgg caa tca acg aag ttc atc aac cca atc     576
   Gly Pro Leu Ala Thr Ser Trp Gln Ser Thr Lys Phe Ile Asn Pro Ile
               180                 185                 190
   aac gat ggg gca gtg tta cca atc ttg aac ctt aac ggc ttt aag att      624
   Asn Asp Gly Ala Val Leu Pro Ile Leu Asn Leu Asn Gly Phe Lys Ile
           195                 200                 205
   tct aac cca acg att ttt ggt cgg act tct gat gaa aag atc aag caa      672
   Ser Asn Pro Thr Ile Phe Gly Arg Thr Ser Asp Glu Lys Ile Lys Gln
       210                 215                 220
   tac ttc gaa agc atg aac tgg gaa cca atc ttt gtt gaa ggt gac gat      720
   Tyr Phe Glu Ser Met Asn Trp Glu Pro Ile Phe Val Glu Gly Asp Asp
   225                 230                 235                 240
   cct gaa aag gtt cac cca gct tta gct aag gcc atg gat gaa gcc gtc      768
   Pro Glu Lys Val His Pro Ala Leu Ala Lys Ala Met Asp Glu Ala Val
                   245                 250                 255
   gaa aag atc aaa gcc att caa aag aac gct cgt gaa aac gat gac gct      816
   Glu Lys Ile Lys Ala Ile Gln Lys Asn Ala Arg Glu Asn Asp Asp Ala
               260                 265                 270
   act tta cca gta tgg ccg atg atc gtc ttc cgc gca cct aag ggc tgg      864
   Thr Leu Pro Val Trp Pro Met Ile Val Phe Arg Ala Pro Lys Gly Trp
           275                 280                 285
   act ggt cct aag tca tgg gat ggc gac aag atc gaa ggt tca ttc cga      912
   Thr Gly Pro Lys Ser Trp Asp Gly Asp Lys Ile Glu Gly Ser Phe Arg
       290                 295                 300
   gct cac caa att cca att cct gtt gac caa acc gac atg gaa cat gcc      960
   Ala His Gln Ile Pro Ile Pro Val Asp Gln Thr Asp Met Glu His Ala
   305                 310                 315                 320
   gat gcg tta gtt gac tgg ttg gaa tca tat caa cca aag gaa ctc ttc     1008
   Asp Ala Leu Val Asp Trp Leu Glu Ser Tyr Gln Pro Lys Glu Leu Phe
                   325                 330                 335
   aat gaa gat ggt tct ttg aag gat gat atc aaa gaa att atc cca act     1056
   Asn Glu Asp Gly Ser Leu Lys Asp Asp Ile Lys Glu Ile Ile Pro Thr
               340                 345                 350
   ggc gat gca cgg atg gcc gct aac cca atc act aat ggt ggg gtt gat     1104
   Gly Asp Ala Arg Met Ala Ala Asn Pro Ile Thr Asn Gly Gly Val Asp
           355                 360                 365
   cca aag gcc ttg aac tta cct aac ttc cgt gat tac gcc gtt gat acg     1152
   Pro Lys Ala Leu Asn Leu Pro Asn Phe Arg Asp Tyr Ala Val Asp Thr
       370                 375                 380
   tct aag cat ggt gcc aac gtt aag caa gat atg atc gtt tgg tca gac     1200
   Ser Lys His Gly Ala Asn Val Lys Gln Asp Met Ile Val Trp Ser Asp
   385                 390                 395                 400
   tac ttg cgt gat gtt atc aag aag aac cca gat aac ttc cgg tta ttt     1248
   Tyr Leu Arg Asp Val Ile Lys Lys Asn Pro Asp Asn Phe Arg Leu Phe
                   405                 410                 415
   ggc cct gat gaa acc atg tca aac cgg tta tat ggt gtc ttt gaa acc     1296
   Gly Pro Asp Glu Thr Met Ser Asn Arg Leu Tyr Gly Val Phe Glu Thr
               420                 425                 430
   act aac cgt caa tgg atg gaa gat att cac cca gat agt gac caa tac     1344
   Thr Asn Arg Gln Trp Met Glu Asp Ile His Pro Asp Ser Asp Gln Tyr
           435                 440                 445
   gaa gca cct gct ggc cgg gtc ttg gat gct caa tta tct gaa cac caa     1392
   Glu Ala Pro Ala Gly Arg Val Leu Asp Ala Gln Leu Ser Glu His Gln
       450                 455                 460
   gct gaa ggt tgg tta gaa ggt tac gtc tta act ggt cgt cat ggc ttg     1440
   Ala Glu Gly Trp Leu Glu Gly Tyr Val Leu Thr Gly Arg His Gly Leu
   465                 470                 475                 480
   ttt gca agt tac gaa gcc ttc tta cgg gtt gtc gac tca atg ttg acg     1488
   Phe Ala Ser Tyr Glu Ala Phe Leu Arg Val Val Asp Ser Met Leu Thr
                   485                 490                 495
   caa cac ttc aag tgg tta cgt aag gcc aac gaa ctt gac tgg cgg aag     1536
   Gln His Phe Lys Trp Leu Arg Lys Ala Asn Glu Leu Asp Trp Arg Lys
               500                 505                 510
   aag tac ccg tca ctc aac att atc gcg gct tca act gtg ttc caa caa     1584
   Lys Tyr Pro Ser Leu Asn Ile Ile Ala Ala Ser Thr Val Phe Gln Gln
           515                 520                 525
   gac cat aat ggg tac acc cac caa gat cca ggt gcc ttg act cat ttg      1632
   Asp His Asn Gly Tyr Thr His Gln Asp Pro Gly Ala Leu Thr His Leu
       530                 535                 540
   gct gaa aag aag cct gaa tat atc cgc gaa tat tta cca gcc gac gcc      1680
   Ala Glu Lys Lys Pro Glu Tyr Ile Arg Glu Tyr Leu Pro Ala Asp Ala
   545                 550                 555                 560
   aac tcc ttg tta gct gtt ggg gac gtc atc ttc cgt agc caa gaa aag      1728
   Asn Ser Leu Leu Ala Val Gly Asp Val Ile Phe Arg Ser Gln Glu Lys
                   565                 570                 575
   atc aac tac gtg gtt acg tcg aag cac cca cgt caa caa tgg ttc agc      1776
   Ile Asn Tyr Val Val Thr Ser Lys His Pro Arg Gln Gln Trp Phe Ser
               580                 585                 590
   att gaa gaa gct aag caa tta gtt gac aac ggt ctt ggt atc att gac      1824
   Ile Glu Glu Ala Lys Gln Leu Val Asp Asn Gly Leu Gly Ile Ile Asp
           595                 600                 605
   tgg gca agc acg gac caa ggt agc gaa cca gat atc gtg ttt gct gct      1872
   Trp Ala Ser Thr Asp Gln Gly Ser Glu Pro Asp Ile Val Phe Ala Ala
       610                 615                 620
   gcc gga acg gaa cca acg ctt gaa acg ttg gct gca atc caa ttg ctc      1920
   Ala Gly Thr Glu Pro Thr Leu Glu Thr Leu Ala Ala Ile Gln Leu Leu
   625                 630                 635                 640
   cat gat agc ttc cca gac atg aag att cgt ttc gtg aac gtg gtc gac      1968
   His Asp Ser Phe Pro Asp Met Lys Ile Arg Phe Val Asn Val Val Asp
                   645                 650                 655
   atc ttg aag tta cgt agc cct gaa aag gac cct cgt ggc ttg tca gat      2016
   Ile Leu Lys Leu Arg Ser Pro Glu Lys Asp Pro Arg Gly Leu Ser Asp
               660                 665                 670
   gct gaa ttt gac cat tac ttc act aag gac aaa cca gtt gtc ttc gcc      2064
   Ala Glu Phe Asp His Tyr Phe Thr Lys Asp Lys Pro Val Val Phe Ala
           675                 680                 685
   ttc cat ggt tac gaa gac ctg gtt cgt gac atc ttc ttt gat cgt cac      2112
   Phe His Gly Tyr Glu Asp Leu Val Arg Asp Ile Phe Phe Asp Arg His
       690                 695                 700
   aac cac aac tta cac gtg cat ggc tac cgt gaa aat ggt gac att acg      2160
   Asn His Asn Leu His Val His Gly Tyr Arg Glu Asn Gly Asp Ile Thr
   705                 710                 715                 720
   aca cca ttc gat gtc cgg gtc atg aac caa atg gac cgt ttc gac tta      2208
   Thr Pro Phe Asp Val Arg Val Met Asn Gln Met Asp Arg Phe Asp Leu
                   725                 730                 735
   gca aaa tct gca att gcg gcg caa cca gca atg gaa aac acc ggt gca      2256
   Ala Lys Ser Ala Ile Ala Ala Gln Pro Ala Met Glu Asn Thr Gly Ala
               740                 745                 750
   gcc ttt gtt caa gac atg gat aac atg ctt gca aaa cac aac gca tac      2304
   Ala Phe Val Gln Asp Met Asp Asn Met Leu Ala Lys His Asn Ala Tyr
           755                 760                 765
   atc cgt gac gcc gga acc gac ttg cca gaa gtt aac gac tgg caa tgg      2352
   Ile Arg Asp Ala Gly Thr Asp Leu Pro Glu Val Asn Asp Trp Gln Trp
       770                 775                 780
   aaa ggt ttg aaa taa                                                  2367
   Lys Gly Leu Lys
   785
<210> 16
   <211> 788
   <212> PRT
   <213> Corynebacterium glutamicum
<400> 16
   Met Ser Thr Asp Tyr Ser Ser Pro Ala Tyr Leu Gln Lys Val Asp Lys
   1               5                   10                  15
   Tyr Trp Arg Ala Ala Asn Tyr Leu Ser Val Gly Gln Leu Tyr Leu Lys
               20                  25                  30
   Asp Asn Pro Leu Leu Gln Arg Pro Leu Lys Ala Ser Asp Val Lys Val
           35                  40                  45
   His Pro Ile Gly His Trp Gly Thr Ile Ala Gly Gln Asn Phe Ile Tyr
   50 55 60
   Ala His Leu Asn Arg Val Ile Asn Lys Tyr Gly Leu Lys Met Phe Tyr
   65 70 75 80
   Val Glu Gly Pro Gly His Gly Gly Gln Val Met Val Ser Asn Ser Tyr
   85 90 95
   Leu Asp Gly Thr Tyr Thr Asp Ile Tyr Pro Glu Ile Thr Gln Asp Val
   100 105 110
   Glu Gly Met Gln Lys Leu Phe Lys Gln Phe Ser Phe Pro Gly Gly Val
   115 120 125
   Ala Ser His Ala Ala Pro Glu Thr Pro Gly Ser Ile His Glu Gly Gly
   130 135 140
   Glu Leu Gly Tyr Ser Ile Ser His Gly Val Gly Ala Ile Leu Asp Asn
   145 150 155 160
   Pro Asp Glu Ile Ala Ala Val Val Val Gly Asp Gly Glu Ser Glu Thr
   165 170 175
   Gly Pro Leu Ala Thr Ser Trp Gln Ser Thr Lys Phe Ile Asn Pro Ile
   180 185 190
   Asn Asp Gly Ala Val Leu Pro Ile Leu Asn Leu Asn Gly Phe Lys Ile
   195 200 205
   Ser Asn Pro Thr Ile Phe Gly Arg Thr Ser Asp Glu Lys Ile Lys Gln
   210 215 220
   Tyr Phe Glu Ser Met Asn Trp Glu Pro Ile Phe Val Glu Gly Asp Asp
   225 230 235 240
   Pro Glu Lys Val His Pro Ala Leu Ala Lys Ala Met Asp Glu Ala Val
   245 250 255
   Glu Lys Ile Lys Ala Ile Gln Lys Asn Ala Arg Glu Asn Asp Asp Ala
   260 265 270
   Thr Leu Pro Val Trp Pro Met Ile Val Phe Arg Ala Pro Lys Gly Trp
   275 280 285
   Thr Gly Pro Lys Ser Trp Asp Gly Asp Lys Ile Glu Gly Ser Phe Arg
   290 295 300
   Ala His Gln Ile Pro Ile Pro Val Asp Gln Thr Asp Met Glu His Ala
   305 310 315 320
   Asp Ala Leu Val Asp Trp Leu Glu Ser Tyr Gln Pro Lys Glu Leu Phe
   325 330 335
   Asn Glu Asp Gly Ser Leu Lys Asp Asp Ile Lys Glu Ile Ile Pro Thr
   340 345 350
   Gly Asp Ala Arg Met Ala Ala Asn Pro Ile Thr Asn Gly Gly Val Asp
   355 360 365
   Pro Lys Ala Leu Asn Leu Pro Asn Phe Arg Asp Tyr Ala Val Asp Thr
   370 375 380
   Ser Lys His Gly Ala Asn Val Lys Gln Asp Met Ile Val Trp Ser Asp
   385 390 395 400
   Tyr Leu Arg Asp Val Ile Lys Lys Asn Pro Asp Asn Phe Arg Leu Phe
   405 410 415
   Gly Pro Asp Glu Thr Met Ser Asn Arg Leu Tyr Gly Val Phe Glu Thr
   420 425 430
   Thr Asn Arg Gln Trp Met Glu Asp Ile His Pro Asp Ser Asp Gln Tyr
   435 440 445
   Glu Ala Pro Ala Gly Arg Val Leu Asp Ala Gln Leu Ser Glu His Gln
   450 455 460
   Ala Glu Gly Trp Leu Glu Gly Tyr Val Leu Thr Gly Arg His Gly Leu
   465 470 475 480
   Phe Ala Ser Tyr Glu Ala Phe Leu Arg Val Val Asp Ser Met Leu Thr
   485 490 495
   Gln His Phe Lys Trp Leu Arg Lys Ala Asn Glu Leu Asp Trp Arg Lys
   500 505 510
   Lys Tyr Pro Ser Leu Asn Ile Ile Ala Ala Ser Thr Val Phe Gln Gln
   515 520 525
   Asp His Asn Gly Tyr Thr His Gln Asp Pro Gly Ala Leu Thr His Leu
   530 535 540
   Ala Glu Lys Lys Pro Glu Tyr Ile Arg Glu Tyr Leu Pro Ala Asp Ala
   545 550 555 560
   Asn Ser Leu Leu Ala Val Gly Asp Val Ile Phe Arg Ser Gln Glu Lys
                           565                     570                     575
   Ile Asn Tyr Val Val Thr Ser Lys His Pro Arg Gln Gln Trp Phe Ser
                   580                     585                     590
   Ile Glu Glu Ala Lys Gln Leu Val Asp Asn Gly Leu Gly Ile Ile Asp
               595                     600                     605
   Trp Ala Ser Thr Asp Gln Gly Ser Glu Pro Asp Ile Val Phe Ala Ala
       610                     615                     620
   Ala Gly Thr Glu Pro Thr Leu Glu Thr Leu Ala Ala Ile Gln Leu Leu
   625                     630                     635                     640
   His Asp Ser Phe Pro Asp Met Lys Ile Arg Phe Val Asn Val Val Asp
                   645                     650                     655
   Ile Leu Lys Leu Arg Ser Pro Glu Lys Asp Pro Arg Gly Leu Ser Asp
               660                     665                     670
   Ala Glu Phe Asp His Tyr Phe Thr Lys Asp Lys Pro Val Val Phe Ala
           675                     680                     685
   Phe His Gly Tyr Glu Asp Leu Val Arg Asp Ile Phe Phe Asp Arg His
       690                     695                     700
   Asn His Asn Leu His Val His Gly Tyr Arg Glu Asn Gly Asp Ile Thr
   705                     710                     715                     720
   Thr Pro Phe Asp Val Arg Val Met Asn Gln Met Asp Arg Phe Asp Leu
                   725                     730                     735
   Ala Lys Ser Ala Ile Ala Ala Gln Pro Ala Met Glu Asn Thr Gly Ala
               740                     745                     750
   Ala Phe Val Gln Asp Met Asp Asn Met Leu Ala Lys His Asn Ala Tyr
           755                     760                     765
   Ile Arg Asp Ala Gly Thr Asp Leu Pro Glu Val Asn Asp Trp Gln Trp
       770                     775                     780
   Lys Gly Leu Lys
   785
<210> 17
   <211> 2367
   <212> DNA
   <213> Lactobacillus plantarum
<220>
   <221> CDS
   <222> (1)..(2364)
<400> 17
   atg aca aca gat tac tca tca cca gca tat ttg caa aaa gtt gat aag       48
   tac tgg cgt gct gcc aac tac tta tca gtt ggt caa ctt tat tta aaa       96
   gat aat cca cta tta caa cgg cca ttg aag gcc agt gac gtt aag gtt      144
   cat cca att ggt cac tgg ggg acg att gcc ggt caa aac ttt atc tat      192
   gct cat ctt aac cgg gtc atc aac aag tac ggt ttg aag atg ttc tac      240
   gtt gaa ggt cca ggt cat ggt ggt caa gtg atg gtt tca aac tct tac      288
   ctt gac ggt act tac acc gat att tat cca gaa att acg cag gat gtt      336
   gaa ggg atg caa aag ctc ttc aag caa ttc tca ttc cca ggt ggg gtt      384
   gct tcc cat gcg gca cct gaa aca ccc ggt tca atc cac gaa ggt ggc      432
   gaa ctt ggt tac tca att tca cac ggg gtt ggg gca att ctt gac aat      480
   cct gac gaa atc gcc gcg gtt gtt gtt ggt gat ggg gaa tcc gaa acg      528
   ggt cca tta gca act tca tgg caa tca acg aag ttc att aac cca atc      576
   aac gac ggg gct gtt tta cca atc ttg aac tta aat ggt ttt aag att      624
   tct aat cca acg att ttt ggt cgg act tct gat gct aag att aag gaa      672
   tac ttc gaa agc atg aat tgg gaa cca atc ttc gtt gaa ggt gac gat      720
   cct gaa aag gtt cac cca gcc tta gct aag gcc atg gat gaa gcc gtt      768
   gaa aag atc aag gca atc cag aag cat gct cgc gaa aat aac gat gca      816
   aca ttg cca gta tgg cca atg atc gtc ttc cgc gca cct aag ggc tgg      864
   act ggt ccg aag tca tgg gac ggt gat aag atc gaa ggt tca ttc cgt      912
   gct cat caa att ccg att cct gtt gat caa aat gac atg gaa cat gcg      960
   gat gct tta gtt gat tgg ctc gaa tca tat caa cca aaa gaa ctc ttc     1008
   aat gaa gat ggc tct ttg aag gat gat att aaa gaa att att cct act     1056
   ggg gac agt cgg atg gct gct aac cca atc acc aat ggt ggg gtc gat     1104
   ccg aaa gcc ttg aac tta cca aac ttc cgt gat tat gcg gtc gat acg     1152
   tcc aaa gaa ggc gcg aat gtt aag caa gat atg atc gtt tgg tca gac     1200
   tat ttg cgg gat gtc atc aag aaa aat cct gat aac ttc cgg ttg ttc     1248
   gga cct gat gaa acc atg tct aac cgt tta tat ggt gtc ttc gaa acc     1296
   act aat cgt caa tgg atg gaa gac att cat cca gat agt gac caa tat     1344
   gaa gca cca gct ggc cgg gtc tta gat gct cag tta tct gaa cac caa     1392
   gct gaa ggt tgg tta gaa ggt tac gtc tta act gga cgt cat ggg tta     1440
   ttt gcc agt tat gaa gcc ttc cta cgc gtt gtg gac tca atg ttg acg      1488
   caa cac ttc aag tgg tta cgt aaa gcc aat gaa ctt gat tgg cgt aaa      1536
   aag tac cca tca ctt aac att atc gcg gct tca act gta ttc caa caa      1584
   gac cat aat ggt tat acc cac caa gat cca ggt gca tta act cat ttg      1632
   gcc gaa aag aaa cca gaa tac att cgt gaa tat tta cca gcc gat gcc      1680
   aac acg tta tta gct gtc ggt gac gtc att ttc cgg agc caa gaa aag      1728
   atc aac tac gtg gtt acg tca aaa cac cca cgt caa caa tgg ttc agc      1776
   att gaa gaa gct aag caa tta gtt gac aat ggt ctt ggt atc att gat      1824
   tgg gca agt acg gac caa ggt agc gaa cca gac att gtc ttt gca gct      1872
   gct ggg acg gaa cca acg ctt gaa acg ttg gct gcc atc caa tta cta      1920
   cac gac agt ttc cca gag atg aag att cgt ttc gtg aac gtg gtc gac      1968
   atc ttg aag tta cgt agt cct gaa aag gat ccg cgg ggc ttg tca gat      2016
   gct gag ttt gac cat tac ttt act aag gac aaa cca gtg gtc ttt gct      2064
   ttc cac ggt tac gaa gac tta gtt cgt gac atc ttc ttt gat cgt cac      2112
   aac cat aac tta tac gtc cac ggt tac cgt gaa aat ggt gat att acc      2160
   aca cca ttc gac gta cgg gtc atg aac cag atg gac cgc ttc gac tta      2208
   gct aag tcg gca att gcg gcg caa cca gca atg gaa aac act ggt gcg      2256
   gcc ttc gtt caa tcc atg gat aat atg ctt gct aaa cac aat gcc tat      2304
   atc cgg gat gcc gga act gac ttg cca gaa gtt aat gat tgg caa tgg      2352
   aag ggt tta aaa taa                                                  2367
<210> 18
   <211> 788
   <212> PRT
   <213> Lactobacillus plantarum
<400> 18
<210> 19
   <211> 2660
   <212> DNA
   <213> Bifidobacterium lactis
<220>
   <221> CDS
   <222> (128)..(2602)
<400> 19
   aggtcagcgt attcgcgtaa cataatcagc gatcgggcac ggagaccggc ctgcaggaca     60
   gcgccgaagc ccgtgcccaa cggaataaac aaatcgcaca tttatgtgca ggagtacagg    120
   agcacac atg act aat cct gtt att ggt acc cca tgg cag aag ctg gat      169
           Met Thr Asn Pro Val Ile Gly Thr Pro Trp Gln Lys Leu Asp
           1               5                   10
   cgt ccg gtt tcc gaa gag gcc atc gaa ggc atg gac aag tac tgg cgc      217
   Arg Pro Val Ser Glu Glu Ala Ile Glu Gly Met Asp Lys Tyr Trp Arg
   15                  20                  25                  30
   gtc gcc aac tac atg tct atc ggc cag atc tac ctg cgt agc aac ccg      265
   Val Ala Asn Tyr Met Ser Ile Gly Gln Ile Tyr Leu Arg Ser Asn Pro
                   35                  40                  45
   ctg atg aag gag ccc ttc acc cgc gat gac gtg aag cac cgt ctg gtc      313
   Leu Met Lys Glu Pro Phe Thr Arg Asp Asp Val Lys His Arg Leu Val
               50                  55                  60
   ggc cac tgg ggc acc acc ccg ggc ctg aac ttc ctt ctc gcc cac atc      361
   Gly His Trp Gly Thr Thr Pro Gly Leu Asn Phe Leu Leu Ala His Ile
           65                  70                  75
   aac cgc ctg atc gcc gat cac cag cag aac acc gtg ttc atc atg ggt      409
   Asn Arg Leu Ile Ala Asp His Gln Gln Asn Thr Val Phe Ile Met Gly
       80                  85                  90
   cct ggc cac ggc ggc cct gca ggt acc gct cag tcc tac atc gac ggc      457
   Pro Gly His Gly Gly Pro Ala Gly Thr Ala Gln Ser Tyr Ile Asp Gly
   95                  100                 105                 110
   acc tac acc gag tac tac ccg aac atc acc aag gac gaa gct ggc ctg      505
   Thr Tyr Thr Glu Tyr Tyr Pro Asn Ile Thr Lys Asp Glu Ala Gly Leu
                   115                 120                 125
   cag aag ttc ttc cgc cag ttc tcc tac ccg ggt ggc att cct tcc cac      553
   Gln Lys Phe Phe Arg Gln Phe Ser Tyr Pro Gly Gly Ile Pro Ser His
               130                 135                 140
   ttc gct ccg gag acg ccg ggc tcc atc cac gaa ggc ggc gag ctg ggc      601
   Phe Ala Pro Glu Thr Pro Gly Ser Ile His Glu Gly Gly Glu Leu Gly
           145                 150                 155
   tac gcc ctg tcg cac gcc tac ggc gcg atc atg gac aac ccg agc ctc      649
   Tyr Ala Leu Ser His Ala Tyr Gly Ala Ile Met Asp Asn Pro Ser Leu
       160                 165                 170
   ttc gtc ccg tgc atc atc ggt gac ggc gaa gcc gag acc ggc cct ctg      697
   Phe Val Pro Cys Ile Ile Gly Asp Gly Glu Ala Glu Thr Gly Pro Leu
   175                 180                 185                 190
   gcc acc ggc tgg cag tcc aac aag ctc gtc aac ccg cgc acc gac ggc      745
   Ala Thr Gly Trp Gln Ser Asn Lys Leu Val Asn Pro Arg Thr Asp Gly
                   195                 200                 205
   atc gtc ctg ccg atc ctg cac ctc aac ggc tac aag atc gcc aac ccg      793
   Ile Val Leu Pro Ile Leu His Leu Asn Gly Tyr Lys Ile Ala Asn Pro
               210                 215                 220
   acg atc ctc gcc cgc atc tcc gac gag gag ctg cac gac ttc ttc cgc      841
   Thr Ile Leu Ala Arg Ile Ser Asp Glu Glu Leu His Asp Phe Phe Arg
           225                 230                 235
   ggc atg ggt tac cac ccg tac gag ttc gtc gcc ggc ttc gac aac gag      889
   Gly Met Gly Tyr His Pro Tyr Glu Phe Val Ala Gly Phe Asp Asn Glu
       240                 245                 250
   gat cac ctg tcg atc cac cgt cgc ttc gcc gag ctc ttc gag acc atc      937
   Asp His Leu Ser Ile His Arg Arg Phe Ala Glu Leu Phe Glu Thr Ile
   255                 260                 265                 270
   ttc gac gag atc tgc gat atc aag gct gcg gct cag acc gac gac atg      985
   Phe Asp Glu Ile Cys Asp Ile Lys Ala Ala Ala Gln Thr Asp Asp Met
                   275                 280                 285
   acc cgt ccg ttc tac ccg atg ctc atc ttc cgc acc ccg aag ggc tgg     1033
   Thr Arg Pro Phe Tyr Pro Met Leu Ile Phe Arg Thr Pro Lys Gly Trp
               290                 295                 300
   acc tgc ccg aag ttc atc gac ggc aag aag acc gaa ggc tcc tgg cgt     1081
   Thr Cys Pro Lys Phe Ile Asp Gly Lys Lys Thr Glu Gly Ser Trp Arg
           305                 310                 315
   gca cac cag gtc ccg ctg gct tcc gcc cgc gac acc gag gcc cac ttc     1129
   Ala His Gln Val Pro Leu Ala Ser Ala Arg Asp Thr Glu Ala His Phe
       320                 325                 330
   gaa gtc ctc aag ggc tgg atg gaa tcc tac aag ccg gag gag ctc ttc     1177
   Glu Val Leu Lys Gly Trp Met Glu Ser Tyr Lys Pro Glu Glu Leu Phe
   335                 340                 345                 350
   aac gcc gac ggc tcc atc aag gag gac gtc acc gca ttc atg cct aag     1225
   Asn Ala Asp Gly Ser Ile Lys Glu Asp Val Thr Ala Phe Met Pro Lys
                   355                 360                 365
   ggc gaa ctg cgc atc ggc gcc aac ccg aat gcc aac ggc ggc cgc atc     1273
   Gly Glu Leu Arg Ile Gly Ala Asn Pro Asn Ala Asn Gly Gly Arg Ile
               370                 375                 380
   cgc gag gat ctg aag ctc cct gag ctc gat cag tac gag atc acc ggc     1321
   Arg Glu Asp Leu Lys Leu Pro Glu Leu Asp Gln Tyr Glu Ile Thr Gly
           385                 390                 395
   gtc aag gaa tac ggc cac ggt tgg ggc cag gtc gag gct ccg cgt tcc      1369
   Val Lys Glu Tyr Gly His Gly Trp Gly Gln Val Glu Ala Pro Arg Ser
       400                 405                 410
   ctc ggc gcg tac tgc cgc gac atc atc aag aac aac ccg gat tcg ttc      1417
   Leu Gly Ala Tyr Cys Arg Asp Ile Ile Lys Asn Asn Pro Asp Ser Phe
   415                 420                 425                 430
   cgc gtc ttc gga cct gac gag acc gcg tcc aac cgt ctg aac gcg acc      1465
   Arg Val Phe Gly Pro Asp Glu Thr Ala Ser Asn Arg Leu Asn Ala Thr
                   435                 440                 445
   tac gag gtc acc aag aag cag tgg gac aac gga tac ctc tcg gct ctc      1513
   Tyr Glu Val Thr Lys Lys Gln Trp Asp Asn Gly Tyr Leu Ser Ala Leu
               450                 455                 460
   gtc gac gag aac atg gcc gtc acc ggc cag gtt gtc gag cag ctc tcc      1561
   Val Asp Glu Asn Met Ala Val Thr Gly Gln Val Val Glu Gln Leu Ser
           465                 470                 475
   gag cat cag tgc gaa ggc ttc ctc gag gcc tac ctg ctc acc ggc cgt      1609
   Glu His Gln Cys Glu Gly Phe Leu Glu Ala Tyr Leu Leu Thr Gly Arg
       480                 485                 490
   cac ggc atc tgg agc tcc tac gag tcc ttc gtg cac gtg atc gac tcc      1657
   His Gly Ile Trp Ser Ser Tyr Glu Ser Phe Val His Val Ile Asp Ser
   495                 500                 505                 510
   atg ctg aac cag cat gcg aag tgg ctc gag gcc acc gtc cgc gag atc      1705
   Met Leu Asn Gln His Ala Lys Trp Leu Glu Ala Thr Val Arg Glu Ile
                   515                 520                 525
   ccg tgg cgt aag ccg atc tcc tcg gtg aac ctc ctg gtc tcc tcg cac      1753
   Pro Trp Arg Lys Pro Ile Ser Ser Val Asn Leu Leu Val Ser Ser His
               530                 535                 540
   gtg tgg cgt cag gat cac aac ggc ttc tcg cac cag gat ccg ggt gtg      1801
   Val Trp Arg Gln Asp His Asn Gly Phe Ser His Gln Asp Pro Gly Val
           545                 550                 555
   acc tcc gtc ctg ctg aac aag acg ttc aac aac gac cac gtg acg aac      1849
   Thr Ser Val Leu Leu Asn Lys Thr Phe Asn Asn Asp His Val Thr Asn
       560                 565                 570
   atc tac ttc gcg acc gat gcc aac atg ctg ctg gcc atc gcc gag aag      1897
   Ile Tyr Phe Ala Thr Asp Ala Asn Met Leu Leu Ala Ile Ala Glu Lys
   575                 580                 585                 590
   tgc ttc aag tcc acc aac aag atc aac gca atc ttc gcc ggc aag cag      1945
   Cys Phe Lys Ser Thr Asn Lys Ile Asn Ala Ile Phe Ala Gly Lys Gln
                   595                 600                 605
   ccg gcc gcg acg tgg atc acc ctc gac gag gta cgc gcc gag ctc gag      1993
   Pro Ala Ala Thr Trp Ile Thr Leu Asp Glu Val Arg Ala Glu Leu Glu
               610                 615                 620
   gct ggt gcc gcc gag tgg aag tgg gct tcc aac gcc aag agc aac gac      2041
   Ala Gly Ala Ala Glu Trp Lys Trp Ala Ser Asn Ala Lys Ser Asn Asp
           625                 630                 635
   gag gtc cag gtt gtc ctc gcc gcc gcc ggc gac gtc ccg acc cag gag      2089
   Glu Val Gln Val Val Leu Ala Ala Ala Gly Asp Val Pro Thr Gln Glu
       640                 645                 650
   atc atg gcc gct tcc gat gcc ctc aac aag atg ggc atc aag ttc aag      2137
   Ile Met Ala Ala Ser Asp Ala Leu Asn Lys Met Gly Ile Lys Phe Lys
   655                 660                 665                 670
   gtc gtc aac gtc gtg gac ctc atc aag ctg cag tcc tcg aag gag aac      2185
   Val Val Asn Val Val Asp Leu Ile Lys Leu Gln Ser Ser Lys Glu Asn
                   675                 680                 685
   gac gag gcc atg tct gac gag gac ttc gcc gac ctg ttc acc gcg gac      2233
   Asp Glu Ala Met Ser Asp Glu Asp Phe Ala Asp Leu Phe Thr Ala Asp
               690                 695                 700
   aag ccg gtc ctc ttc gcc tac cac tcc tat gcc cag gac gtt cgt ggc      2281
   Lys Pro Val Leu Phe Ala Tyr His Ser Tyr Ala Gln Asp Val Arg Gly
           705                 710                 715
   ctc atc tac gac cgc ccg aac cac gac aac ttc acc gtt gtc gga tac      2329
   Leu Ile Tyr Asp Arg Pro Asn His Asp Asn Phe Thr Val Val Gly Tyr
       720                 725                 730
   aag gag cag ggc tcc acg acg acg ccg ttc gac atg gtg cgt gtc aac      2377
   Lys Glu Gln Gly Ser Thr Thr Thr Pro Phe Asp Met Val Arg Val Asn
   735                 740                 745                 750
   gac atg gat cgc tac gcc ctt cag gcc aag gcc ctc gag ctc atc gac      2425
   Asp Met Asp Arg Tyr Ala Leu Gln Ala Lys Ala Leu Glu Leu Ile Asp
                   755                 760                 765
   gcc gac aag tat gcc gac aag atc aac gag ctc aac gag ttc cgc aag      2473
   Ala Asp Lys Tyr Ala Asp Lys Ile Asn Glu Leu Asn Glu Phe Arg Lys
               770                 775                 780
   acc gcg ttc cag ttc gcc gtc gac aat ggc tat gac att cct gag ttc      2521
   Thr Ala Phe Gln Phe Ala Val Asp Asn Gly Tyr Asp Ile Pro Glu Phe
           785                 790                 795
   acc gat tgg gtg tac ccg gat gtc aag gtc gac gag acc tcc atg ctc      2569
   Thr Asp Trp Val Tyr Pro Asp Val Lys Val Asp Glu Thr Ser Met Leu
       800                 805                 810
   tcc gcc acc gcc gcg acc gcc ggc gac aac gag tgagcatagt ctcatcgctt   2622
   Ser Ala Thr Ala Ala Thr Ala Gly Asp Asn Glu
   815                 820                 825
   agccgatgaa aggcccgggt gtccgcaccc gggccttt                            2660
<210> 20
   <211> 825
   <212> PRT
   <213> Bifidobacterium lactis
<400> 20
   Met Thr Asn Pro Val Ile Gly Thr Pro Trp Gln Lys Leu Asp Arg Pro
   1               5                   10                  15
   Val Ser Glu Glu Ala Ile Glu Gly Met Asp Lys Tyr Trp Arg Val Ala
               20                  25                  30
   Asn Tyr Met Ser Ile Gly Gln Ile Tyr Leu Arg Ser Asn Pro Leu Met
           35                  40                  45
   Lys Glu Pro Phe Thr Arg Asp Asp Val Lys His Arg Leu Val Gly His
       50                  55                  60
   Trp Gly Thr Thr Pro Gly Leu Asn Phe Leu Leu Ala His Ile Asn Arg
   65                  70                  75                  80
   Leu Ile Ala Asp His Gln Gln Asn Thr Val Phe Ile Met Gly Pro Gly
                   85                  90                  95
   His Gly Gly Pro Ala Gly Thr Ala Gln Ser Tyr Ile Asp Gly Thr Tyr
               100                 105                 110
   Thr Glu Tyr Tyr Pro Asn Ile Thr Lys Asp Glu Ala Gly Leu Gln Lys
           115                 120                 125
   Phe Phe Arg Gln Phe Ser Tyr Pro Gly Gly Ile Pro Ser His Phe Ala
       130                 135                 140
   Pro Glu Thr Pro Gly Ser Ile His Glu Gly Gly Glu Leu Gly Tyr Ala
   145                 150                 155                 160
   Leu Ser His Ala Tyr Gly Ala Ile Met Asp Asn Pro Ser Leu Phe Val
                   165                 170                 175
   Pro Cys Ile Ile Gly Asp Gly Glu Ala Glu Thr Gly Pro Leu Ala Thr
               180                 185                 190
   Gly Trp Gln Ser Asn Lys Leu Val Asn Pro Arg Thr Asp Gly Ile Val
           195                 200                 205
   Leu Pro Ile Leu His Leu Asn Gly Tyr Lys Ile Ala Asn Pro Thr Ile
       210                 215                 220
   Leu Ala Arg Ile Ser Asp Glu Glu Leu His Asp Phe Phe Arg Gly Met
   225                 230                 235                 240
   Gly Tyr His Pro Tyr Glu Phe Val Ala Gly Phe Asp Asn Glu Asp His
                   245                 250                 255
   Leu Ser Ile His Arg Arg Phe Ala Glu Leu Phe Glu Thr Ile Phe Asp
               260                 265                 270
   Glu Ile Cys Asp Ile Lys Ala Ala Ala Gln Thr Asp Asp Met Thr Arg
           275                 280                 285
   Pro Phe Tyr Pro Met Leu Ile Phe Arg Thr Pro Lys Gly Trp Thr Cys
       290                 295                 300
   Pro Lys Phe Ile Asp Gly Lys Lys Thr Glu Gly Ser Trp Arg Ala His
   305                 310                 315                 320
   Gln Val Pro Leu Ala Ser Ala Arg Asp Thr Glu Ala His Phe Glu Val
                   325                 330                 335
   Leu Lys Gly Trp Met Glu Ser Tyr Lys Pro Glu Glu Leu Phe Asn Ala
               340                 345                 350
   Asp Gly Ser Ile Lys Glu Asp Val Thr Ala Phe Met Pro Lys Gly Glu
           355                 360                 365
   Leu Arg Ile Gly Ala Asn Pro Asn Ala Asn Gly Gly Arg Ile Arg Glu
       370                 375                 380
   Asp Leu Lys Leu Pro Glu Leu Asp Gln Tyr Glu Ile Thr Gly Val Lys
   385                 390                 395                 400
   Glu Tyr Gly His Gly Trp Gly Gln Val Glu Ala Pro Arg Ser Leu Gly
                   405                 410                 415
   Ala Tyr Cys Arg Asp Ile Ile Lys Asn Asn Pro Asp Ser Phe Arg Val
               420                 425                 430
   Phe Gly Pro Asp Glu Thr Ala Ser Asn Arg Leu Asn Ala Thr Tyr Glu
           435                 440                 445
   Val Thr Lys Lys Gln Trp Asp Asn Gly Tyr Leu Ser Ala Leu Val Asp
       450                 455                 460
   Glu Asn Met Ala Val Thr Gly Gln Val Val Glu Gln Leu Ser Glu His
   465                 470                 475                 480
   Gln Cys Glu Gly Phe Leu Glu Ala Tyr Leu Leu Thr Gly Arg His Gly
                   485                 490                 495
   Ile Trp Ser Ser Tyr Glu Ser Phe Val His Val Ile Asp Ser Met Leu
               500                 505                 510
   Asn Gln His Ala Lys Trp Leu Glu Ala Thr Val Arg Glu Ile Pro Trp
           515                 520                 525
   Arg Lys Pro Ile Ser Ser Val Asn Leu Leu Val Ser Ser His Val Trp
       530                 535                 540
   Arg Gln Asp His Asn Gly Phe Ser His Gln Asp Pro Gly Val Thr Ser
   545                 550                 555                 560
   Val Leu Leu Asn Lys Thr Phe Asn Asn Asp His Val Thr Asn Ile Tyr
                   565                 570                 575
   Phe Ala Thr Asp Ala Asn Met Leu Leu Ala Ile Ala Glu Lys Cys Phe
               580                 585                 590
   Lys Ser Thr Asn Lys Ile Asn Ala Ile Phe Ala Gly Lys Gln Pro Ala
           595                 600                 605
   Ala Thr Trp Ile Thr Leu Asp Glu Val Arg Ala Glu Leu Glu Ala Gly
       610                 615                 620
   Ala Ala Glu Trp Lys Trp Ala Ser Asn Ala Lys Ser Asn Asp Glu Val
   625                 630                 635                 640
   Gln Val Val Leu Ala Ala Ala Gly Asp Val Pro Thr Gln Glu Ile Met
                   645                 650                 655
   Ala Ala Ser Asp Ala Leu Asn Lys Met Gly Ile Lys Phe Lys Val Val
               660                 665                 670
   Asn Val Val Asp Leu Ile Lys Leu Gln Ser Ser Lys Glu Asn Asp Glu
           675                 680                 685
   Ala Met Ser Asp Glu Asp Phe Ala Asp Leu Phe Thr Ala Asp Lys Pro
       690                 695                 700
   Val Leu Phe Ala Tyr His Ser Tyr Ala Gln Asp Val Arg Gly Leu Ile
   705                 710                 715                 720
   Tyr Asp Arg Pro Asn His Asp Asn Phe Thr Val Val Gly Tyr Lys Glu
                   725                 730                 735
   Gln Gly Ser Thr Thr Thr Pro Phe Asp Met Val Arg Val Asn Asp Met
               740                 745                 750
   Asp Arg Tyr Ala Leu Gln Ala Lys Ala Leu Glu Leu Ile Asp Ala Asp
           755                 760                 765
   Lys Tyr Ala Asp Lys Ile Asn Glu Leu Asn Glu Phe Arg Lys Thr Ala
       770                 775                 780
   Phe Gln Phe Ala Val Asp Asn Gly Tyr Asp Ile Pro Glu Phe Thr Asp
   785                 790                 795                 800
   Trp Val Tyr Pro Asp Val Lys Val Asp Glu Thr Ser Met Leu Ser Ala
                   805                 810                 815
   Thr Ala Ala Thr Ala Gly Asp Asn Glu
               820                 825
<210> 21
   <211> 1386
   <212> DNA
   <213> Corynebacterium glutamicum
<220>
   <221> CDS
   <222> (1)..(1383)
<400> 21
   atg tct gac aca ccg acc tca gct ctg atc acc acg gtc aac cgc agc      48
   Met Ser Asp Thr Pro Thr Ser Ala Leu Ile Thr Thr Val Asn Arg Ser
   1               5                   10                  15
   ttc gat gga ttc gat ttg gaa gaa gta gca gca gac ctt gga gtt cgg      96
   Phe Asp Gly Phe Asp Leu Glu Glu Val Ala Ala Asp Leu Gly Val Arg
               20                  25                  30
   ctc acc tac ctg ccc gac gaa gaa cta gaa gta tcc aaa gtt ctc gcg     144
   Leu Thr Tyr Leu Pro Asp Glu Glu Leu Glu Val Ser Lys Val Leu Ala
           35                  40                  45
   gcg gac ctc ctc gct gag ggg cca gct ctc atc atc ggt gta gga aac     192
   Ala Asp Leu Leu Ala Glu Gly Pro Ala Leu Ile Ile Gly Val Gly Asn
       50                  55                  60
   acg ttt ttc gac gcc cag gtc gcc gct gcc ctc ggc gtc cca gtg cta     240
   Thr Phe Phe Asp Ala Gln Val Ala Ala Ala Leu Gly Val Pro Val Leu
   65                  70                  75                  80
   ctg ctg gta gac aag caa ggc aag cac gtt gct ctt gct cgc acc cag     288
   Leu Leu Val Asp Lys Gln Gly Lys His Val Ala Leu Ala Arg Thr Gln
                   85                  90                  95
   gta aac aat gcc ggc gca gtt gtt gca gca gca ttt acc gct gaa caa     336
   Val Asn Asn Ala Gly Ala Val Val Ala Ala Ala Phe Thr Ala Glu Gln
               100                 105                 110
   gag cca atg ccg gat aag ctg cgc aag gct gtg cgc aac cac agc aac     384
   Glu Pro Met Pro Asp Lys Leu Arg Lys Ala Val Arg Asn His Ser Asn
           115                 120                 125
   ctc gaa cca gtc atg agc gcc gaa ctc ttt gaa aac tgg ctg ctc aag     432
   Leu Glu Pro Val Met Ser Ala Glu Leu Phe Glu Asn Trp Leu Leu Lys
       130                 135                 140
   cgc gca cgc gca gag cac tcc cac att gtg ctg cca gaa ggt gac gac     480
   Arg Ala Arg Ala Glu His Ser His Ile Val Leu Pro Glu Gly Asp Asp
   145                 150                 155                 160
   gac cgc atc ttg atg gct gcc cac cag ctg ctt gat caa gac atc tgt     528
   Asp Arg Ile Leu Met Ala Ala His Gln Leu Leu Asp Gln Asp Ile Cys
                   165                 170                 175
   gac atc acg atc ctg ggc gat cca gta aag atc aag gag cgc gct acc     576
   Asp Ile Thr Ile Leu Gly Asp Pro Val Lys Ile Lys Glu Arg Ala Thr
               180                 185                 190
   gaa ctt ggc ctg cac ctt aac act gca tac ctg gtc aat ccg ctg aca     624
   Glu Leu Gly Leu His Leu Asn Thr Ala Tyr Leu Val Asn Pro Leu Thr
           195                 200                 205
   gat cct cgc ctg gag gaa ttc gcc gaa caa ttc gcg gag ctg cgc aag     672
   Asp Pro Arg Leu Glu Glu Phe Ala Glu Gln Phe Ala Glu Leu Arg Lys
       210                 215                 220
   tca aag agc gtc act atc gat gaa gcc cgc gaa atc atg aag gat att     720
   Ser Lys Ser Val Thr Ile Asp Glu Ala Arg Glu Ile Met Lys Asp Ile
   225                 230                 235                 240
   tcc tac ttc ggc acc atg atg gtc cac aac ggc gac gcc gac gga atg     768
   Ser Tyr Phe Gly Thr Met Met Val His Asn Gly Asp Ala Asp Gly Met
                   245                 250                 255
   gta tcc ggt gca gca aac acc acc gca cac acc att aag cca agc ttc     816
   Val Ser Gly Ala Ala Asn Thr Thr Ala His Thr Ile Lys Pro Ser Phe
               260                 265                 270
   cag atc atc aaa act gtt cca gaa gca tcc gtc gtt tct tcc atc ttc     864
   Gln Ile Ile Lys Thr Val Pro Glu Ala Ser Val Val Ser Ser Ile Phe
               275                 280                 285
   ctc atg gtg ctg cgc ggg cga ctg tgg gca ttc ggc gac tgt gct gtt        912
   Leu Met Val Leu Arg Gly Arg Leu Trp Ala Phe Gly Asp Cys Ala Val
       290                 295                 300
   aac ccg aac cca act gct gaa cag ctt ggt gaa atc gcc gtt gtg tca        960
   Asn Pro Asn Pro Thr Ala Glu Gln Leu Gly Glu Ile Ala Val Val Ser
   305                 310                 315                 320
   gca aaa act gca gca caa ttt ggc att gat cct cgc gta gcc atc ttg       1008
   Ala Lys Thr Ala Ala Gln Phe Gly Ile Asp Pro Arg Val Ala Ile Leu
                   325                 330                 335
   tcc tac tcc act ggc aac tcc ggc gga ggc tca gat gtg gat cgc gcc       1056
   Ser Tyr Ser Thr Gly Asn Ser Gly Gly Gly Ser Asp Val Asp Arg Ala
               340                 345                 350
   atc gac gct ctt gca gaa gca cgc cga ctt aac cca gaa cta tgc gtc       1104
   Ile Asp Ala Leu Ala Glu Ala Arg Arg Leu Asn Pro Glu Leu Cys Val
           355                 360                 365
   gat gga cca ctt cag ttc gac gcc gcc gtc gac ccg ggt gtg gcg cgc       1152
   Asp Gly Pro Leu Gln Phe Asp Ala Ala Val Asp Pro Gly Val Ala Arg
       370                 375                 380
   aag aag atg cca gac tct gac gtc gct ggc cag gca aat gtg ttt atc       1200
   Lys Lys Met Pro Asp Ser Asp Val Ala Gly Gln Ala Asn Val Phe Ile
   385                 390                 395                 400
   ttc cct gac ctg gaa gcc gga aac atc ggc tac aaa act gca caa cgc       1248
   Phe Pro Asp Leu Glu Ala Gly Asn Ile Gly Tyr Lys Thr Ala Gln Arg
                   405                 410                 415
   acc ggt cac gcc ctg gca gtt ggt ccg att ctg cag ggc cta aac aaa       1296
   Thr Gly His Ala Leu Ala Val Gly Pro Ile Leu Gln Gly Leu Asn Lys
               420                 425                 430
   cca gtc aac gac ctt tcc cgt ggc gca aca gtc cct gac atc gtc aac       1344
   Pro Val Asn Asp Leu Ser Arg Gly Ala Thr Val Pro Asp Ile Val Asn
           435                 440                 445
   aca gta gcc atc aca gca att cag gca gga gga cgc agc taa               1386
   Thr Val Ala Ile Thr Ala Ile Gln Ala Gly Gly Arg Ser
       450                 455                 460
<210> 22
   <211> 461
   <212> PRT
   <213> Corynebacterium glutamicum
<400> 22
   Met Ser Asp Thr Pro Thr Ser Ala Leu Ile Thr Thr Val Asn Arg Ser
   1               5                   10                  15
   Phe Asp Gly Phe Asp Leu Glu Glu Val Ala Ala Asp Leu Gly Val Arg
               20                  25                  30
   Leu Thr Tyr Leu Pro Asp Glu Glu Leu Glu Val Ser Lys Val Leu Ala
           35                  40                  45
   Ala Asp Leu Leu Ala Glu Gly Pro Ala Leu Ile Ile Gly Val Gly Asn
       50                  55                  60
   Thr Phe Phe Asp Ala Gln Val Ala Ala Ala Leu Gly Val Pro Val Leu
   65                  70                  75                  80
   Leu Leu Val Asp Lys Gln Gly Lys His Val Ala Leu Ala Arg Thr Gln
                   85                  90                  95
   Val Asn Asn Ala Gly Ala Val Val Ala Ala Ala Phe Thr Ala Glu Gln
               100                 105                 110
   Glu Pro Met Pro Asp Lys Leu Arg Lys Ala Val Arg Asn His Ser Asn
           115                 120                 125
   Leu Glu Pro Val Met Ser Ala Glu Leu Phe Glu Asn Trp Leu Leu Lys
       130                 135                 140
   Arg Ala Arg Ala Glu His Ser His Ile Val Leu Pro Glu Gly Asp Asp
   145                 150                 155                 160
   Asp Arg Ile Leu Met Ala Ala His Gln Leu Leu Asp Gln Asp Ile Cys
                   165                 170                 175
   Asp Ile Thr Ile Leu Gly Asp Pro Val Lys Ile Lys Glu Arg Ala Thr
               180                 185                 190
   Glu Leu Gly Leu His Leu Asn Thr Ala Tyr Leu Val Asn Pro Leu Thr
           195                 200                 205
   Asp Pro Arg Leu Glu Glu Phe Ala Glu Gln Phe Ala Glu Leu Arg Lys
       210                 215                 220
   Ser Lys Ser Val Thr Ile Asp Glu Ala Arg Glu Ile Met Lys Asp Ile
   225                 230                 235                 240
   Ser Tyr Phe Gly Thr Met Met Val His Asn Gly Asp Ala Asp Gly Met
                   245                 250                 255
   Val Ser Gly Ala Ala Asn Thr Thr Ala His Thr Ile Lys Pro Ser Phe
               260                 265                 270
   Gln Ile Ile Lys Thr Val Pro Glu Ala Ser Val Val Ser Ser Ile Phe
           275                 280                 285
   Leu Met Val Leu Arg Gly Arg Leu Trp Ala Phe Gly Asp Cys Ala Val
       290                 295                 300
   Asn Pro Asn Pro Thr Ala Glu Gln Leu Gly Glu Ile Ala Val Val Ser
   305                 310                 315                 320
   Ala Lys Thr Ala Ala Gln Phe Gly Ile Asp Pro Arg Val Ala Ile Leu
                   325                 330                 335
   Ser Tyr Ser Thr Gly Asn Ser Gly Gly Gly Ser Asp Val Asp Arg Ala
               340                 345                 350
   Ile Asp Ala Leu Ala Glu Ala Arg Arg Leu Asn Pro Glu Leu Cys Val
           355                 360                 365
   Asp Gly Pro Leu Gln Phe Asp Ala Ala Val Asp Pro Gly Val Ala Arg
       370                 375                 380
   Lys Lys Met Pro Asp Ser Asp Val Ala Gly Gln Ala Asn Val Phe Ile
   385                 390                 395                 400
   Phe Pro Asp Leu Glu Ala Gly Asn Ile Gly Tyr Lys Thr Ala Gln Arg
                   405                 410                 415
   Thr Gly His Ala Leu Ala Val Gly Pro Ile Leu Gln Gly Leu Asn Lys
               420                 425                 430
   Pro Val Asn Asp Leu Ser Arg Gly Ala Thr Val Pro Asp Ile Val Asn
           435                 440                 445
   Thr Val Ala Ile Thr Ala Ile Gln Ala Gly Gly Arg Ser
       450                 455                 460
<210> 23
   <211> 2644
   <212> DNA
   <213> Corynebacterium glutamicum
<220>
   <221> CDS
   <222> (1214)..(2641)
<400> 23
   ttccaaagtc tggcacacga ggtcctgaga cttggaatca cgacgagcct gctccgaagc     60
   tgcatcgtaa tcaatgtcct cagggttcac gatcacctcg atggtgtcgg aatgatcaag    120
   ttccttcagt tccaacggag tgaacttcgc ctgagcaggt ccacgacgac caaaaacgtg    180
   cacttcctta gcctgattct tagccaagct ctcatagaca ttgtcaggga tttcagtaac    240
   tagcagctca tcgccagtct tcgccaaaat acgagcaacg tccaacgcca cgttaccgac    300
   accaacaacc gctaccttct cagcagaaag atcccagttg cgttcaaagt tcggattgcc    360
   atcatagaaa ccaacgaact cgccagcgcc ccacgaacct tccagatcag aacctggaac    420
   ccgaagatcc tggtcgccag tagcgccagt ggagaacacg atcgcgtcat aaaactcacg    480
   caactcctca acagtgatgt ccttgccgac ctcaatgttg cccaagaaac gcagctgctc    540
   cttgtccatc acattgtgca gggacttcac gatgcccttg atgcgagggt gatcaggcgc    600
   aacaccataa cggatcaaac cgaaaggcgc tggcatgcgt tcaaaaagat caatctgcac    660
   gtccgtgtcg gatttcatca acaaatcaga cgcgtagatt cctgctggac ctgcaccgac    720
   aacggcaaca cgcaaagggc gagacatata aagttcgatt ccttaaaggg gttctaaaaa    780
   atgtggagta tgtgagcggg gttccactag tagattcgac tcctatcggg gtgcgactgc    840
   taatggtgcc ctgctatcaa ccctccatga tacgtggtaa gtgcagacta ataaaggcca    900
   gtcggggagt attgggggct ttgctggggg cagatttgtc acgctgcgcg ctttcataga    960
   ccccattaat gtggggtgaa gagctgtaaa gtaccgctaa aaactttgca aagggtgctt   1020
   cgcaacttgt aaccgctccg tattgttttc tacggcaata agcatttgtg ctgctcaaag   1080
   cgtggaattg agatcggttt gaaaattaca aaataaaact ttgcaaaccg ggctgtacgc   1140
   aaggcggacg aacgctaaac tatgtaagaa atcacaactt cccctcagta gtgccaggag   1200
   gcacaagcct gaa gtg tca tca atg aga agg ttc agg ctg aaa cta gaa      1249
                  Val Ser Ser Met Arg Arg Phe Arg Leu Lys Leu Glu
                  1               5                   10
   agg cga tgt atg tct gac aca ccg acc tca gct ctg atc acc acg gtc     1297
   Arg Arg Cys Met Ser Asp Thr Pro Thr Ser Ala Leu Ile Thr Thr Val
           15                  20                  25
   aac cgc agc ttc gat gga ttc gat ttg gaa gaa gta gca gca gac ctt     1345
   Asn Arg Ser Phe Asp Gly Phe Asp Leu Glu Glu Val Ala Ala Asp Leu
       30                  35                  40
   gga gtt cgg ctc acc gac ctg ccc gac gaa gaa cta gaa gta tcc aaa     1393
   Gly Val Arg Leu Thr Asp Leu Pro Asp Glu Glu Leu Glu Val Ser Lys
   45                  50                  55                  60
   gtt ctc gcg gcg gac ctc ctc gct gag ggg cca gct ctc atc atc ggt     1441
   Val Leu Ala Ala Asp Leu Leu Ala Glu Gly Pro Ala Leu Ile Ile Gly
                   65                  70                  75
   gta gga aac acg ttt ttc gac gcc cag gtc gcc gct gcc ctc ggc gtc     1489
   Val Gly Asn Thr Phe Phe Asp Ala Gln Val Ala Ala Ala Leu Gly Val
               80                  85                  90
   cca gtg cta ctg ctg gta gac aag caa ggc aag cac gtt gct ctt gct     1537
   Pro Val Leu Leu Leu Val Asp Lys Gln Gly Lys His Val Ala Leu Ala
           95                  100                 105
   cgc acc cag gta aac aat gcc ggc gca gtt gtt gca gca gca ttt acc     1585
   Arg Thr Gln Val Asn Asn Ala Gly Ala Val Val Ala Ala Ala Phe Thr
       110                 115                 120
   gct gaa caa gag cca atg ccg gat aag ctg cgc aag gct gtg cgc aac     1633
   Ala Glu Gln Glu Pro Met Pro Asp Lys Leu Arg Lys Ala Val Arg Asn
   125                 130                 135                 140
   cac agc aac ctc gaa cca gtc atg agc gcc gaa ctc ttt gaa aac tgg     1681
   His Ser Asn Leu Glu Pro Val Met Ser Ala Glu Leu Phe Glu Asn Trp
                   145                 150                 155
   ctg ctc aag cgc gca cgc gca gag cac tcc cac att gtg ctg cca gaa     1729
   Leu Leu Lys Arg Ala Arg Ala Glu His Ser His Ile Val Leu Pro Glu
               160                 165                 170
   ggt gac gac gac cgc atc ttg atg gct gcc cac cag ctg ctt gat caa     1777
   Gly Asp Asp Asp Arg Ile Leu Met Ala Ala His Gln Leu Leu Asp Gln
           175                 180                 185
   gac atc tgt gac atc acg atc ctg ggc gat cca gta cag atc aag gag     1825
   Asp Ile Cys Asp Ile Thr Ile Leu Gly Asp Pro Val Gln Ile Lys Glu
       190                 195                 200
   cgc gct acc gaa ctt ggc ctg cac ctt aac act gca tac ctg gtc aat     1873
   Arg Ala Thr Glu Leu Gly Leu His Leu Asn Thr Ala Tyr Leu Val Asn
   205                 210                 215                 220
   ccg ctg aca gat cct cgc ctg gag gaa ttc gcc gaa caa ttc gcg gag     1921
   Pro Leu Thr Asp Pro Arg Leu Glu Glu Phe Ala Glu Gln Phe Ala Glu
                   225                 230                 235
   ctg cgc aag tca aag agc gtc act atc gat gaa gcc cgc gaa atc atg     1969
   Leu Arg Lys Ser Lys Ser Val Thr Ile Asp Glu Ala Arg Glu Ile Met
               240                 245                 250
   aag gat att tgc tac ttc ggc acc atg atg gtc cac aac ggc gac gcc     2017
   Lys Asp Ile Cys Tyr Phe Gly Thr Met Met Val His Asn Gly Asp Ala
           255                 260                 265
   gac gga atg gta tcc ggt gca gca aac acc acc gca cac acc att aag     2065
   Asp Gly Met Val Ser Gly Ala Ala Asn Thr Thr Ala His Thr Ile Lys
       270                 275                 280
   cca agc ttc cag atc atc aaa act gtt cca gaa gca tcc gtc gtt tct     2113
   Pro Ser Phe Gln Ile Ile Lys Thr Val Pro Glu Ala Ser Val Val Ser
   285                 290                 295                 300
   tcc atc ttc ctc atg gtg ctg cgc ggg cga ctg tgg gca ttc ggc gac     2161
   Ser Ile Phe Leu Met Val Leu Arg Gly Arg Leu Trp Ala Phe Gly Asp
                   305                 310                 315
   tgt gct gtt aac ccg aac cca act gct gaa cag ctt ggt gaa atc gcc     2209
   Cys Ala Val Asn Pro Asn Pro Thr Ala Glu Gln Leu Gly Glu Ile Ala
               320                 325                 330
   gtt gtg tca gca aaa act gca gca caa ttt ggc att gat cct cgc gta      2257
   Val Val Ser Ala Lys Thr Ala Ala Gln Phe Gly Ile Asp Pro Arg Val
           335                 340                 345
   gcc atc ttg tcc tac tcc act ggc aac tcc ggc gga ggc tca gat gtg      2305
   Ala Ile Leu Ser Tyr Ser Thr Gly Asn Ser Gly Gly Gly Ser Asp Val
       350                 355                 360
   gat cgc gcc atc gac gct ctt gca gaa gca cgc cga ctt aac cca gaa      2353
   Asp Arg Ala Ile Asp Ala Leu Ala Glu Ala Arg Arg Leu Asn Pro Glu
   365                 370                 375                 380
   cta tgc gtc gat gga cca ctt cag ttc gac gcc gcc gtc gac ccg ggt      2401
   Leu Cys Val Asp Gly Pro Leu Gln Phe Asp Ala Ala Val Asp Pro Gly
                   385                 390                 395
   gtg gcg cgc aag aag atg cca gac tct gac gtc gct ggc cag gca aat      2449
   Val Ala Arg Lys Lys Met Pro Asp Ser Asp Val Ala Gly Gln Ala Asn
               400                 405                 410
   gtg ttt atc ttc cct gac ctg gaa gcc gga aac atc ggc tac aaa act      2497
   Val Phe Ile Phe Pro Asp Leu Glu Ala Gly Asn Ile Gly Tyr Lys Thr
           415                 420                 425
   gca caa cgc acc ggt cac gcc ctg gca gtt ggt ccg att ctg cag ggc      2545
   Ala Gln Arg Thr Gly His Ala Leu Ala Val Gly Pro Ile Leu Gln Gly
       430                 435                 440
   cta aac aaa cca gtc aac gac ctt tcc cgt ggc gca aca gtc cct gac      2593
   Leu Asn Lys Pro Val Asn Asp Leu Ser Arg Gly Ala Thr Val Pro Asp
   445                 450                 455                 460
   atc gtc aac aca gta gcc atc aca gca att cag gca gga gga cgc agc      2641
   Ile Val Asn Thr Val Ala Ile Thr Ala Ile Gln Ala Gly Gly Arg Ser
                   465                 470                 475
   taa                                                                  2644
<210> 24
   <211> 476
   <212> PRT
   <213> Corynebacterium glutamicum
<400> 24
   Val Ser Ser Met Arg Arg Phe Arg Leu Lys Leu Glu Arg Arg Cys Met
   1               5                   10                  15
   Ser Asp Thr Pro Thr Ser Ala Leu Ile Thr Thr Val Asn Arg Ser Phe
               20                  25                  30
   Asp Gly Phe Asp Leu Glu Glu Val Ala Ala Asp Leu Gly Val Arg Leu
           35                  40                  45
   Thr Asp Leu Pro Asp Glu Glu Leu Glu Val Ser Lys Val Leu Ala Ala
       50                  55                  60
   Asp Leu Leu Ala Glu Gly Pro Ala Leu Ile Ile Gly Val Gly Asn Thr
   65                  70                  75                  80
   Phe Phe Asp Ala Gln Val Ala Ala Ala Leu Gly Val Pro Val Leu Leu
                   85                  90                  95
   Leu Val Asp Lys Gln Gly Lys His Val Ala Leu Ala Arg Thr Gln Val
               100                 105                 110
   Asn Asn Ala Gly Ala Val Val Ala Ala Ala Phe Thr Ala Glu Gln Glu
           115                 120                 125
   Pro Met Pro Asp Lys Leu Arg Lys Ala Val Arg Asn His Ser Asn Leu
       130                 135                 140
   Glu Pro Val Met Ser Ala Glu Leu Phe Glu Asn Trp Leu Leu Lys Arg
   145                 150                 155                 160
   Ala Arg Ala Glu His Ser His Ile Val Leu Pro Glu Gly Asp Asp Asp
                   165                 170                 175
   Arg Ile Leu Met Ala Ala His Gln Leu Leu Asp Gln Asp Ile Cys Asp
               180                 185                 190
   Ile Thr Ile Leu Gly Asp Pro Val Gln Ile Lys Glu Arg Ala Thr Glu
           195                 200                 205
   Leu Gly Leu His Leu Asn Thr Ala Tyr Leu Val Asn Pro Leu Thr Asp
       210                 215                 220
   Pro Arg Leu Glu Glu Phe Ala Glu Gln Phe Ala Glu Leu Arg Lys Ser
   225                 230                 235                 240
   Lys Ser Val Thr Ile Asp Glu Ala Arg Glu Ile Met Lys Asp Ile Cys
                   245                 250                 255
   Tyr Phe Gly Thr Met Met Val His Asn Gly Asp Ala Asp Gly Met Val
               260                 265                 270
   Ser Gly Ala Ala Asn Thr Thr Ala His Thr Ile Lys Pro Ser Phe Gln
           275                 280                 285
   Ile Ile Lys Thr Val Pro Glu Ala Ser Val Val Ser Ser Ile Phe Leu
       290                 295                 300
   Met Val Leu Arg Gly Arg Leu Trp Ala Phe Gly Asp Cys Ala Val Asn
   305                 310                 315                 320
   Pro Asn Pro Thr Ala Glu Gln Leu Gly Glu Ile Ala Val Val Ser Ala
                   325                 330                 335
   Lys Thr Ala Ala Gln Phe Gly Ile Asp Pro Arg Val Ala Ile Leu Ser
               340                 345                 350
   Tyr Ser Thr Gly Asn Ser Gly Gly Gly Ser Asp Val Asp Arg Ala Ile
           355                 360                 365
   Asp Ala Leu Ala Glu Ala Arg Arg Leu Asn Pro Glu Leu Cys Val Asp
       370                 375                 380
   Gly Pro Leu Gln Phe Asp Ala Ala Val Asp Pro Gly Val Ala Arg Lys
   385                 390                 395                 400
   Lys Met Pro Asp Ser Asp Val Ala Gly Gln Ala Asn Val Phe Ile Phe
                   405                 410                 415
   Pro Asp Leu Glu Ala Gly Asn Ile Gly Tyr Lys Thr Ala Gln Arg Thr
               420                 425                 430
   Gly His Ala Leu Ala Val Gly Pro Ile Leu Gln Gly Leu Asn Lys Pro
           435                 440                 445
   Val Asn Asp Leu Ser Arg Gly Ala Thr Val Pro Asp Ile Val Asn Thr
       450                 455                 460
   Val Ala Ile Thr Ala Ile Gln Ala Gly Gly Arg Ser
   465                 470                 475
<210> 25
   <211> 37
   <212> DNA
   <213> Artificial sequence
<220>
   <223> primer
<400> 25
   cggggtacct ataaagttcg attccttaaa ggggttc 37
<210> 26
   <211> 38
   <212> DNA
   <213> Artificial sequence
<220>
   <223> primer
<400> 26
   cgcggatccg tgccaatgcc attagctgcg tcctcctg 38
<210> 27
   <211> 1063
   <212> DNA
   <213> Escherichia coli
<220>
   <221> CDS
   <222> (201)..(860)
<400> 27
   cccgcttcga cctgcaaatc tatcccacga agcgcctgaa cgccgcctgg ataggttttt      60
   ttaagctgtt gaagttccag tgcaatggtc ataaattttt acttacctta cgttcttaca     120
   ctttatctgt ggtttaaatc gtcccggagt tgccctatat tagccaaacg taattatttg     180
   gttacaggtc gttaacctcc atg aaa gac ata gat aca ctc atc agc aac aat     233
                         Met Lys Asp Ile Asp Thr Leu Ile Ser Asn Asn
                         1               5                   10
   gca cta tgg tca aaa atg ctg gtg gaa gag gat ccc ggg ttt ttt gag       281
   Ala Leu Trp Ser Lys Met Leu Val Glu Glu Asp Pro Gly Phe Phe Glu
               15                  20                  25
   aaa ctg gca caa gcg caa aaa ccg cgc ttt cta tgg att gga tgt tcc       329
   Lys Leu Ala Gln Ala Gln Lys Pro Arg Phe Leu Trp Ile Gly Cys Ser
           30                  35                  40
   gac agt cgc gtt cct gca gaa cgt tta acc ggt ctt gag ccg ggc gaa       377
   Asp Ser Arg Val Pro Ala Glu Arg Leu Thr Gly Leu Glu Pro Gly Glu
       45                  50                  55
   ctc ttt gtt cac cgt aat gtt gct aac ctg gtc att cac act gac ctg       425
   Leu Phe Val His Arg Asn Val Ala Asn Leu Val Ile His Thr Asp Leu
   60                  65                  70                  75
   aac tgc ctt tcc gtg gtt cag tat gca gtg gat gta ctc gaa gtt gaa       473
   Asn Cys Leu Ser Val Val Gln Tyr Ala Val Asp Val Leu Glu Val Glu
                   80                  85                  90
   cac att att atc tgt ggc cac tac ggt tgc ggc ggc gta caa gcc gca       521
   His Ile Ile Ile Cys Gly His Tyr Gly Cys Gly Gly Val Gln Ala Ala
               95                  100                 105
   gtt gaa aac ccg gaa ctg ggg ctt atc aac aac tgg ctg ctg cat atc       569
   Val Glu Asn Pro Glu Leu Gly Leu Ile Asn Asn Trp Leu Leu His Ile
           110                 115                 120
   cgc gat atc tgg ttc aaa cat agc tca ttg ctc ggc gaa atg ccg caa       617
   Arg Asp Ile Trp Phe Lys His Ser Ser Leu Leu Gly Glu Met Pro Gln
       125                 130                 135
   gag cgc cgt ctg gat acc ttg tgt gaa ctg aac gtc atg gaa cag gtg       665
   Glu Arg Arg Leu Asp Thr Leu Cys Glu Leu Asn Val Met Glu Gln Val
   140                 145                 150                 155
   tat aac ctg ggc cac tcc acc att atg caa tca gcg tgg aaa cgc ggg       713
   Tyr Asn Leu Gly His Ser Thr Ile Met Gln Ser Ala Trp Lys Arg Gly
                   160                 165                 170
   cag aaa gtt acc att cac ggc tgg gcc tac ggc att cac gac ggc ttg       761
   Gln Lys Val Thr Ile His Gly Trp Ala Tyr Gly Ile His Asp Gly Leu
               175                 180                 185
   ctg cgt gat ctg gat gtt acc gcc acc aac cgc gaa acc ctt gag caa       809
   Leu Arg Asp Leu Asp Val Thr Ala Thr Asn Arg Glu Thr Leu Glu Gln
           190                 195                 200
   cgt tac cgt cac ggg att tcc aac ctc aag ctg aaa cac gcc aac cac       857
   Arg Tyr Arg His Gly Ile Ser Asn Leu Lys Leu Lys His Ala Asn His
       205                 210                 215
   aaa taaaaatgcc atgccggatg caacacatcc ggcaacttca cacttactcg            910
   Lys
   220
   tccagcagaa tcactttgcc gatatacggc agatgacggt aacgctgtgc gtaatcaatg     970
   ccgtaaccca ccacaaactc atccgggatc gagaaaccga taaattctac cgggacgttc    1030
   acttcacgac gggacggttt atccagcagc gta                                 1063
<210> 28
   <211> 220
   <212> PRT
   <213> Escherichia coli
<400> 28
   Met Lys Asp Ile Asp Thr Leu Ile Ser Asn Asn Ala Leu Trp Ser Lys
   1               5                   10                  15
   Met Leu Val Glu Glu Asp Pro Gly Phe Phe Glu Lys Leu Ala Gln Ala
               20                  25                  30
   Gln Lys Pro Arg Phe Leu Trp Ile Gly Cys Ser Asp Ser Arg Val Pro
           35                  40                  45
   Ala Glu Arg Leu Thr Gly Leu Glu Pro Gly Glu Leu Phe Val His Arg
       50                  55                  60
   Asn Val Ala Asn Leu Val Ile His Thr Asp Leu Asn Cys Leu Ser Val
   65                  70                  75                  80
   Val Gln Tyr Ala Val Asp Val Leu Glu Val Glu His Ile Ile Ile Cys
                   85                  90                  95
   Gly His Tyr Gly Cys Gly Gly Val Gln Ala Ala Val Glu Asn Pro Glu
               100                 105                 110
   Leu Gly Leu Ile Asn Asn Trp Leu Leu His Ile Arg Asp Ile Trp Phe
           115                 120                 125
   Lys His Ser Ser Leu Leu Gly Glu Met Pro Gln Glu Arg Arg Leu Asp
       130                 135                 140
   Thr Leu Cys Glu Leu Asn Val Met Glu Gln Val Tyr Asn Leu Gly His
   145                 150                 155                 160
   Ser Thr Ile Met Gln Ser Ala Trp Lys Arg Gly Gln Lys Val Thr Ile
                   165                 170                 175
   His Gly Trp Ala Tyr Gly Ile His Asp Gly Leu Leu Arg Asp Leu Asp
               180                 185                 190
   Val Thr Ala Thr Asn Arg Glu Thr Leu Glu Gln Arg Tyr Arg His Gly
           195                 200                 205
   Ile Ser Asn Leu Lys Leu Lys His Ala Asn His Lys
       210                 215                 220
<210> 29
   <211> 1060
   <212> DNA
   <213> Escherichia coli
<220>
   <221> CDS
   <222> (201)..(857)
<400> 29
   gcaggttggg agacgtgcaa cgcactggcg gcacgggtga agctgccatg ttcagccacg     60
   gcaagaaaat aattgatatg tcgagagagc attcgcaacc tataagtaaa tccaatggaa    120
   ctcatcataa atgagacttt taccttatga caatcggcga gtagtctgcc tctcattcca    180
   gagacagaca gaggttaacg gtg aaa gag att att gat gga ttc ctt aaa ttc    233
                         Val Lys Glu Ile Ile Asp Gly Phe Leu Lys Phe
                         1               5                   10
   cag cgc gag gca ttt ccg aag cgg gaa gcc ttg ttt aaa cag ctg gcg      281
   Gln Arg Glu Ala Phe Pro Lys Arg Glu Ala Leu Phe Lys Gln Leu Ala
               15                  20                  25
   aca cag caa agc ccg cgc aca ctt ttt atc tcc tgc tcc gac agc cgt      329
   Thr Gln Gln Ser Pro Arg Thr Leu Phe Ile Ser Cys Ser Asp Ser Arg
           30                  35                  40
   ctg gtc cct gag ctg gtg acg caa cgt gag cct ggc gat ctg ttc gtt      377
   Leu Val Pro Glu Leu Val Thr Gln Arg Glu Pro Gly Asp Leu Phe Val
       45                  50                  55
   att cgc aac gcg ggc aat atc gtc cct tcc tac ggg ccg gaa ccc ggt      425
   Ile Arg Asn Ala Gly Asn Ile Val Pro Ser Tyr Gly Pro Glu Pro Gly
   60                  65                  70                  75
   ggc gtt tct gct tcg gtg gag tat gcc gtc gct gcg ctt cgg gta tct      473
   Gly Val Ser Ala Ser Val Glu Tyr Ala Val Ala Ala Leu Arg Val Ser
                   80                  85                  90
   gac att gtg att tgt ggt cat tcc aac tgt ggc gcg atg acc gcc att      521
   Asp Ile Val Ile Cys Gly His Ser Asn Cys Gly Ala Met Thr Ala Ile
               95                  100                 105
   gcc agc tgt cag tgc atg gac cat atg cct gcc gtc tcc cac tgg ctg      569
   Ala Ser Cys Gln Cys Met Asp His Met Pro Ala Val Ser His Trp Leu
           110                 115                 120
   cgt tat gcc gat tca gcc cgc gtc gtt aat gag gcg cgc ccg cat tcc      617
   Arg Tyr Ala Asp Ser Ala Arg Val Val Asn Glu Ala Arg Pro His Ser
       125                 130                 135
   gat tta ccg tca aaa gct gcg gcg atg gta cgt gaa aac gtc att gct      665
   Asp Leu Pro Ser Lys Ala Ala Ala Met Val Arg Glu Asn Val Ile Ala
   140                 145                 150                 155
   cag ttg gct aat ttg caa act cat cca tcg gtg cgc ctg gcg ctc gaa      713
   Gln Leu Ala Asn Leu Gln Thr His Pro Ser Val Arg Leu Ala Leu Glu
                   160                 165                 170
   gag ggg cgg atc gcc ctg cac ggc tgg gtc tac gac att gaa agc ggc      761
   Glu Gly Arg Ile Ala Leu His Gly Trp Val Tyr Asp Ile Glu Ser Gly
               175                 180                 185
   agc atc gca gct ttt gac ggc gca acc cgc cag ttt gtg cca ctg gcc      809
   Ser Ile Ala Ala Phe Asp Gly Ala Thr Arg Gln Phe Val Pro Leu Ala
           190                 195                 200
   gct aat cct cgc gtt tgt gcc ata ccg cta cgc caa ccg acc gca gcg      857
   Ala Asn Pro Arg Val Cys Ala Ile Pro Leu Arg Gln Pro Thr Ala Ala
       205                 210                 215
   taaccttatt tttaaaccat caggagttcc accatgattc agtcacaaat taaccgcaat    917
   attcgtcttg atcttgccga tgccattttg ctcagcaaag ctaaaaaaga tctctcattt    977
   gccgagattg ccgacggcac cggtctggca gaagcctttg taaccgcggc tttgctgggt   1037
   cagcaggcgc ttcctgccga cgc                                           1060
<210> 30
   <211> 219
   <212> PRT
   <213> Escherichia coli
<400> 30
   Val Lys Glu Ile Ile Asp Gly Phe Leu Lys Phe Gln Arg Glu Ala Phe
   1               5                   10                  15
   Pro Lys Arg Glu Ala Leu Phe Lys Gln Leu Ala Thr Gln Gln Ser Pro
               20                  25                  30
   Arg Thr Leu Phe Ile Ser Cys Ser Asp Ser Arg Leu Val Pro Glu Leu
           35                  40                  45
   Val Thr Gln Arg Glu Pro Gly Asp Leu Phe Val Ile Arg Asn Ala Gly
       50                  55                  60
   Asn Ile Val Pro Ser Tyr Gly Pro Glu Pro Gly Gly Val Ser Ala Ser
   65                  70                  75                  80
   Val Glu Tyr Ala Val Ala Ala Leu Arg Val Ser Asp Ile Val Ile Cys
                   85                  90                  95
   Gly His Ser Asn Cys Gly Ala Met Thr Ala Ile Ala Ser Cys Gln Cys
               100                 105                 110
   Met Asp His Met Pro Ala Val Ser His Trp Leu Arg Tyr Ala Asp Ser
           115                 120                 125
   Ala Arg Val Val Asn Glu Ala Arg Pro His Ser Asp Leu Pro Ser Lys
       130                 135                 140
   Ala Ala Ala Met Val Arg Glu Asn Val Ile Ala Gln Leu Ala Asn Leu
   145                 150                 155                 160
   Gln Thr His Pro Ser Val Arg Leu Ala Leu Glu Glu Gly Arg Ile Ala
                   165                 170                 175
   Leu His Gly Trp Val Tyr Asp Ile Glu Ser Gly Ser Ile Ala Ala Phe
               180                 185                 190
   Asp Gly Ala Thr Arg Gln Phe Val Pro Leu Ala Ala Asn Pro Arg Val
           195                 200                 205
   Cys Ala Ile Pro Leu Arg Gln Pro Thr Ala Ala
       210                 215